# EUROPEAN PATENT SPECIFICATION

(11) **EP 3 049 439 B1**
(45) Date of publication and mention of the grant of the patent: **25.12.2019**
(21) Application number: 14777078.8
(22) Date of filing: 26.09.2014
(51) Int. Cl.: C07K 16/28, C07K 16/30

(54) **BISPECIFIC NANOBODIES**
BISPEZIFISCHE NANOKÖRPER
NANOCORPS BISPÉCIFIQUES

(30) Priority: 26.09.2013 US 201361882877 P
(43) Date of publication of application: 03.08.2016
(73) Proprietor: Ablynx N.V., 9052 Ghent-Zwijnaarde (BE)
(72) Inventor: ROOBROUCK, Annelies, B-9700 Oudenaarde (BE); STORTELERS, Catelijne, B-9000 Gent (BE); VANLANDSCHOOT, Peter, B-9881 Bellem (BE); CONDE, Miguel, B-9000 Gent (BE); STAELENS, Stephanie, B-8560 Wevelgem (BE); SOARES, Hugo, PT-2670-559 Loures (PT); SCHOLS, Dominique, B-3020 Herent (BE)
(74) Representative: Hoffmann Eitle
(86) International application number: PCT/EP2014/070692
(87) International publication number: WO 2015/044386

(56) References cited:
- WO-A1-2007/066106
- WO-A1-2009/030285
- WO-A1-2012/042026
- WO-A2-2007/042289
- WO-A2-2010/037838
- WO-A2-2013/064701
- ROB C. ROOVERS ET AL: "A biparatopic anti-EGFR nanobody efficiently inhibits solid tumour growth", INTERNATIONAL JOURNAL OF CANCER, vol. 129, no. 8, 15 October 2011 (2011-10-15), pages 2013-2024, XP055086969, ISSN: 0020-7136, DOI: 10.1002/ijc.26145
- C. ROZAN ET AL: "Single-Domain Antibody-Based and Linker-Free Bispecific Antibodies Targeting Fc RIII Induce Potent Antitumor Activity without Recruiting Regulatory T Cells", MOLECULAR CANCER THERAPEUTICS, vol. 12, no. 8, 1 August 2013 (2013-08-01) , pages 1481-1491, XP055156453, ISSN: 1535-7163, DOI: 10.1158/1535-7163.MCT-12-1012
- LU D ET AL: "Simultaneous blockade of both the epidermal growth factor receptor and the insulin-like growth factor receptor signaling pathways in cancer cells with a fully human recombinant bispecific antibody", JOURNAL OF BIOLOGICAL CHEMISTRY, AMERICAN SOCIETY FOR BIOCHEMISTRY AND MOLECULAR BIOLOGY, US, vol. 279, no. 4, 23 January 2004 (2004-01-23), pages 2856-2865, XP002316541, ISSN: 0021-9258, DOI: 10.1074/JBC.M310132200
- ROBINSON M K ET AL: "Targeting ErbB2 and ErbB3 with a bispecific single-chain Fv enhances targeting selectivity and induces a therapeutic effect in vitro", BRITISH JOURNAL OF CANCER, NATURE PUBLISHING GROUP, GB, vol. 99, no. 9, 28 October 2008 (2008-10-28), pages 1415-1425, XP009115294, ISSN: 0007-0920, DOI: 10.1038/SJ.BJC.6604700 [retrieved on 2008-10-07]

## Description

### FIELD OF THE INVENTION

The present invention relates to bispecific polypeptides as defined by the claims comprising a first, functional and a second, anchoring immunoglobulin single variable domain (ISV), wherein said first ISV binds to a first target on the surface of a cancer cell with a low affinity and, when bound inhibits a function of said first target, and a said second ISV binds to a second target on the surface of said cell with a high affinity and wherein said first target is different from said second target. The present disclosure further discloses methods for identifying and making the same.

### BACKGROUND

Historically, a major problem with the modalities of cancer treatment was the lack of specificity for the cancer cell. A new era in cancer therapy began with antibodies, which can confer true specific and targeted therapy. Already in 1997 the first monoclonal, *i.e.* rituximab, was approved. Monoclonal antibodies are now widely recognized as therapeutic molecules, with more than 23 approvals in the US only, of which already 9 in the field of cancer. Unfortunately, none of them are able to cure cancer as single agents. Nevertheless six out of the ten best selling drugs nowadays are monoclonal antibodies. This initial success prompted numerous companies to also develop therapies based on monoclonal antibodies. However, the ratio of approved monoclonal antibody therapies compared to the number of monoclonal antibodies entering clinical studies is declining.

Various cancer cells over-express targets, which are involved in the malignant process. HER2 and FGFR are well known examples. Nevertheless, not all malignant cells over-express targets which contribute to the malignancy. Moreover, cancer cells rarely have unique targets on their surface. Instead cancer cells have generally a different constitution of targets than normal cells. Indeed, many differences between normal and malignant cells are only expression differences. This is also one of the reasons why diagnostic biomarkers are so hard to validate for cancer. It is not surprising that current cancer treatments face the difficulty of killing cancer cells but evading normal cells, considering that the therapeutic antibodies will not only bind their cognate target on the cancer cell but also on the normal cell, impairing the function of both. This results in toxicity and unwanted side-effects. The most commonly observed side-effects of these treatments include nausea, diarrhea, constipation, problems with blood clotting and wound healing, high blood pressure, gastrointestinal perforation, dizziness, anemia, emphydema, pain and fatigue. For patients, such side-effects can take over daily life. They can make patients uncomfortable at best and miserable at worst, affecting their ability to stick to their treatments, or making treatments less effective than they could be. These side-effects result in a high burden on both the patient as well as society. Several clinical trials even had to stop because of the severity of the side-effects and toxicity. For instance, initial results with the antibody 12F4 of GSK looked very promising. However, cardiovascular events necessitated to stop the clinical studies. Another example is presented by the blockade of the PDGFRβ by CDP860, an engineered Fab' fragment-polyethylene glycol conjugate, which leaded to severe fluid accumulation in patients with ovarian or colorectal cancer, associated with increased tumor vascularized volume (Jayson et al. 2005, J Clin Oncol 23:973-981).

An attempt to decrease toxicity and side-effects was by increasing the affinity of the antibodies for the functional targets. Hence, lower doses could be administered of antibodies, which would preferentially bind to the cancer cells over-expressing these targets but less to normal cells having fewer targets. Although in theory this would decrease toxicity and side-effects, a major draw-back of increased affinity was reduced tumour penetration due to rapid removal of the antibody following target-mediated internalisation (Schmidt et al. 2008 Canc Imm Imm 57(12): 1879-1890; Ackermann et al 2008 Mol Cancer Ther 2008;7:2233-2240).

A further attempt to decrease toxicity and unwanted side-effects was by creating bispecific antibodies binding to two different targets (see review by Kontermann, MAbs 2012 4(2):182-97. doi: 10.4161/mabs.4.2.19000. Epub 2012 Mar 1: Dual targeting strategies with bispecific antibodies). Bispecific antibodies can be used for dual targeting of cell surface receptors essentially in two manners: (i) by targeting of cell surface receptors expressed on the same cell (by acting *in cis*), and (ii) for retargeting of a therapeutically active moiety, i.e. effector molecules and effector cells (by acting *in trans*). In its simplest cis-format, a cancer cell can be considered comprising a unique combination of two targets compared to normal cells. This combination of targets is not present as such on normal cells, but each individual target is present on a particular normal cell type. Merely providing a mixture of two monoclonal antibodies (mAbs) each binding a specific target would not increase specificity for the cancer cell. It was thus hypothesized that this shortcoming could be overcome by creating bispecific antibodies capable of simultaneous binding to two different targets (see e.g. Chames and Baty 2009 mAbs I:6 539-549). Although bispecific antibodies have been successfully generated, they are hard to produce since they require the fusion of a heavy and light chain, which in practice results in an overrepresentation of wrong fusion products. Various different bispecific formats have therefore been suggested, mostly based on combining monovalent fragments, but none of which has been approved. It is believed that monovalent fragments lack the required high affinity and long retention times of conventional antibodies. MEHD7945A is an example of a two-in-one Mab with specificity for EGFR and Her3, which is currently being tested in early clinical trials is, Both arms have high affinities to EGFR (1.9nM), and Her3 (0.39 nM), but simultaneous binding to both receptors was not demonstrated. In all, few candidates based on these formats have reached the clinic. Based hereon, it was suggested to develop new formats.

Apart from the format, it was considered desirable that each of the individual binding domains in the bispecific antibody should bind to a target which contributes to the malignancy, thereby thwarting possible redundancy or resistance of single targets. For instance, the bispecific antibody may bind two epitopes on the same receptor. Jaenichen et al (2009) describes a bispecific Nanobody, in which the domains bind to different epitopes on CXCR4. Also bispecifics with two functionalities on different cells have been generated, e.g. targeting the host immune system towards the cancer cell (trans-format). The most widely used application of this approach is in cancer immunotherapy, where bispecific antibodies have been engineered that simultaneously bind to a cytotoxic cell (using a receptor-like CD3) and a target like a tumour cell to be destroyed. Although this approach increases the effectiveness of the therapy by destroying cancer cells, the specificity problem remains.

The bispecific antibodies of the art are specifically designed to bind simultaneously multiple receptor activation and downstream signal transduction pathways.

Roovers et al 2011 J Canc 129:2013-2024 relates to a biparatopic anti-EGFR Nanobody® inhibiting solid tumor growth.

WO2007/042289 in the name of Ablynx relates to Nanobodies® and polypeptides against EGFR and/or IGF-IR, which are both tumor associated antigens (TAAs).

Rozan et al 2013 Mol Canc Ther 12:1481-1491 relates to single-domain antibody-based and linker-free bispecific antibodies which target and activate FcγRIIIa.

WO2007/066106 in the name of Domantis relates to ligands that have binding specificity for EGFR and/or VEGF and which both function to inhibit the respective function of its target, but do not function to enhance binding of its partner.

WO2013/064701 in the name of ArgenX relates to bispecific antibodies and methods for isolating the same, but which are not both present on the surface of a cell.

WO2010/037838 in the name of Micromet relates to cross-species specific bispecific single chain antibodies, of which the 1^{st} moiety binds CD3ε, functioning to recruit the activity of a human immune effector cell, while the 2^{nd} moiety of is a TAA.

WO2009/030285 in the name of Ablynx relates to binding molecules with multiple binding sites, compositions comprising the same and uses thereof, in which the first binding site overlaps with the second binding site.

Lu et al 2004 JBC 279:2856-2865 relates to the simultaneous blockade of both the EGFR and the IGFR signaling pathways in cancer cells with a fully human recombinant bispecific antibody, in which both binding moieties have a high affinity for its respective target.

Robinson et al 2008 Br J Canc 99:1415-1425 relates to the targeting the heterodimer of the TAA's ErbB2 and ErbB3 with a biparatopic single-chain Fv (scFv).

WO2012/042026 in the name of Ablynx relates to biological materials related to c-Met, possibly in combination with VEGF and/or EGFR, which all are inhibitors and TAA's.

It will be apparent that only a few pathologies, e.g. cancer types, are amenable to this approach since not all diseased or aberrant cells, e.g. malignant cells over-express targets which contribute to the pathology e.g. malignancy.

Binding to a cancer cell surface target is sometimes insufficient to deliver potent therapeutic effect. Recently the concept of conjugating cytotoxic compounds to monoclonal antibodies (called antibody-drug conjugates or ADC) has gained a great deal of interest to improve efficacy. For targeted delivery of a cytotoxic payload, the choice of a target that discriminates between tumour and normal cells is even more critical than for functional blocking antibodies, due to the high toxicity of the payloads. To our knowledge there is no precedent for the use of bi-specific antibodies in ADCs or Radio Immuno Therapy (RIT) to improve tumour selectivity.

Accordingly, there is room for improvement.

### SUMMARY OF THE INVENTION

Antibody therapy is now an important part of the physician's armamentarium to battle diseases and especially cancer. All of the contemporaneously approved antibody therapies rely on monospecific antibodies. However, the medical use of many of these antibodies is severely hampered by their intrinsic, systemic toxicity. The key reason underlying this generalized toxicity is their pleiotropic binding pattern: the antibodies bind their cognate targets not only on the diseased cells, such as cancer cells, but also on normal cells, resulting in toxicity and unwanted side-effects when administered in high doses.

The art is in need of more effective therapies, such as cancer therapies, having superior selectivity and specificity for diseased cells, such as cancer cells, over normal cells, thereby reducing toxicity and side-effects.

The present inventors hypothesized that the specificity of the antibody therapy for the diseased cell e.g. cancer cell over the normal cell could be increased significantly by bispecific polypeptides comprising at least two subunits having different affinities and functionalities. This concept not only increases the operational specificity for a diseased cell, thereby decreasing toxicity and side-effects, it also widens the number of possible therapeutic targets. In the invention, these subunits or building blocks are immunoglobulin single variable domains (ISVs), such as Nanobodies. The first ISV of said bispecific polypeptide binds to a first target on the surface of a cell, but should have --counter-intuitively-- a low affinity for its target, which renders this ISV essentially inactive in the absence of additional binding to a cell marker. If bound to the target, the first ISV inhibits the function thereof, such as a cell surface receptor involved in the malignant process (functional ISV). However, said first ISV will only effectively bind to its target, when it is supported by the second ISV (anchoring ISV). The second ISV of said bispecific polypeptide binds with a high affinity to a second target on the surface of a cell, which is different from the first target (anchoring ISV). If bound to the target, the anchoring ISV preferably inhibits the function thereof to a limited amount, if at all. Although the first target can be present on normal cells, the low affinity of the functional ISV, and consequently absence of binding, the function of normal cells will not or only minimally be impaired. Preferably, the function of the normal cells is also impaired marginally by binding of the anchoring ISV, since this anchoring ISV is specifically developed to minimalize its impact on the normal function of the second target. Only in case of cells expressing both targets, the anchoring ISV binds with high affinity and thereby enables the functional ISV to effectively disturb the function of the first target. This concept not only increases the specificity for the diseased cell, e.g. a cancer cell, thereby decreasing toxicity and side-effects, it also widens the number of possible targets.

The concept is a broadly useful. However, before this concept could be validated, various practical problems had to be overcome by the present inventors.
(1) As set out above, in general antibodies are screened for highest affinity, while the low affinity binders will be discarded. In this case, not only low affinity binders are required, these low affinity binders must at the same time hinder the function of its target when bound. Since binding is not straightforward, testing its function is challenging.
(2) On the anchoring arm, it must further be ascertained and tested that the high affinity binders have only a minimal impact on the function of its target.
(3) It must be established that the combined interaction of the two building blocks, e.g. ISVs, in a bispecific format is correctly read-out, differentiating from a possible additive effect of each individual binder.

The present inventors overcame these problems by *inter alia* devising specific screening methods as will become clear further in the application.

In order to validate the generality of the concept the present inventors used the selective targeting of leukemic stem cells in AML as a test case, mainly for three reasons.

First, acute myelogenous leukemia (AML) provides the targets necessary to demonstrate the feasibility of the concept as such, since the targets are also ubiquitously expressed on normal cells. Second, it is hard to specifically bind to the chosen targets, since they are part of large families of related receptors and thus difficult to differentiate from each other. Hence, if the feasibility of the concept is demonstrated with the chosen targets, it can be safely assumed that the concept works with other targets as well. Furthermore, there is a clear medical need in AML.

After demonstrating the feasibility of the concept in AML, the inventors further corroborated the broad generality of the concept using other formats, including combinations of non-related targets, for which the co-localisation in the cell membrane is unknown. Enhanced tumour selectivity was shown with anti-CEA anchoring ISVs and anti-EGFR functional ISVs. The concept is not only applicable in the cancer field but in all fields in which specificity and selectivity of the target cell versus a normal cell is a problem (see supra). Indeed, the inventors demonstrated a potency in crease of 150-fold in HIV inhibition using anti-CD4 functional ISVs and anti-CXCR4 anchoring ISVs. Another area where bispecific targeting can be readily employed is in the preferential blockade or engagement of subsets of normal cells. As an example, being able to specifically modulate inflammatory and immune pathways only on specific T cell subsets (i.e. those relevant to the disease process) could provide greater efficacy and lesser toxicities. It was demonstrated that also different but closely related T-cell subsets involved in inflammation can be specifically blocked by this approach, i.e. ISVs against the interleukin receptors 12 (IL-12R) for T_{H1} cells, and interleukin 23 receptor 23 (IL-23R) for T_{H17} cells were used as functional ISVs and an anti-CD4 ISV was used as an anchoring ISV.

### Increasing specificity and selectivity in targeting tumor cells ^{∼} AML

Leukemia is a malignant disease of the bone marrow and blood that is characterized by the uncontrolled accumulation of white blood cells. Leukemia is classified as either myelogenous or lymphocytic, according to the type of cell involved (myeloid precursor cells or T and B lymphocytes, respectively). Leukemia is furthermore classified as either chronic or acute, based on the clinical presentation and course. Acute leukemia is a rapidly progressing form of the disease that results in the accumulation of immature, functionless cells (blasts) in the blood, bone marrow and tissues. The marrow often can no longer produce enough normal red blood cells, white blood cells and platelets, leading to anemia, reduced ability to fight infections, and easy bruising and bleeding. Chronic leukemia progresses more slowly and allows a greater number of functional, more mature cells to be produced. The diagnosis of leukemia requires a blood test, bone marrow biopsy and, in some instances, lumbar puncture. Histology, flow cytometry (immunophenotyping), cytochemistry and cytogenetics (DNA analysis) of the bone marrow and/or blood are used to determine the exact type and subtype of leukemia. There are four main types of leukaemia: (1) Acute lymphocytic leukemia (ALL, also known as acute lymphoid leukemia or acute lymphoblastic leukemia); (2) Chronic myelogenous leukemia (CML, also referred to as chronic granulocytic leukemia, chronic myelocytic leukemia or chronic myeloid leukemia); (3) Chronic lymphocytic leukemia (CLL, also called chronic lymphoid leukemia). Hairy cell leukemia (HCL) is a rare type of chronic lymphoid leukemia; and (4) Acute myelogenous leukemia (AML, also known as acute myelocytic leukemia, acute myeloblastic leukemia, acute granulocytic leukemia or acute non-lymphocytic leukemia). The hallmarks of AML are an abnormal proliferation of myeloid progenitor cells ("blasts") in bone marrow, reduced rate of self-destruction and arrest in cellular differentiation. When the blast cells lose their ability to differentiate in a normal fashion and to respond to normal regulators of cell proliferation, the result is frequent infections, bleeding and organ infiltration. The leukemic cells are endowed with an abnormal survival advantage with respect to normal healthy cells, such that the bone marrow and peripheral blood become increasingly populated by immature blast cells that edge out normal blood cells. AML is the most common malignant myeloid disorder in adults. In the U.S. during the year 2009: AML: 12,810 new cases (approximately 90% in adults); ALL: 5,760; CML: 5,050; CLL: ^{∼}15,490 new cases; other leukemias: 5,680. The median age at presentation is 70 years, and the disease affects more men than women although pediatric AML is not uncommon. The current treatment is aggressive chemotherapy. There is a complete remission in 50-80 % of the patients, yet frequent minimal residual disease and relapse. Autologous or allogeneic stem cell transplantation is required to restore immunity. AML is associated with the lowest survival rate of all leukemias. The 5 year survival rate for patients under 60 years is 30%, while the 5 year survival rate for patients over 65 years is less than 10%. Hence, there is a clear medical need.

AML is assumed to originate from CD34+CD38- immature leukemic stem cells (LSC) that reside in the bone marrow. Only CD34+CD38- blasts or LSCs are capable of engrafting and establishing AML in NOD/SCID mice. The CD34+CD38- LSC in the bone marrow can evade chemotherapy-induced death. Stromal cells can protect AML cells from chemotherapy-induced apoptosis. Accordingly, therapy is only successful if able to eliminate AML leukemic stem cells in the bone marrow (BM).

Hence, selective and effective targeting of human AML LSCs requires cell surface antigens that are preferentially expressed on AML LSC compared with normal hematopoietic stem cells, including CD123, CD44, CLL-1, CD96, CD47, CD32, CXCR4, Tim-3 and CD25. Monoclonal antibodies (mAbs) targeting CD44, CD123, and CD47 have demonstrated efficacy against AML LSC in xenotransplant models.

The existence of LSCs is a subject of debate within medical research, because many studies have not been successful in discovering the similarities and differences between normal tissue stem cells and cancer stem cells. Tumor stem cells are proposed to persist in tumors as a distinct population and cause relapse and metastasis by giving rise to new tumors. Therefore, development of specific therapies targeted at CSCs (Cancer stem cells) holds hope for improvement of survival and quality of life of cancer patients, especially for sufferers of metastatic disease.

The first conclusive evidence for cancer stem cells was published in 1997 in Nature Medicine. Bonnet and Dick isolated a subpopulation of leukaemic cells that express a specific surface marker CD34, but lack the CD38 marker. The authors established that the CD34⁺/CD38⁻ subpopulation is capable of initiating tumors in NOD/SCID mice that is histologically similar to the donor. Further evidence comes from histology, the study of the tissue structure of tumors. Many tumors are very heterogeneous and contain multiple cell types native to the host organ. Heterogeneity is commonly retained by tumor metastases. This implies that the cell that produced them had the capacity to generate multiple cell types. In other words, it possessed multi-differentiative potential, a classical hallmark of stem cells. As LSCs would form a very small proportion of the tumor, this may not necessarily select for drugs that act specifically on the stem cells. In human acute myeloid leukemia the frequency of these cells is less than 1 in 10,000. The theory suggests that conventional chemotherapies kill differentiated or differentiating cells, which form the bulk of the tumor but are unable to generate new cells. A population of LSCs, which gave rise to it, could remain untouched and cause a relapse of the disease.

In the current work the model antigens CD123 and CXCR4 have been used. Although to our current knowledge the co-expression of CD123 and CXCR4 on CD34+/CD38- AML LSCs has not been reported, there are numerous studies reporting the expression of either of these antigens in AML LSCs.

Expression of CD123 has been demonstrated on AML blasts, as well as on the CD34+/CD38-subpopulation in different AML patients. It is often expressed in conjunction with CD34 in other leukemias, for instance, B acute lymphoblastic leukemia (B-ALL). The blasts in 91% of B-ALL patients expressed both antigens, whereas 11% expressed neither. In contrast, the bone marrow normal B-cell precursors were found to express either CD123 or CD34, but not the combination. (Hassanein et al. 2009, Am J Clin Pathol 2009 Oct;132(4):573-80: Distinct expression patterns of CD123 and CD34 on normal bone marrow B-cell precursors ("hematogones") and B lymphoblastic leukemia blasts).

Similarly, CXCR4 expression has been demonstrated on AML blasts as well as on CD34+/CD38- AML LSCs, but it is also expressed in normal haematopoietic stem cells. The CXCR4 inhibitor Plerixafor has been found to be a strong inducer of mobilization of hematopoietic stem cells from the bone marrow to the bloodstream as peripheral blood stem cells. Peripheral blood stem cell mobilization, which is important as a source of hematopoietic stem cells for transplantation, is generally performed using G-CSF, but is ineffective in around 15 to 20% of patients. Combination of G-CSF with Plerixafor increases the percentage of persons that respond to the therapy and produce enough stem cells for transplantation. The drug is approved for patients with lymphoma and multiple myeloma, and early stage clinical studies for use of Plerixafor in AML are on going.

A bispecific Nanobody that inhibits the CXCR4 only in the CXCR4/CD123 combination context would have the potential to target selectively LSC for release from the bone marrow into the periphery where they become accessible for standard chemotherapy in setting of post-remission therapy in AML patients. Normal haematopoietic stem cells and progenitor cells and normal white blood cells which do not express CD123 (or only at very low levels) would not be affected.

The G-protein coupled receptor (GPCR) CXCR4 and its ligand stromal derived factor-1 (SDF-1/CXCL12) are important players involved in cross-talk between leukemia cells and the bone marrow (BM) microenvironment. CXCR4 expression is associated with poor prognosis in AML patients with and without the mutated FLT3 gene. CXCL12, which is constrictively secreted from the BM stromal cells and AML cells, is critical for the survival and retention of AML cells within the BM. In vitro, CXCR4 antagonists were shown to inhibit the migration of AML cells in response to CXCL12. In addition, such antagonists were shown to inhibit the survival and colony forming potential of AML cells and abrogate the protective effects of stromal cells on chemotherapy-induced apoptosis in AML cells. In vivo, using immune deficient mouse models, CXCR4 antagonists were found to induce the mobilization of AML cells and progenitor cells into the circulation and enhance anti-leukemic effects of chemotherapy. Despite GPCRs representing one of the major pharmaceutical targets, it is surprising that the clinical practice of cancer treatment includes only a few drugs that act on GPCR-mediated signaling. Notwithstanding the recognition that GPCRs can act as oncogenes and tumour suppressors by regulating oncogenic signalling networks, few drugs targeting GPCRs are utilized in cancer therapy. Among the sporadic examples is the gold standard of endocrine treatment for hormone responsive prostate and breast cancers.

The present inventors therefore designed a CXCR4-IL3Ra bispecific antibody. This bispecific antibody has the potential to target selectively LSC, since IL3Ra (also known as CD123) is a marker for LSC. Normal HSC and HPG and normal white blood cells would not be affected by the CXCR4 Nanobody, since these cells do not or only weakly express CD123.

In an initial *in vitro* Proof of Concept study that AML cell lines with different endogeneous expression levels of CXCR4 and CD123 were used for testing the potencies of CXCR4-CD123 bi-specifics. The bi-specific polypeptides were tested for potencies in a CXCR4-dependent chemotaxis assay, comparing cell lines expressing only CXCR4 or CXCR4 with the second receptor. An unprecedented 15-150 increase in potency of the bi-specific polypeptides was measured compared to the monovalent CXCR4 Nanobody, but only on the cells that express both targets. There was a clear effect of the position of the CXCR4 Nanobody in the bi-specific polypeptide, and the selective potency increase was only observed for the CXCR4 Nanobody with the lower affinity.

Although this concept was tested with two distinct CXCR4 Nanobodies, with different epitopes and affinities, only combinations with a CXCR4 Nanobody of lower potency (65 nM as monovalent) showed enhancement. This would indicate that the affinity is a critical parameter.

Moreover, changing to a completely different anchor using a CD4 Nanobody of the same affinity (1 nM) resulted in a potency increase of 150-fold. Since the expression levels of the CD4 anchor were much higher than CD123 on the same cells, it appears that the relative expression levels of anchor to functional target may be a further determinant for the level of enhancement achieved.

### Increasing specificity and selectivity in targeting tumor cells ^{∼} EGFR

The epidermal growth factor receptor (EGFR) is a member of the ErbB tyrosine kinase receptor that is expressed in many normal human cells of epithelial origin, playing an important role in cell growth, differentiation, and proliferation. In the skin it is normally expressed in the epidermis, sebaceous glands, and hair follicular epithelium, where it plays a number of important roles in the maintenance of normal skin health. It is often overexpressed or dysregulated in a variety of solid tumours, including gastrointestinal malignancies. Dysregulated EGFR may result in uncontrolled cell growth, proliferation, and angiogenesis, and is associated with a poorer prognosis, manifested by increased metastatic potential and poorer overall survival.

EGFR has been demonstrated to be involved in tumor growth, metastasis and angiogenesis. Further, many cancers express EGFR, such as bladder cancer, ovarian cancer, colorectal cancer, breast cancer, lung cancer (e.g., non-small cell lung carcinoma), gastric cancer, pancreatic cancer, prostate cancer, head and neck cancer, renal cancer and gall bladder cancer. Agents targeting the EGFR-mediated signaling pathway are increasingly part of the therapeutic tools for the treatment of advanced lung, head-and-neck, and colorectal carcinoma. The EGFR inhibitors approved in Europe include the mAbs panitumumab and cetuximab, and the tyrosine kinase inhibitors erlotinib and gefitinib. Although these drugs have been proven effective in the treatment of a variety of malignancies, the entire class of EGFR agents is associated with a high prevalence of dermatologic side-effects, most commonly skin rash, and a high rate of patient discontinuation due to toxicity. This reversible condition requires intervention in approximately one third of patients. Skin rash has been reported in 80%-90% of patients with colorectal cancers treated with EGFR-targeted mAbs. In the clinical setting, up to 32% of physicians have reported discontinuing, and 76% have reported holding EGFR treatment because of skin toxicity (Melosky et *al.* 2009). In addition to the target-related toxicity, due to high EGFR expression in liver and other normal tissues, the administrated dose is high, as the antibodies are first saturating the normal tissues. Targeting EGFR with currently available therapeutics is not effective in all patients, or for all cancers (e.g., EGFR-expressing cancers). Thus, a need exists for improved agents for treating EGFR-expressing cancer and other EGFR-related pathological conditions.

As a second, anchoring target, carcinoembryonic antigen (CEA, also known as CEACAM5) was used. CEA is a well-known tumour specific antigen expressed on many tumour types. It is an established tumour-associated marker for gastrointestinal tract cancers, also found in breast and lung cancers. CEA is a glycosylphosphatidylinisotol (GPI)-anchored cell surface glycoprotein that plays a role in cellular adhesion. A soluble form is increased in the serum in cancer, and is used as a biomarker (normal serum CEA levels ≤ 5 ng/mL; elevated CEA levels > 5 ng/mL). CEA expression is restricted to primates, and expression is low in normal tissue, in which expression can reach 60 times higher levels in tumour than that in healthy tissues. However, CEA is shed by phospholipases from the cell surface through cleavage of its GPI-linkage, which causes the protein to be released in circulation, acting as a sink.

Co-expression of EGFR and CEA has been reported for gastric and colorectal cancers, in primary tumours and in peritoneal metastasis, with in most cases higher membrane expression of CEA than EGFR (Ito *et al.* 2013, Tiernan et *al.* 2013). This makes CEA a useful target to serve as anchor for combining with EGFR for functional blockade in a tumour-selective manner.

Since the avidity increase relies on two membrane proteins expressed on the same cell, the soluble CEA is not expected to act as sink for the bi-specific CEA Nanobody.

We have also in this case demonstrated potency enhancements with bispecific polypeptides for the EGFR and CEACAM5 target combination, exclusively on cells that co-express both receptors.

### Increasing specificity and selectivity in targeting T cell subsets in inflammation

T cell-mediated immunity is an adaptive process of developing antigen (Ag)-specific T lymphocytes to eliminate viral, bacterial or parasitic infections, or malignant cells. T cell-mediated immunity can also involve aberrant recognition of self-Ag, leading to autoimmune inflammatory diseases. T cell-mediated immunity is the central element of the adaptive immune system and includes a primary response by naive T cells, effector functions by activated T cells, and persistence of Ag-specific memory T cells. IL-12 is involved in the differentiation of naive T cells into Th1 cells. IL-23 induces the differentiation of naive CD4+ T cells into highly pathogenic helper T cells.

The IL-23 and IL-12 receptors belong to the same cytokine receptor family. Both receptors are heterodimers, of which both subunits are required for high-affinity binding of the ligand and activity. The IL12Rβ1 is the common receptor shared by both heterodimers, and binds both IL-12 and IL-23 via the shared 40 subunit. The IL12Rβ2 binds specifically to IL-12 p35 subunit, and hence is specific for the IL-12R. Similarly, IL-23R is the specific subunit binding to the p39 subunit of IL-23. IL-12 and IL-23 cytokines respectively drive Th1 and Th17 type responses. The expression of each of these receptors is restricted to specific cell types, in both mouse and human. While IL12Rβ2 is expressed by NK cells and a subset of T cells, the expression of IL-23R is restricted to specific T cell subsets, a small number of B cells and innate lymphoid cells.

IL-23 contributes to chronic inflammation by inducing the production of IL-17 by memory T cells. Inflammation mediated by Tₕ₁₇ cells has been identified in several human organs or tissues, including the eye, brain, skin, liver, colon, kidney, testes, joint, and lung. Numerous cytokines induced by activated Tₕ₁₇ cells, such as IL-22, IL-17, IFN-γ, TNF-α, and IL-6, play essential roles during the inflammatory diseases. These cytokines lead to the onset of the uveitis, autoimmune encephalomyelitis, psoriasis, hepatitis, inflammatory bowel disease, nephritis, testitis, rheumatic arthritis, and asthma.

We have demonstrated that CD4-IL-12Rβ2 and CD4-IL-23R bispecific polypeptides show selective functional blockade in a T cell subset-specific manner, in assays with heterogeneous T cells as well as PBMCs. Furthermore, selective binding of the bispecific polypeptides to CD4+ T cell subsets was shown, whereas monovalent IL12Rβ2 Nanobodies showed only poor binding to CD4+ and CD8+ T cells.

With respect to affinities, even very low affinity Nanobodies on the functional arm gave potency enhancements upon formatting with a high affinity anchoring CD4 Nanobody. Although cell binding could not always be accurately measured for Nanobodies with fast off-rates (>1.E-02), ligand competition demonstrated functional blocking with IC50 ranging between 10-16 nM.

### Increasing specificity and selectivity in targeting HIV ∼ CXCR4 and CD4

Infection with the Human Immunodeficiency Virus (HIV), if left untreated, almost always leads to death of the infected person. HIV infects the CD4⁺ T-cells and leads to a decline in the number of CD4⁺ T-cells in the infected person. When CD4⁺ T cell numbers decline below a critical level, cell-mediated immunity is effectively lost, and infections with a variety of opportunistic microbes appear, resulting in Acquired Immunodeficiency Syndrome (AIDS). Because the HIV-infected person can no longer defend against these opportunistic infections, the patient will ultimately succumb to one of these infections.

There currently is no cure available for HIV / AIDS. However, HIV infected persons can suppress proliferation of the virus through a variety of anti-viral treatment options. Current treatment for HIV infection consists of Highly Active AntiRetroviral Therapy, or HAART. HAART consists of the administration of a cocktail of multiple antiviral compounds. However, because HIV readily mutates the virus often becomes resistant to one or more compounds in the HAART cocktail. In addition, HAART is associated with a number of side effects. New therapies to treat HIV infection are needed therefore.

A critical event during HIV-infection is entry of HIV into CD4⁺ T-cells. Once the virus has entered the T-cells, the virus hijacks the replication machinery of the T-cell to produce additional copies of HIV thereby furthering the infection. Precluding the entry of HIV into CD4⁺ T-cells provides an important therapeutic option for the treatment and prevention of HIV infection.

HIV has the ability to mutate frequently and has been shown to be able to "out-mutate", and become resistant to, a number of antiviral treatment regimes, including regimes that are targeted towards the inhibition of HIV proteases and HIV reverse transcriptases. Interestingly, the options for HIV to "mutate around" therapies directed at blocking cell entry are more limited. If a cell entry point (*e.g.,* CXCR4) is blocked by an agent (*e.g.,* a blocking antibody) thereby preventing HIV from binding, the virus cannot readily mutate to find another point of entry. In addition, the virus cannot readily mutate to remove the agent (*e.g*., the blocking antibody). However, a challenge in therapies based on preventing HIV from entering the cells is that the receptors used by HIV for cell entry have a "natural" function as well. Administering a binding agent that prevents HIV from binding may result, for instance, in a receptor that is constitutively activated or in a receptor that cannot be activated because a natural ligand to the receptor is precluded from binding. The immunoglobulin single variable domain and polypeptide constructs thereof that are disclosed herein overcome this challenge because, while they inhibit HIV from binding CXCR4, they do not prevent binding of a natural ligand to CXCR4 (anchoring ISV). While not being limited to a specific mechanism, it is presumed that the immunoglobulin single variable domain and polypeptide constructs thereof have this ability because they selectively bind CXCR4 at a site of binding of HIV, and do not bind at the site where the natural ligand binds.

HIV enters CD4⁺ T-cells by binding of glycoproteins, such as gp120, on the surface of the HIV capsid to receptors on the CD4⁺ T cells followed by fusion of the viral envelope with the cell membrane and the release of the HIV capsid into the cell. HIV binds to the CD4⁺ cell by binding of gp120 to CD4 and a chemokine receptor, either CXCR5 or CXCR4, on the cell surface. Once gp120 is bound to the CD4 protein, the envelope complex undergoes a structural change, exposing the chemokine binding domains of gp120 and allowing them to interact with the target chemokine receptor. This two-pronged attachment of gp120 to the CD4⁺ T-cell brings the virus and cell membranes close together, allowing fusion of the membranes and subsequent entry of the viral capsid into the cell. Thus, preventing HIV from binding gp120, CXCR4 or CXCR5 provides a powerful strategy to treat infection by HIV and to prevent infection by HIV.

The inventors showed that simultaneous binding to both CXCR4 and CD4 of the bispecific CXCR4-CD4 polypeptides results in strongly enhanced potencies in the neutralization of CXCR4-using HIV1 . Because of its selectivity, the bispecific Nanobody can be administered safely over a longer time, leading to an improved treatment.

### BRIEF DESCRIPTION OF THE FIGURES

- Figure 1.1:: Schematic representation of the model system.
- Figure 1.2:: Binding of anti-IL-3Ra Nanobodies to cell-expressed IL-3Ra
- Figure 1.3:: Binding of CXCR4 Nanobodies to different CXCR4 expressing cell lines (A, B), and ligand displacement for CXCR4 binding (panel C, D) (14E2 = 14E02).
- Figure 1.4:: Binding of CXCR4-IL-3Ra bispecific polypeptides to CXCR4 -VLPs and recombinant IL-3Ra ectodomain
- Figure 1.5:: Antigen expression levels of CXCR4 an IL-3Ra on the distinct cell lines as determined by FACS with monoclonal antibodies anti-CXCR4 12G5 and anti-IL-3Ra 7G3, respectively.
- Figure 1.6:: Binding of bispecific CXCR4-IL-3Ra Nanobodies to cells with different relative expression levels of the two receptors. Representative examples of bispecific polypeptides of CXCR4 Nanobody 281F12 and 14D09 are depicted.
- Figure 1.7:: MCF signals for the binding of the Nanobody® constructs at 4.6nM to Jurkat E6-1 and MOLM-13 cell lines. X indicates anti-CXCR4 building block, I indicates anti-IL3Ra building block, X-I indicates anti-CXCR4 at N-terminal and anti-IL3Ra at C-terminal, I-X indicates the reversed orientation.
- Figure 1.8:: Titration of different monovalent and bispecific CXCR4-IL3Ra polypeptides in a CXCL-12 induced chemotaxis assay on Jurkat E6-1 and MOLM-13 cell lines. 14D09 and 281F12 are anti-CXCR4 building blocks; 55A01 and 57A07 are anti-IL3Ra building blocks.
- Figure 2.1:: Binding characteristics of monovalent CD4 Nanobodies.
- Figure 2.2:: Binding of the monovalent and bispecific CD4-CXCR4 Nanobodies to CXCR4 on viral lipid particles (CXCR4-lip) versus empty control particles (null-lip) in ELISA.
- Figure 2.3:: Binding analysis of selected bispecific CXCR4-CD4 polypeptides to cell-expressed CXCR4 expressed on Jurkat E6.1 cells, and to CXCR4 and CD4-coexpressing THP-1 and MOLM-13 cells. Bispecifics polypeptides with the 35GS linker were used. Detection was done via anti-tag antibodies.
- Figure 2.4:: Inhibition of SDF-1 mediated chemotaxis of CXCR4-CD4 bispecific polypeptides to Jurkat E6.1 and Molm-13 cells. Bispecific CXCR4#2-CD4#8 polypeptides with the 35GS-linkers are shown.
- Figure 2.5:: Inhibition of HIV1 entry by CXCR4-CD4 Nanobodies of wild-type NL4.3 and AMD3100-resistant HIV1 variants in MT-4 cells.
- Figure 3.1:: The expression levels of IL12Rβ1, IL23R, and CD4 on activated T cells towards the T_{H1} phenotype were determined with control IL-12Rβ1 antibody, polyclonal IL-23R antibody, followed by secondary anti-mouse PE, anti-goat PE, and APC labeled CD4 antibodies.
- Figure 3.2:: Binding of IL23R (panel A), IL12Rβ1 (panel B) and CD4 Nanobodies (panel C) to T cells differentiated towards the T_{H17} phenotype by flow cytometry. Activated T-cells were differentiated within PBMC mixture towards Th17 cells in the presence of cytokine cocktail and recombinant IL-23.
- Figure 3.4:: Overview of panel of CD4-IL12Rβ2, CD4-Il12Rβ1 and CD4-IL23R bispecifics.
- Figure 3.5:: Dose response curves of the bispecific and monovalent IL12R and IL23R Nanobodies on MOLM-13 cells in FACS. CD4 expression levels on MOLM-13 cells. (US, unstained, a-CD4, detection using anti-human CD4 APC.
- Figure 3.6:: Dose response curves of the bispecific CD4-IL12Rβ2, CD4-IL12Rβ1, and CD4-IL23R polypeptides compared to their respective monovalent Nanobodies on activated T cells in FACS.
- Figure 3.7:: Binding analysis of monovalent Nanobodies and bispecific polypeptides to isolated CD8+ T cells. As irrelevant control Nanobody Cablys3 is used. Detection was done via anti-Flag detection. Onset shows the expression levels of T cell markers with control antibodies for CD3 and CD8, respectively, after isolation of CD8 positive cells from human buffycoats.
- Figure 3.8:: Nanobody binding to T_{H1} activated cells gated for CD8+ (dark grey) or CD4+ (light grey) in a multi-colour FACS experiment. Nanobody binding was determined using anti-flag-APC detection.
- Figure 3.9:: Blockade of IL-12 induced cytokine production function in human T cells by bispecific polypeptides and monovalent Nanobodies. Panel A - C; IL-12 Titration Panel D, B, D etc.

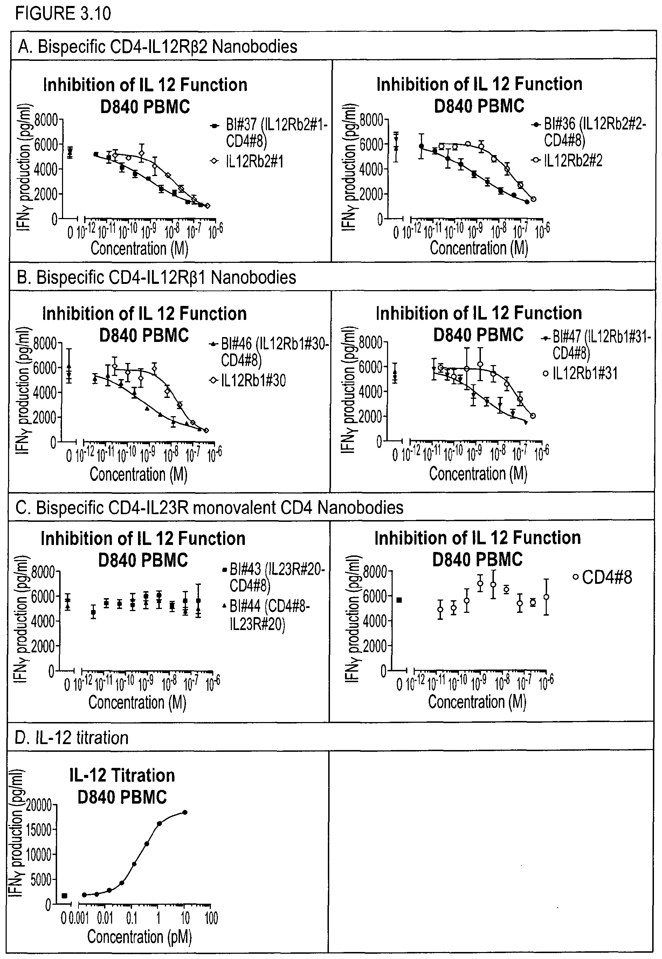

- Figure 3.10:: Inhibition of IL-12 dependent IFN-γ secretion by monovalent Nanobodies and bispecific polypeptides in human PBMCs. Representative graphs obtained with T cells from one donor are shown.

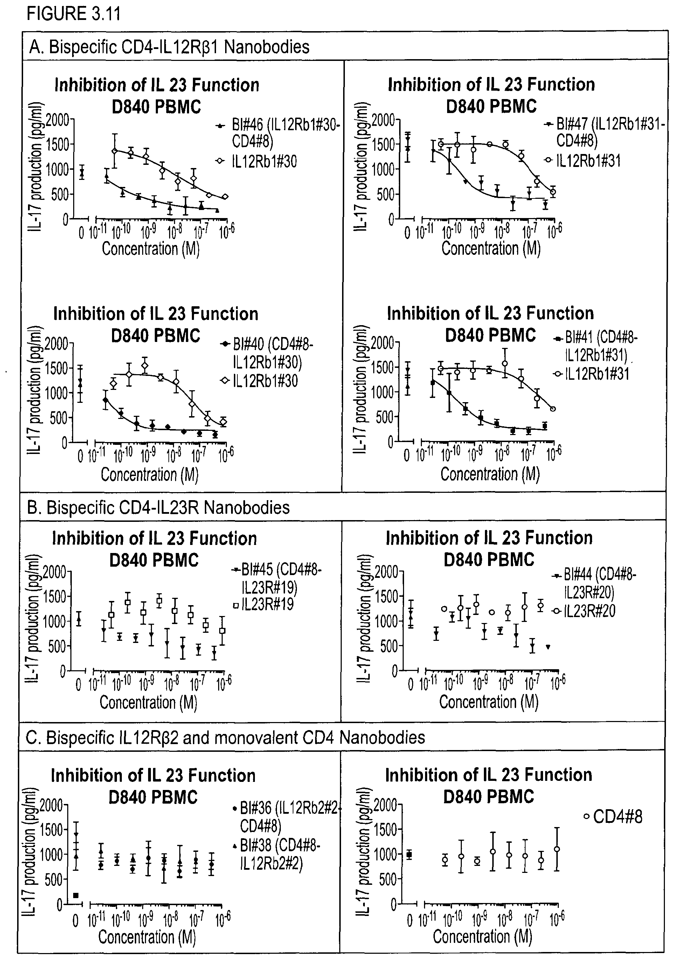

- Figure 3.11:: Inhibition of IL-23 dependent IL-17 secretion by monovalent Nanobodies and bispecific polypeptides in human PBMCs.

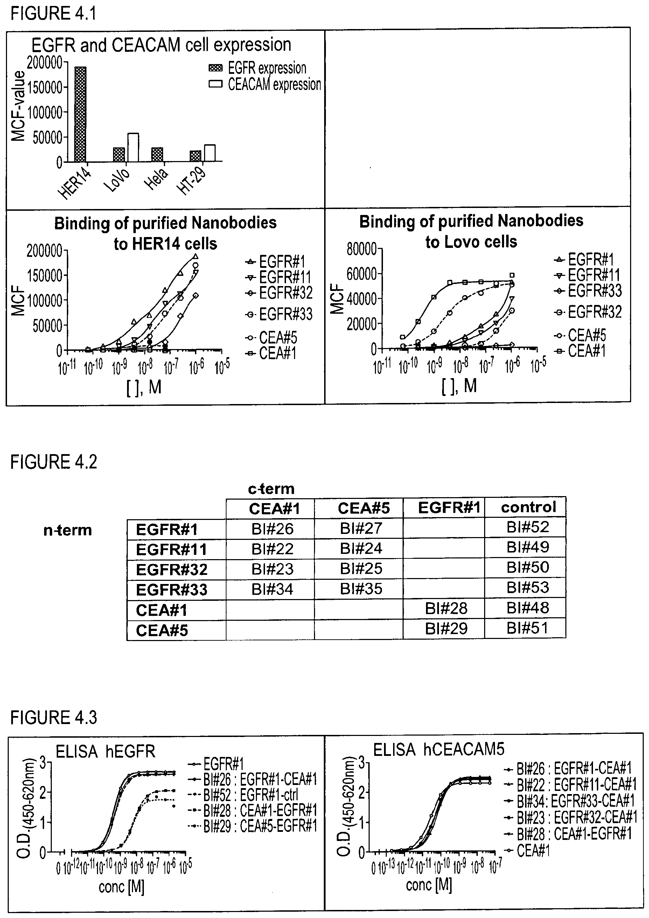

- Figure 4.1:: Binding analysis of monovalent Nanobodies to HER-14 cells expressing only EGFR, and LoVo cells expressing both EGFR and CEACAM5 determined by flow cytometry via anti-Flag tag detection. The expression of EGFR and CEACAM5 on Lovo, HT-29, HeLa and Her14 cells detected by polyclonal Anti-Human EGF R-PE the Anti-Human CEACAM5 / CD66e Antibody (PE) respectively.
- Figure 4.2:: Overview of generated EGFR-CEA bispecific polypeptides and monospecific Nanobodies.
- Figure 4.3:: Effect of formatting into bispecific EGFR-CEA polypeptides on target binding by ELISA on recombinant EGFR or CEACAM5, respectively. Binding was detected via anti-flag-HRP secondary antibodies.

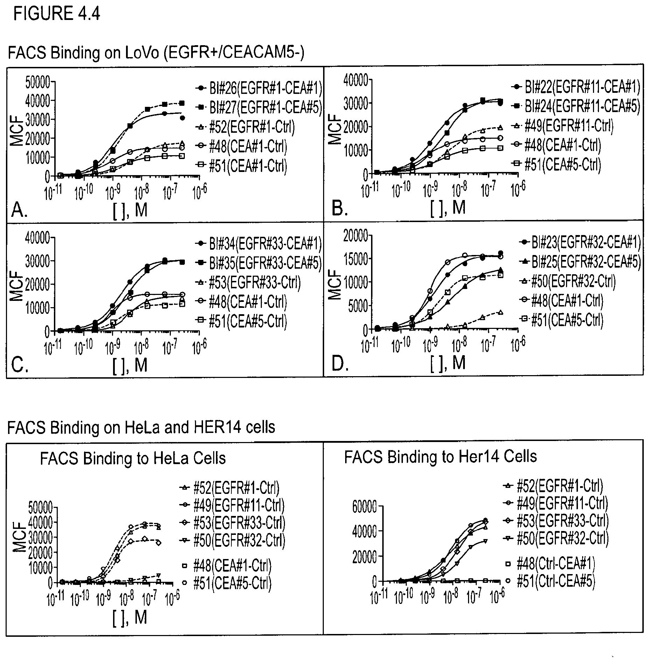

- Figure 4.4:: Binding analysis of the monospecific Nanobodies and bispecific polypeptides on EGFR+/CEA- HER-14 cells and EGFR+/CEA+ LoVo cells by flow cytometry.

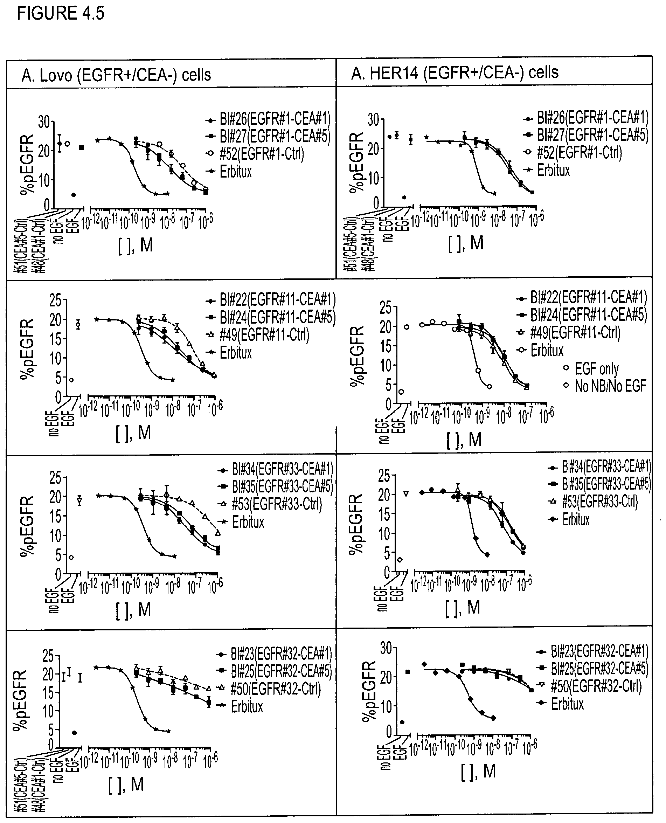

- Figure 4.5:: Dose-dependent inhibition of EGF-mediated EGFR tyrosine phosphorylation by bispecific polypeptides and monospecific Nanobodies on EGFR+/CEA+ LoVo cells and EGFR+/CEA- Her14 cells. Data indicate average values of duplicates+Stdev.

### DESCRIPTION OF THE INVENTION

Immunoglobulin sequences, such as antibodies and antigen binding fragments derived there from (*e.g.*, immunoglobulin single variable domains or ISVs) are used to specifically target their respective antigens in research and therapeutic applications. The generation of immunoglobulin single variable domains such as *e.g*., VHHs or Nanobodies may involve the immunization of an experimental animal such as a Llama, construction of phage libraries from immune tissue, selection of phage displaying antigen binding immunoglobulin single variable domains and screening of said domains and engineered constructs thereof for the desired specificities (WO 94/04678). Alternatively, similar immunoglobulin single variable domains such as *e.g*., dAbs can be generated by selecting phage displaying antigen binding immunoglobulin single variable domains directly from naive or synthetic libraries and subsequent screening of said domains and engineered constructs thereof for the desired specificities (Ward et al., Nature, 1989, 341: 544-6; Holt et al., Trends Biotechnol., 2003, 21(11):484-490; as well as for example WO 06/030220, WO 06/003388 and other published patent applications of Domantis Ltd.). Unfortunately, the use of monoclonal and/or heavily engineered antibodies also carries a high manufacturing cost and may result in suboptimal tumor penetration compared to other strategies.

The "polypeptide of the invention" as used herein is as defined by the claims. Specifically, the invention provides:

A polypeptide comprising a first and a second immunoglobulin single variable domain (ISV), wherein
- said first ISV binds to a first target with an average KD value of between 10 nM and 200 nM as measured by surface plasmon resonance;
- said second ISV binds to a second target with an average KD value of between 10 nM and 0.1 pM as measured by surface plasmon resonance; and
wherein said first ISV and said second ISV bind to said first target and said second target present on the surface of the same cell,
wherein said first target is different from said second target,
wherein said second ISV enhances binding of said first ISV,
wherein binding by said first ISV inhibits a function of said first target,,
wherein said first target is chosen from the group consisting of Receptor Tyrosine Kinases (preferably class I), GPCRs, DDR1, Discoidin I (CD167a antigen), DDR2, ErbB-1, C-erbB-2, FGFR-1, FGFR-3, CD135 antigen, CD 117 antigen, Protein tyrosine kinase-1, c-Met, CD148 antigen, C-ret, ROR1, ROR2, Tie-1, Tie-2, CD202b antigen, Trk-A, Trk-B, Trk-C, VEGFR-1, VEGFR-2, VEGFR-3, Notch receptor 1-4, FAS receptor, DR5, DR4, CD47, CX3CR1, CXCR-3, CXCR-4, CXCR-7, Chemokine binding protein 2, and CCR1, CCR2, CCR3, CCR4, CCR5, CCR6, CCR7, CCR8, CCR9, CCR10 and CCR11; and
said second target is chosen from the group consisting of carcinoembryonic antigen ("CEA"), MART-1, gp100, MAGE-1, HER-2, and Lewis^{Y} antigens, CD123, CD44, CLL-1, CD96, CD47, CD32, CXCR4, Tim-3, CD25, TAG-72, Ep-CAM, PSMA, PSA, GD2, GD3, CD4, CD5, CD19, CD20, CD22, CD33, CD36, CD45, CD52, and CD147; and Cytokine receptors including Interleukin-2 receptor gamma chain (CD132 antigen); Interleukin-10 receptor alpha chain (IL-10R-A); Interleukin-10 receptor beta chain (IL-10R-B); Interleukin-12 receptor beta-1 chain (IL-12R-beta1);
Interleukin-12 receptor beta-2 chain (IL-12 receptor beta-2); Interleukin-13 receptor alpha-1 chain (IL-13R-alpha-1) (CD213 al antigen); Interleukin-13 receptor alpha-2 chain (Interleukin-13 binding protein); Interleukin-17 receptor (IL-17 receptor); Interleukin-17B receptor (IL-17B receptor); Interleukin 21 receptor precursor (IL-21R); Interleukin-1 receptor, type I (IL-1R-1) (CD121a); Interleukin-1 receptor, type II (IL-1R-beta) (CDw121b); Interleukin-1 receptor antagonist protein (IL-1ra); Interleukin-2 receptor alpha chain (CD25 antigen); Interleukin-2 receptor beta chain (CD122 antigen); Interleukin-3 receptor alpha chain (IL-3R-alpha) (CD123 antigen).

The invention further provides a pharmaceutical composition comprising the polypeptide of the invention.

The invention further provides the polypeptide of the invention for use in a method for delivering a prophylactic or therapeutic polypeptide, a polypeptide-drug conjugate (PDC) or imaging agent to a specific location, tissue or cell type in the body, the method comprising the steps of administering to a subject a polypeptide of the invention;
or for use in treating a subject in need thereof.

### Definitions:

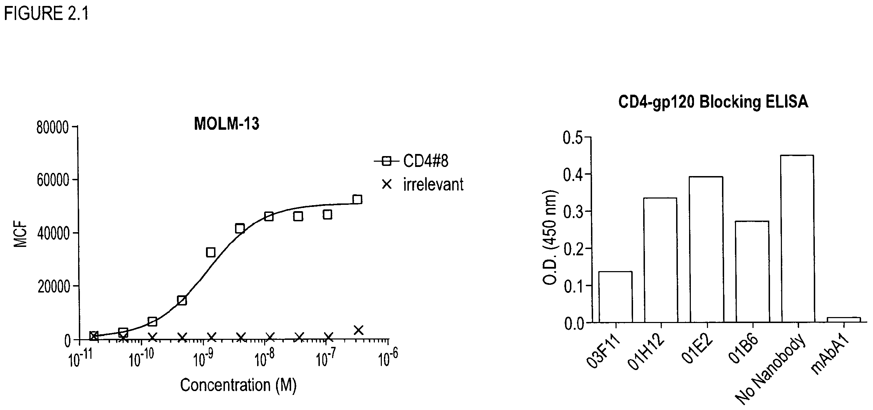

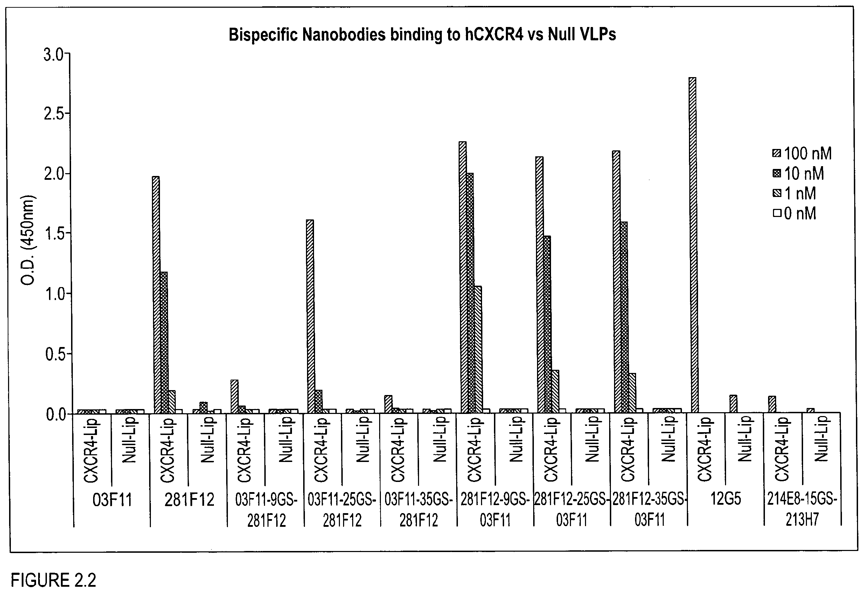

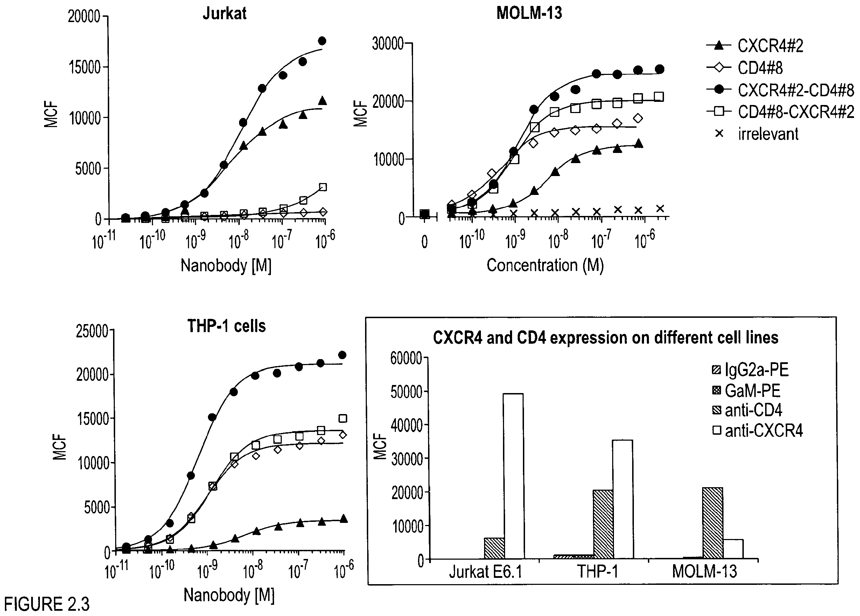

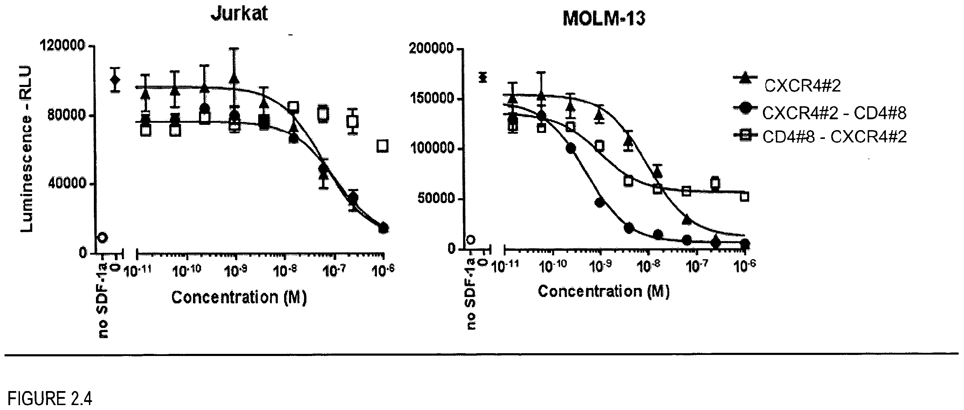

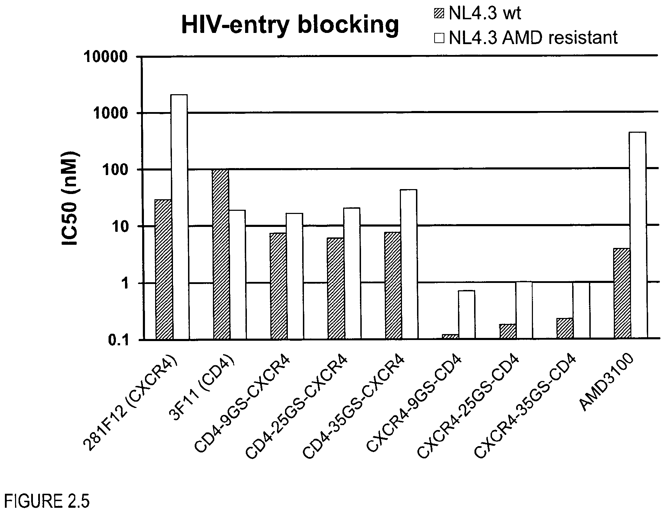

a) Unless indicated or defined otherwise, all terms used have their usual meaning in the art, which will be clear to the skilled person. Reference is for example made to the standard handbooks mentioned in paragraph a) on page 46 of WO 08/020079.
b) Unless indicated otherwise, the term "immunoglobulin single variable domain" or "ISV" is used as a general term to include but not limited to antigen-binding domains or fragments such as V_{HH} domains or V_{H} or V_{L} domains, respectively. The terms antigen-binding molecules or antigen-binding protein are used interchangeably and include also the term Nanobodies. The immunoglobulin single variable domains can be light chain variable domain sequences (e.g., a V_{L}-sequence), or heavy chain variable domain sequences (e.g., a V_{H}-sequence); more specifically, they can be heavy chain variable domain sequences that are derived from a conventional four-chain antibody or heavy chain variable domain sequences that are derived from a heavy chain antibody. Accordingly, the immunoglobulin single variable domains can be domain antibodies, or immunoglobulin sequences that are suitable for use as domain antibodies, single domain antibodies, or immunoglobulin sequences that are suitable for use as single domain antibodies, "dAbs", or immunoglobulin sequences that are suitable for use as dAbs, or Nanobodies, including but not limited to V_{HH} sequences. The invention includes immunoglobulin sequences of different origin, comprising mouse, rat, rabbit, donkey, human and camelid immunoglobulin sequences. The immunoglobulin single variable domain includes fully human, humanized, otherwise sequence optimized or chimeric immunoglobulin sequences. The immunoglobulin single variable domain and structure of an immunoglobulin single variable domain can be considered - without however being limited thereto - to be comprised of four framework regions or "FR's", which are referred to in the art and herein as "Framework region 1" or "FR1"; as "Framework region 2" or "FR2"; as "Framework region 3" or "FR3"; and as "Framework region 4" or "FR4", respectively; which framework regions are interrupted by three complementary determining regions or "CDR's", which are referred to in the art as "Complementarity Determining Region 1" or "CDR1"; as "Complementarity Determining Region 2" or "CDR2"; and as "Complementarity Determining Region 3" or "CDR3", respectively. It is noted that the terms Nanobody or Nanobodies are registered trademarks of Ablynx N.V. and thus may also be referred to as Nanobody® or Nanobodies®, respectively.
c) Unless indicated otherwise, the terms "immunoglobulin sequence", "sequence", "nucleotide sequence" and "nucleic acid" are as described in paragraph b) on page 46 of WO 08/020079.
d) Unless indicated otherwise, all methods, steps, techniques and manipulations that are not specifically described in detail can be performed and have been performed in a manner known per se, as will be clear to the skilled person. Reference is for example again made to the standard handbooks and the general background art mentioned herein and to the further references cited therein; as well as to for example the following reviews Presta, Adv. Drug Deliv. Rev. 2006, 58 (5-6): 640-56; Levin and Weiss, Mol. Biosyst. 2006, 2(1): 49-57; Irving et al., J. Immunol. Methods, 2001, 248(1-2), 31-45; Schmitz et al., Placenta, 2000, 21 Suppl. A, S106-12, Gonzales et al., Tumour Biol., 2005, 26(1), 31-43, which describe techniques for protein engineering, such as affinity maturation and other techniques for improving the specificity and other desired properties of proteins such as immunoglobulins.
e) Amino acid residues will be indicated according to the standard three-letter or one-letter amino acid code. Reference is made to Table A-2 on page 48 of the International application WO 08/020079 of Ablynx N.V. entitled *"Immunoglobulin single variable domains directed against IL-6R and polypeptides comprising the same for the treatment of diseases and disorders associated with II-6 mediated signalling".*
f) For the purposes of comparing two or more nucleotide sequences, the percentage of *"sequence identity"* between a first nucleotide sequence and a second nucleotide sequence may be calculated or determined as described in paragraph e) on page 49 of WO 08/020079, such as by dividing *[the number of nucleotides in the first nucleotide sequence that are identical to the nucleotides at the corresponding positions in the second nucleotide sequence*] by [*the total number of nucleotides in the first nucleotide sequence*] and multiplying by [*100%*], in which each deletion, insertion, substitution or addition of a nucleotide in the second nucleotide sequence - compared to the first nucleotide sequence - is considered as a difference at a single nucleotide (position); or using a suitable computer algorithm or technique, again as described in paragraph e) on pages 49 of WO 08/020079.
g) For the purposes of comparing two or more immunoglobulin single variable domains or other amino acid sequences such e.g. the polypeptides of the invention etc., the percentage of *"sequence identity"* between a first amino acid sequence and a second amino acid sequence (also referred to herein as *"amino acid identity*") may be calculated or determined as described in paragraph f) on pages 49 and 50 of WO 08/020079, such as by dividing [*the number of amino acid residues in the first amino acid sequence that are identical to the amino acid residues at the corresponding positions in the second amino acid sequence*] *by* [*the total number of amino acid residues in the first amino acid sequence*] and multiplying by [*100%*], in which each deletion, insertion, substitution or addition of an amino acid residue in the second amino acid sequence - compared to the first amino acid sequence - is considered as a difference at a single amino acid residue (position), *i.e.,* as an "amino acid difference" as defined herein; or using a suitable computer algorithm or technique, again as described in paragraph f) on pages 49 and 50 of WO 08/020079.
   Also, in determining the degree of sequence identity between two immunoglobulin single variable domains, the skilled person may take into account so-called "conservative" amino acid substitutions, as described on page 50 of WO 08/020079.
   Any amino acid substitutions applied to the polypeptides described herein may also be based on the analysis of the frequencies of amino acid variations between homologous proteins of different species developed by Schulz et al., Principles of Protein Structure, Springer-Verlag, 1978, on the analyses of structure forming potentials developed by Chou and Fasman, Biochemistry 13: 211, 1974 and Adv. Enzymol., 47: 45-149, 1978, and on the analysis of hydrophobicity patterns in proteins developed by Eisenberg et al., Proc. Natl. Acad. Sci. USA 81: 140-144, 1984; Kyte & Doolittle; J Molec. Biol. 157: 105-132, 1981, and Goldman et al., Ann. Rev. Biophys. Chem. 15: 321-353, 1986. Information on the primary, secondary and tertiary structure of Nanobodies is given in the description herein and in the general background art cited above. Also, for this purpose, the crystal structure of a V_{HH} domain from a llama is for example given by Desmyter et al., Nature Structural Biology, Vol. 3, 9, 803 (1996); Spinelli et al., Natural Structural Biology (1996); 3, 752-757; and Decanniere et al., Structure, Vol. 7, 4, 361 (1999). Further information about some of the amino acid residues that in conventional V_{H} domains form the V_{H}/V_{L} interface and potential camelizing substitutions on these positions can be found in the prior art cited above.
h) Immunoglobulin single variable domains and nucleic acid sequences are said to be *"exactly the same"* if they have 100% sequence identity (as defined herein) over their entire length.
i) When comparing two immunoglobulin single variable domains, the term *"amino acid difference"* refers to an insertion, deletion or substitution of a single amino acid residue on a position of the first sequence, compared to the second sequence; it being understood that two immunoglobulin single variable domains can contain one, two or more such amino acid differences.
j) When a nucleotide sequence or amino acid sequence is said to "comprise" another nucleotide sequence or amino acid sequence, respectively, or to "essentially consist of" another nucleotide sequence or amino acid sequence, this has the meaning given in paragraph i) on pages 51-52 of WO 08/020079.
k) The term "in essentially isolated form" has the meaning given to it in paragraph j) on pages 52 and 53 of WO 08/020079.
l) The terms "domain" and "binding domain" have the meanings given to it in paragraph k) on page 53 of WO 08/020079.
m) The terms "antigenic determinant" and "epitope", which may also be used interchangeably herein, have the meanings given to it in paragraph I) on page 53 of WO 08/020079.
n) As further described in paragraph m) on page 53 of WO 08/020079, an amino acid sequence (such as an antibody, a polypeptide of the invention, or generally an antigen binding protein or polypeptide or a fragment thereof) that can (specifically) bind to, that has affinity for and/or that has specificity for a specific antigenic determinant, epitope, antigen or protein (or for at least one part, fragment or epitope thereof) is said to be *"against"* or *"directed against"* said antigenic determinant, epitope, antigen or protein.
o) The term *"specificity"* refers to the number of different types of antigens or antigenic determinants to which a particular antigen-binding molecule or antigen-binding protein (such as an ISV, Nanobody or a polypeptide of the invention) molecule can bind. The specificity of an antigen-binding protein can be determined based on affinity and/or avidity.
   The affinity, represented by the equilibrium constant for the dissociation of an antigen with an antigen-binding protein (K_{D} or KD), is a measure for the binding strength between an antigenic determinant, i.e. the target, and an antigen-binding site on the antigen-binding protein, i.e. the ISV or Nanobody: the lesser the value of the K_{D}, the stronger the binding strength between an antigenic determinant and the antigen-binding molecule (alternatively, the affinity can also be expressed as the affinity constant (K_{A}), which is 1/K_{D}). As will be clear to the skilled person (for example on the basis of the further disclosure herein), affinity can be determined in a manner known per se, depending on the specific antigen of interest.
   Avidity is the affinity of the polypeptide, i.e. the ligand is able to bind via two (or more) pharmacophores (ISV) in which the multiple interactions synergize to enhance the "apparent" affinity. Avidity is the measure of the strength of binding between the polypeptide of the invention and the pertinent antigens. The polypeptide of the invention is able to bind via its two (or more) ISVs, such as Nanobodies, to the at least two targets, in which the multiple interactions, e.g. the first ISV, such as Nanobody, binding to the first target and the second ISV, such as Nanobody, binding to the second target, synergize to enhance the "apparent" affinity. Avidity is related to both the affinity between an antigenic determinant and its antigen binding site on the antigen-binding molecule and the number of pertinent binding sites present on the antigen-binding molecules. For example, and without limitation, polypeptides that contain two or more building blocks, such as ISVs or Nanobodies directed against different targets on a cell and in particular against human CXCR4 and human CD123 may (and usually will) bind with higher avidity than each of the individual monomers or individual building blocks, such as, for instance, the monovalent ISVs or Nanobodies, comprised in the polypeptides of the invention.
   In the present invention, monovalent antigen-binding proteins (such as the building blocks, ISVs, amino acid sequences, Nanobodies and/or polypeptides of the invention) are said to bind to their antigen with a high affinity when the dissociation constant (K_{D}) is 10⁻⁹ to 10⁻¹² moles/liter or less, and preferably 10⁻¹⁰ to 10⁻¹² moles/liter or less and more preferably 10⁻¹¹ to 10⁻¹² moles/liter (i.e. with an association constant (K_{A}) of 10⁹ to 10¹² liter/ moles or more, and preferably 10¹⁰ to 10¹² liter/moles or more and more preferably 10¹¹ to 10¹² liter/moles).
   In the present invention, monovalent antigen-binding proteins (such as the building blocks, ISVs, amino acid sequences, Nanobodies and/or polypeptides of the invention) are said to bind to their antigen with a low affinity when the dissociation constant (K_{D}) is 10⁻⁶ to 10⁻⁹ moles/liter or more, and preferably 10⁻⁶ to 10⁻⁸ moles/liter or more and more preferably 10⁻⁶ to 10⁻⁷ moles/liter (i.e. with an association constant (K_{A}) of 10⁶ to 10⁹ liter/moles or more, and preferably 10⁶ to 10⁸ liter/moles or more and more preferably 10⁶ to 10⁷ liter/moles).
   A medium affinity can be defined as values ranging in between high-low, e.g. 10⁻⁸ to 10⁻¹⁰ moles/liter.
   Any K_{D} value greater than 10⁻⁴ mol/liter (or any K_{A} value lower than 10⁴ M⁻¹) liters/mol is generally considered to indicate non-specific binding.
   The polypeptides of the invention comprise a first and a second building block, which are a first and a second ISV, such as a first and a second Nanobody. Preferably the affinity of each ISV, such as Nanobody, is determined individually. In other words, the affinity is determined for the monovalent ISV, such as Nanobody, independent of avidity effects due to the other ISV, such as Nanobody, which might or might not be present. The affinity for a monovalent ISV, such as Nanobody, can be determined on the monovalent ISV,such as Nanobody, per se, i.e. when said monovalent ISV, such as Nanobody, is not comprised in the polypeptide of the invention. In the alternative or in addition, the affinity for a monovalent ISV, such as Nanobody, can be determined on one target while the other target is absent.
   The binding of an antigen-binding protein to an antigen or antigenic determinant can be determined in any suitable manner known per se, including, for example, Scatchard analysis and/or competitive binding assays, such as radioimmunoassays (RIA), enzyme immunoassays (EIA) and sandwich competition assays, and the different variants thereof known per se in the art; as well as the other techniques mentioned herein.
   The dissociation constant may be the actual or apparent dissociation constant, as will be clear to the skilled person. Methods for determining the dissociation constant will be clear to the skilled person, and for example include the techniques mentioned herein. In this respect, it will also be clear that it may not be possible to measure dissociation constants of more than 10⁻⁴ moles/liter or 10⁻³ moles/liter (e.g., of 10⁻² moles/liter). Optionally, as will also be clear to the skilled person, the (actual or apparent) dissociation constant may be calculated on the basis of the (actual or apparent) association constant (K_{A}), by means of the relationship [K_{D} = 1/K_{A}].
   The affinity denotes the strength or stability of a molecular interaction. The affinity is commonly given as by the K_{D}, or dissociation constant, which has units of mol/liter (or M). The affinity can also be expressed as an association constant, K_{A}, which equals 1/K_{D} and has units of (mol/liter)⁻¹ (or M⁻¹). In the present specification, the stability of the interaction between two molecules (such as an amino acid sequence, Nanobody or polypeptide of the invention and its intended target) will mainly be expressed in terms of the K_{D} value of their interaction; it being clear to the skilled person that in view of the relation K_{A} =1/K_{D}, specifying the strength of molecular interaction by its K_{D} value can also be used to calculate the corresponding K_{A} value. The K_{D}-value characterizes the strength of a molecular interaction also in a thermodynamic sense as it is related to the free energy (DG) of binding by the well known relation DG=RT.In(K_{D}) (equivalently DG=-RT.In(K_{A})), where R equals the gas constant, T equals the absolute temperature and In denotes the natural logarithm.
   The K_{D} for biological interactions which are considered meaningful (e.g. specific) are typically in the range of 10⁻¹⁰M (0.1 nM) to 1O⁻⁵M (10000 nM). The stronger an interaction is, the lower is its K_{D}.
   The K_{D} can also be expressed as the ratio of the dissociation rate constant of a complex, denoted as k_{off}, to the rate of its association, denoted kₒₙ (so that K_{D} =k_{off}/kₒₙ and K_{A} = kₒₙ/k_{off}). The off-rate k_{off} has units s⁻¹ (where s is the SI unit notation of second). The on-rate kₒₙ has units M⁻¹s⁻¹. The on-rate may vary between 10² M⁻¹s⁻¹ to about 10⁷ M⁻¹s⁻¹, approaching the diffusion-limited association rate constant for bimolecular interactions. The off-rate is related to the half-life of a given molecular interaction by the relation t_{1/2}=In(2)/k_{off}. The off-rate may vary between 10⁻⁶s⁻¹ (near irreversible complex with a t_{1/2} of multiple days) to 1s⁻¹ (t_{1/2} = 0.69s).
   The affinity of a molecular interaction between two molecules can be measured via different techniques known per se, such as the well known surface plasmon resonance (SPR) biosensor technique (see for example Ober et al., Intern. Immunology, 13, 1551-1559, 2001). The term "surface plasmon resonance", as used herein, refers to an optical phenomenon that allows for the analysis of real-time biospecific interactions by detection of alterations in protein concentrations within a biosensor matrix, where one molecule is immobilized on the biosensor chip and the other molecule is passed over the immobilized molecule under flow conditions yielding kₒₙ, k_{off} measurements and hence K_{D} (or K_{A}) values. This can for example be performed using the well-known BIAcore(R) system (BIAcore International AB, a GE Healthcare company, Uppsala, Sweden and Piscataway, NJ). For further descriptions, see Jonsson, U., et al. (1993) Ann. Biol. Clin. 51 :19-26; Jonsson, U., et al. (1991) Biotechniques 11 :620-627; Johnsson, B., et al. (1995) J Mol. Recognit. 8: 125-131; and Johnnson, B., et al. (1991) Anal. Biochem. 198:268-277.
   It will also be clear to the skilled person that the measured K_{D} may correspond to the apparent K_{D} if the measuring process somehow influences the intrinsic binding affinity of the implied molecules for example by artefacts related to the coating on the biosensor of one molecule. Also, an apparent K_{D} may be measured if one molecule contains more than one recognition site for the other molecule. In such situation the measured affinity may be affected by the avidity of the interaction by the two molecules.
   Another approach that may be used to assess affinity is the 2-step ELISA (Enzyme-Linked Immunosorbent Assay) procedure of Friguet et al. (J. Immunol. Methods, 77, 305-19, 1985). This method establishes a solution phase binding equilibrium measurement and avoids possible artefacts relating to adsorption of one of the molecules on a support such as plastic.
   However, the accurate measurement of K_{D} may be quite labour-intensive and as consequence, often apparent K_{D} values are determined to assess the binding strength of two molecules. It should be noted that as long all measurements are made in a consistent way (e.g. keeping the assay conditions unchanged) apparent K_{D} measurements can be used as an approximation of the true K_{D} and hence in the present document K_{D} and apparent K_{D} should be treated with equal importance or relevance.
   Finally, it should be noted that in many situations the experienced scientist may judge it to be convenient to determine the binding affinity relative to some reference molecule. For example, to assess the binding strength between molecules A and B, one may e.g. use a reference molecule C that is known to bind to B and that is suitably labelled with a fluorophore or chromophore group or other chemical moiety, such as biotin for easy detection in an ELISA or FACS (Fluorescent activated cell sorting) or other format (the fluorophore for fluorescence detection, the chromophore for light absorption detection, the biotin for streptavidin-mediated ELISA detection). Typically, the reference molecule C is kept at a fixed concentration and the concentration of A is varied for a given concentration or amount of B. As a result an IC₅₀ value is obtained corresponding to the concentration of A at which the signal measured for C in absence of A is halved. Provided K_{D ref}, the K_{D} of the reference molecule, is known, as well as the total concentration Cref of the reference molecule, the apparent K_{D} for the interaction A-B can be obtained from following formula: K_{D} =IC₅₀/(1+c_{ref}/ K_{D ref}). Note that if C_{ref} << K_{D ref}, K_{D} ≈ IC₅₀. Provided the measurement of the IC₅₀ is performed in a consistent way (e.g. keeping c_{ref} fixed) for the binders that are compared, the strength or stability of a molecular interaction can be assessed by the IC₅₀ and this measurement is judged as equivalent to K_{D} or to apparent K_{D} throughout this text.
p) The half-life of an amino acid sequence, compound or polypeptide of the invention can generally be defined as described in paragraph o) on page 57 of WO 08/020079 and as mentioned therein refers to the time taken for the serum concentration of the amino acid sequence, compound or polypeptide to be reduced by 50%, in vivo, for example due to degradation of the sequence or compound and/or clearance or sequestration of the sequence or compound by natural mechanisms. The *in vivo* half-life of an amino acid sequence, compound or polypeptide of the invention can be determined in any manner known per se, such as by pharmacokinetic analysis. Suitable techniques will be clear to the person skilled in the art, and may for example generally be as described in paragraph o) on page 57 of WO 08/020079. As also mentioned in paragraph o) on page 57 of WO 08/020079, the half-life can be expressed using parameters such as the t1/2-alpha, t1/2-beta and the area under the curve (AUC). Reference is for example made to the Experimental Part below, as well as to the standard handbooks, such as Kenneth, A et al: Chemical Stability of Pharmaceuticals: A Handbook for Pharmacists and Peters et al, Pharmacokinete analysis: A Practical Approach (1996). Reference is also made to "Pharmacokinetics", M Gibaldi & D Perron, published by
   Marcel Dekker, 2nd Rev. edition (1982). The terms "increase in half-life" or "increased half-life" as also as defined in paragraph o) on page 57 of WO 08/020079 and in particular refer to an increase in the t1/2-beta, either with or without an increase in the t1/2-alpha and/or the AUC or both.
q) In respect of a target or antigen, the term "interaction site" on the target or antigen means a site, epitope, antigenic determinant, part, domain or stretch of amino acid residues on the target or antigen that is a site for binding to a ligand, receptor or other binding partner, a catalytic site, a cleavage site, a site for allosteric interaction, a site involved in multimerisation (such as homomerization or heterodimerization) of the target or antigen; or any other site, epitope, antigenic determinant, part, domain or stretch of amino acid residues on the target or antigen that is involved in a biological action or mechanism of the target or antigen. More generally, an "interaction site" can be any site, epitope, antigenic determinant, part, domain or stretch of amino acid residues on the target or antigen to which an amino acid sequence or polypeptide of the invention can bind such that the target or antigen (and/or any pathway, interaction, signalling, biological mechanism or biological effect in which the target or antigen is involved) is modulated (as defined herein).
r) An immunoglobulin single variable domain or polypeptide is said to be *"specific for"* a first target or antigen compared to a second target or antigen when it binds to the first antigen with an affinity/avidity (as described above, and suitably expressed as a K_{D} value, K_{A} value, K_{off} rate and/or Kₒₙ rate) that is at least 10 times, such as at least 100 times, and preferably at least 1000 times, and up to 10.000 times or more better than the affinity with which said amino acid sequence or polypeptide binds to the second target or polypeptide. For example, the first antigen may bind to the target or antigen with a K_{D} value that is at least 10 times less, such as at least 100 times less, and preferably at least 1000 times less, such as 10.000 times less or even less than that, than the K_{D} with which said amino acid sequence or polypeptide binds to the second target or polypeptide. Preferably, when an immunoglobulin single variable domain or polypeptide is "specific for" a first target or antigen compared to a second target or antigen, it is directed against (as defined herein) said first target or antigen, but not directed against said second target or antigen.
s) The terms *"cross-block", "cross-blocked"* and *"cross-blocking"* are used interchangeably herein to mean the ability of an immunoglobulin single variable domain or polypeptide to interfere with the binding of the natural ligand to its receptor(s). The extent to which an immunoglobulin single variable domain or polypeptide of the invention is able to interfere with the binding of another compound such as the natural ligand to its target, *e.g*., CXCR4, and therefore whether it can be said to cross-block according to the invention, can be determined using competition binding assays. One particularly suitable quantitative cross-blocking assay uses a FACS- or an ELISA-based approach or Alphascreen to measure competition between the labelled (*e.g*., His tagged or biotinylated) immunoglobulin single variable domain or polypeptide according to the invention and the other binding agent in terms of their binding to the target. The experimental part generally describes suitable FACS-, ELISA- or Alphascreen-displacement-based assays for determining whether a binding molecule cross-blocks or is capable of cross-blocking an immunoglobulin single variable domain or polypeptide according to the invention. It will be appreciated that the assay can be used with any of the immunoglobulin single variable domains or other binding agents described herein. Thus, in general, a cross-blocking amino acid sequence or other binding agent according to the invention is for example one which will bind to the target in the above cross-blocking assay such that, during the assay and in the presence of a second amino acid sequence or other binding agent of the invention, the recorded displacement of the immunoglobulin single variable domain or polypeptide according to the invention is between 60% and 100% *(e.g.,* in ELISA/Alphascreen based competition assay) or between 80% to 100% (*e.g.,* in FACS based competition assay) of the maximum theoretical displacement (e.g. displacement by cold (*e.g.,* unlabeled) immunoglobulin single variable domain or polypeptide that needs to be cross-blocked) by the to be tested potentially cross-blocking agent that is present in an amount of 0.01 mM or less (cross-blocking agent may be another conventional monoclonal antibody such as IgG, classic monovalent antibody fragments (Fab, scFv)) and engineered variants (*e.g*., diabodies, triabodies, minibodies, VHHs, dAbs, VHs, VLs).
t) An amino acid sequence such as e.g. an immunoglobulin single variable domain or polypeptide according to the invention is said to be a *"VHH1 type immunoglobulin single variable domain"* or *"VHH type 1 sequence",* if said VHH1 type immunoglobulin single variable domain or VHH type 1 sequence has 85% identity (using the VHH1 consensus sequence as the query sequence and use the blast algorithm with standard setting, *i.e.,* blosom62 scoring matrix) to the VHH1 consensus sequence (QVQLVESGGGLVQPGGSLRLSCAASGFTLDYYAIGWFRQAPGKEREGVSCISSS-DGSTYYADSVKGRFTISRDNAKNTVYLQMNSLKPEDTAVYYCAA), and mandatorily has a cysteine in position 50, *i.e.,* C50 (using Kabat numbering).
u) An amino acid sequence such as *e.g.,* an immunoglobulin single variable domain or polypeptide according to the invention is said to be *"cross-reactive"* for two different antigens or antigenic determinants (such as serum albumin from two different species of mammal, such as human serum albumin and cynomolgus monkey serum albumin) if it is specific for (as defined herein) both these different antigens or antigenic determinants.
v) As further described in paragraph q) on pages 58 and 59 of WO 08/020079, the amino acid residues of an immunoglobulin single variable domain are numbered according to the general numbering for V_{H} domains given by Kabat et al. ("Sequence of proteins of immunological interest", US Public Health Services, NIH Bethesda, MD, Publication No. 91), as applied to V_{HH} domains from Camelids in the article of Riechmann and Muyldermans, J. Immunol. Methods 2000 Jun 23; 240 (1-2): 185-195 (see for example Figure 2 of this publication), and accordingly FR1 of an immunoglobulin single variable domain comprises the amino acid residues at positions 1-30, CDR1 of an immunoglobulin single variable domain comprises the amino acid residues at positions 31-35, FR2 of an immunoglobulin single variable domain comprises the amino acids at positions 36-49, CDR2 of an immunoglobulin single variable domain comprises the amino acid residues at positions 50-65, FR3 of an immunoglobulin single variable domain comprises the amino acid residues at positions 66-94, CDR3 of an immunoglobulin single variable domain comprises the amino acid residues at positions 95-102, and FR4 of an immunoglobulin single variable domain comprises the amino acid residues at positions 103-113.
w) The Figures, Sequence Listing and the Experimental Part/Examples are only given to further illustrate the invention and should not be interpreted or construed as limiting the scope of the invention and/or of the appended claims in any way, unless explicitly indicated otherwise herein.
x) The half maximal inhibitory concentration (IC50) is a measure of the effectiveness of a compound in inhibiting a biological or biochemical function, e.g. a pharmacological effect. This quantitative measure indicates how much of the ISV or Nanobody (inhibitor) is needed to inhibit a given biological process (or component of a process, i.e. an enzyme, cell, cell receptor, chemotaxis, anaplasia, metastasis, invasiveness, etc) by half. In other words, it is the half maximal (50%) inhibitory concentration (IC) of a substance (50% IC, or IC50). The IC50 of a drug can be determined by constructing a dose-response curve and examining the effect of different concentrations of antagonist such as the ISV or Nanobody of the invention on reversing agonist activity. IC50 values can be calculated for a given antagonist such as the ISV or Nanobody of the invention by determining the concentration needed to inhibit half of the maximum biological response of the agonist.
   The term half maximal effective concentration (EC50) refers to the concentration of a compound which induces a response halfway between the baseline and maximum after a specified exposure time. In the present context it is used as a measure of a polypeptide's, ISV's or Nanobody's potency. The EC50 of a graded dose response curve represents the concentration of a compound where 50% of its maximal effect is observed. Concentration is preferably expressed in molar units.
   In biological systems, small changes in ligand concentration typically result in rapid changes in response, following a sigmoidal function. The inflection point at which the increase in response with increasing ligand concentration begins to slow is the EC50. This can be determined mathematically by derivation of the best-fit line. Relying on a graph for estimation is convenient in most cases. In case the EC50 is provided in the examples section, the experiments were designed to reflect the KD as accurate as possible. In other words, the EC50 values may then be considered as KD values. The term "average KD" relates to the average KD value obtained in at least 1, but preferably more than 1, such as at least 2 experiments. The term "average" refers to the mathematical term "average" (sums of data divided by the number of items in the data).
   It is also related to IC50 which is a measure of a compound's inhibition (50% inhibition). For competition binding assays and functional antagonist assays IC50 is the most common summary measure of the dose-response curve. For agonist/stimulator assays the most common summary measure is the EC50.

### Bispecific polypeptides

The present invention relates to particular polypeptides, also referred to as "polypeptides of the invention" that are as defined by the claims and comprise or essentially consist of (i) a first building block consisting of a first immunoglobulin single variable domain, wherein said first immunoglobulin single variable domain binds a first target on the surface of a cell with low affinity, but when bound impairs or inhibits a function of said first target (functional ISV); and (ii) a second building block consisting of a second immunoglobulin single variable domain, wherein said second immunoglobulin single variable domain binds a second target on the surface of a cell with high affinity, but when bound impairs or inhibits the function of said second target preferably only minimally (anchoring ISV). In addition or alternatively, the function of said second target is preferably not vital to the cell, e.g. redundant. Consequently, inhibiting the function of said second target (the "anchor") will result in limited or negligible side-effects and/or toxicity. Nevertheless, inhibiting the function of only said second target (anchor) on normal cells, *i.e.* without inhibiting the function of said first target, is already a significant reduction of the toxicity and side-effects when compared to a treatment using high affinity antibodies against either one or both targets. The polypeptides of the present invention provide a more specific inhibition of tumor proliferation and arrest or killing of the tumor cells than prior art antibodies.The bispecific polypeptides of the invention comprise at least two binding moieties, which are ISVs, such as Nanobodies, wherein at least the first binding moiety (functional ISV) is specific for a tumor associated antigen (e.g., an antigen expressed on a tumor cell, also called 'tumor marker'). The terms bispecific polypeptide, bispecific and bispecific antibody are used interchangeably herein.

Accordingly, the present invention relates to a polypeptide as defined by the claims comprising a first (functional) and a second (anchoring) immunoglobulin single variable domain (ISV),
- wherein said first ISV (functional ISV), binds to a first target with a low affinity;
- said second ISV (anchoring ISV) binds to a second target with a high affinity; and
wherein said first target and said second target are present on the surface of a cell and wherein said first target is different from said second target, and optionally said first building block (functional building block or anchoring ISV) and said second building block (anchoring building block or anchoring ISV) are linked via a linker.

The polypeptides of the invention are designed to reduce or impair a contribution of the first target to the disorder, e.g. a malignant process. The terms "malignant process" and "malignancy" are used interchangeably herein. In the present context, malignancy is the tendency of a medical condition, especially tumors, to become progressively worse and to potentially result in death. Malignancy is characterized by anaplasia, invasiveness, and/or metastasis. The pharmacologic effect of the polypeptides of the invention therefore will reside eventually in inhibiting or impairing at least one, but preferably more than one of anaplasia, invasiveness, metastasis, proliferation, differentiation, migration and/or survival of said cell. The pharmacologic effect of the polypeptides of the invention therefore will reside in increasing or supporting at least one, but preferably more than one of apoptosis, cell killing and/or growth arrest of said cell. The phenomena characterized by these terms are well known in the art.

The bispecific or multispecific polypeptides of the present invention comprise or essentially consist of at least two building blocks, which are ISVs, of which the first ISV has an increased affinity for its antigen, i.e. the first target, upon binding by the second ISV to its antigen, i.e. the second target. Such increased affinity (apparent affinity), due to avidity effects, is also called 'conditional bispecific or multispecific binding'. Such bispecific or multispecific polypeptide is also called 'a conditionally binding bispecific or multispecific polypeptide of the invention'.

It will be appreciated that the order of the first building block and the second building block on the polypeptide (orientation) can be chosen according to the needs of the person skilled in the art, as well as the relative affinities which may depend on the location of these building blocks in the polypeptide, and whether the polypeptide comprises a linker, is a matter of design choice. However, some orientations, with or without linkers, may provide preferred binding characteristics in comparison to other orientations. For instance, the order of the first and the second building block in the polypeptide of the invention can be (from N-terminus to C-terminus): (i) first building block (a first ISV such as a first Nanobody) - [linker] -second building block (a second ISV such as a second Nanobody); or (ii) second building block (a second ISV such as a second Nanobody) - [linker] -first building block (a first ISV such as a first Nanobody); (wherein the linker is optional). All orientations are encompassed by the invention, and polypeptides that contain an orientation that provides desired binding characteristics can be easily identified by routine screening, for instance as exemplified in the examples section.

Binding of the second antigen by the second, anchoring ISV enhances binding of the first antigen by the first, functional ISV of said at least two ISVs, as a result the potency of the first, functional ISV, such as Nanobody comprised in the bispecific polypeptide is increased compared to the corresponding monovalent ISV, e.g. a Nanobody.

As used herein, the term "potency" is a measure of an agent, such as a polypeptide, ISV or Nanobody, its biological activity. Potency of an agent can be determined by any suitable method known in the art, such as for instance as described in the examples section. Cell culture based potency assays are often the preferred format for determining biological activity since they measure the physiological response elicited by the agent and can generate results within a relatively short period of time. Various types of cell based assays, based on the mechanism of action of the product, can be used, including but not limited to proliferation assays, cytotoxicity assays, reporter gene assays, cell surface receptor binding assays and assays to measure induction/inhibition of functionally essential protein or other signal molecule (such as phosphorylated proteins, enzymes, cytokines, cAMP and the like), all well known in the art. Results from cell based potency assays can be expressed as "relative potency" as determined by comparison of the bispecific polypeptide of the invention to the response obtained for the corresponding reference monovalent ISV, e.g. a polypeptide comprising only one ISV or one Nanobody, optionally further comprising an irrelevant Nanobody, such as Cablys (cf. examples section).

A compound, e.g. the bispecific polypeptide, is said to be more potent than the reference compound, e.g. the corresponding monovalent or monospecific ISV or Nanobody or polypeptide comprising the corresponding monovalent or monospeciic ISV or Nanobody, when the response obtained for the compound, e.g. the bispecific polypeptide, is at least 2 times, but preferably at least 3 times, such as at least 4 times, at least 5 times, at least 6 times, at least 7 times, at least 8 times, at least 9 times, at least 10 times, at least 15 times, at least 20 times, at least 25 times, at least 50 times, at least 75 times, at least 100 times, and even more preferably even at least 200 times, or even at least 500 times, or even 1000 times better (e.g. functionally better) than the response by the reference compound, e.g. the corresponding monovalent ISV or Nanobody in a given assay.

The cell of the present disclosure relates in particular to mammalian cells, and preferably to primate cells and even more preferably to human cells. The cell is preferably a cancer cell, wherein said cancer is as defined herein, preferably a leukaemia, and even more preferably AML.

The membrane (also called plasma membrane or phospholipid bilayer) surrounds the cytoplasm of a cell, which is the outer boundary of the cell, i.e. the membrane is the surface of the cell. This membrane serves to separate and protect a cell from its surrounding environment and is made mostly from a double layer of phospholipids. Embedded within this membrane is a variety of protein molecules, such as channels, pumps and cellular receptors. Since the membrane is fluid, the protein molecules can travel within the membrane.

### First building block (functional building block)

As defined by the claims, a polypeptide of the invention contains at least two building blocks, which are ISVs such as Nanobodies, of which the first ISV, such as a Nanobody, is directed against a first target involved in a disease or disorder, such as a malignancy, and in particular involved in a leukaemia such as AML, and even more particularly against human CXCR4. Preferably, said first target is unique to a diseased cell, e.g. a cancer cell, e.g. said first target is not expressed on a normal cell. However, this will not be the case generally. In most cases, said first target will be present on both normal and diseased cells, such as cancer cells. Hence, to increase specificity to the diseased cell, e.g. cancer cell and/or decrease side-effects and toxicity due to e.g. binding to normal cells, the first ISV, such as a Nanobody, in such polypeptides will bind to said first target and in particular human CXCR4, with increased avidity compared to the corresponding monomer or monovalent ISV, such as a Nanobody, when both the first and second target are present on a cell, preferably a cancer cell (cis-format). When bound to the first target, said first, functional ISV, such as a Nanobody, will inhibit a function of said first target.

A function of a target relates to any change in a measurable biological or biochemical property elicited by said target, including physiological changes of the cell such as changes in proliferation, differentiation, anaplasia, invasiveness, metastasis, migration, survival, apoptosis, transport processes, metabolism, motility, cytokine release, cytokine composition, second messengers, enzymes, receptors, etc. Preferably the function of a target is determined by cell culture based potency assays as described above.

It will be appreciated that due to its low affinity, the function of said first ISV, such as a Nanobody, cannot be tested or ascertained directly in all cases. The present inventors demonstrated that it is nonetheless possible to test low affinity binders which impair or inhibit the function of their cognate targets (see examples section). For instance, the present inventors used family members of a previously identified high affinity member and mutated this in order to decrease the affinity. By using family members, it was ascertained that the same epitope on the target was bound. The term "family" as used in the present specification refers to a group of ISV, Nanobody and/or VHH sequences that have identical lengths (i.e. they have the same number of amino acids within their sequence) and of which the amino acid sequence between position 8 and position 106 (according to Kabat numbering) has an amino acid sequence identity of 89% or more. Family members are derived from a common ancestor during the B cell maturation process.

When, designing the polypeptides of the invention, the first ISV is chosen for its low affinity per se, disregarding the influence of any avidity effects.

Accordingly the present invention relates to a polypeptide as defined by the claims, wherein said first ISV binds to a first target with an average KD value of between 10 nM and 200 nM, such as an average KD value of 10, 15, 20, 30, 40, 50, 60, 70, 80, 90, 100, 110, 120, 130, 140, 150, 160, 170, 180, 190 nM, or 200 nM. The KD is determined by SPR.

In a further aspect, the present invention relates to a polypeptide as defined by the claims, wherein said first ISV has a low affinity when measured as a monovalent.

The present invention also relates to a polypeptide as defined by the claims, wherein said first ISV binds to a first target on the surface of a cell with an EC50 value of between 1 nM and 200 nM, such as an average EC50 value of 2, 3, 4, 5, 6, 7, 8, 9, 10, 15, 20, 30, 40, 50, 60, 70, 80, 90, 100, 110, 120, 130, 140, 150, 160, 170, 180, 190 or 200 nM.

Accordingly the present invention relates to a polypeptide as described herein, wherein said average EC50 is measured on cells comprising said target 1 but substantially lacking said target 2.

The present invention relates also to a polypeptide as defined by the claims, wherein said average KD is determined (indirectly) by SPR.

The first ISV may for example be directed against a first antigenic determinant, epitope, part, domain, subunit or confirmation (where applicable) of said first target, such as, for instance, a Receptor Tyrosine Kinase (RTK) or a G-protein coupled receptor (GPCR) participating in malignancy, and in particular human CXCR4 (OMIM 162643). If the first ISV, such as a Nanobody, binds to said first target a function of said first target is impaired or inhibited.

The first target of the polypeptide of the invention can be any target as defined by the claims, such as a cellular receptor, on the surface of a cell which is known to participate in malignancy.

For instance, receptor tyrosine kinases (RTK) and RTK-mediated signal transduction pathways are involved in tumour initiation, maintenance, angiogenesis, and vascular proliferation. About 20 different RTK classes have been identified, of which the most extensively studied are: 1. RTK class I (EGF receptor family) (ErbB family), 2. RTK class II (Insulin receptor family), 3. RTK class III (PDGF receptor family), 4. RTK class IV (FGF receptor family), 5. RTK class V (VEGF receptors family), 6. RTK class VI (HGF receptor family), 7. RTK class VII (Trk receptor family), 8. RTK class VIII (Eph receptor family), 9. RTK class IX (AXL receptor family), 10. RTK class X (LTK receptor family), 11. RTK class XI (TIE receptor family), 12. RTK class XII (ROR receptor family), 13. RTK class XIII (DDR receptor family), 14. RTK class XIV (RET receptor family), 15. RTK class XV (KLG receptor family), 16. RTK class XVI (RYK receptor family), 17. RTK class XVII (MuSK receptor family). In particular, targets such as epidermal growth factor receptors (EGFR), platelet-derived growth factor receptors (PDGFR), vascular endothelial growth factor receptors (VEGFR), c-Met, HER3, plexins, integrins, CD44, RON and on receptors involved in pathways such as the Ras/Raf/mitogen-activated protein (MAP)-kinase and phosphatidylinositol-3 kinase (PI3K)/ Akt/ mammalian target of rapamycin (mTOR) pathways.

Furthermore, a tight operational relationship occurs between GPCRs and other receptors responding to growth factors. GPCRs signaling may precede, follow, parallel or synergize the signaling of receptors for steroids, epidermal growth factor (EGF), platelet derived growth factor (PDGF), *etc.* In lung, gastric, colorectal, pancreatic and prostatic cancers, sustained GPCRs stimulation is promoted by activatory autocrine and paracrine loops.

There are two principal signal transduction pathways involving the G protein-coupled receptors: the cAMP signal pathway and the phosphatidylinositol signal pathway, both of which can participate in malignancy. When a ligand binds to the GPCR it causes a conformational change in the GPCR, which allows it to act as a guanine nucleotide exchange factor (GEF). The GPCR can then activate an associated G-protein by exchanging its bound GDP for a GTP. The G-protein's α subunit, together with the bound GTP, can then dissociate from the β and γ subunits to further affect intracellular signaling proteins or target functional proteins directly depending on the α subunit type (Gas, Gai/o, Gaq/11, Gα12/13). Hence, the eventual functions of said first target are signal transduction, e.g. the transmission and processing of cues from the outside environment to the inside of the cell, upon which the cell reacts. In cancer cells, the normal process is altered.

Preferably, the first target is chosen from Discoidin domain receptor (DDR), a receptor tyrosine kinase that is distinguished by a unique extracellular domain homologous to the lectin Discoidin I (CD167a antigen), DDR2, ErbB-1, C-erbB-2, FGFR-1, FGFR-3, CD135 antigen, CD 117 antigen, Protein tyrosine kinase-1, c-Met, CD148 antigen, C-ret, ROR1, ROR2, Tie-1, Tie-2, CD202b antigen, Trk-A, Trk-B, Trk-C, VEGFR-1, VEGFR-2, VEGFR-3, Notch receptor 1-4, FAS receptor, DR5, DR4, CD47, CX3CR1, CXCR-3, CXCR-4, CXCR-7, Chemokine binding protein 2, and CCR1, CCR2, CCR3, CCR4, CCR5, CCR6, CCR7, CCR8, CCR9, CCR10 and CCR11.

Accordingly, the present invention relates to polypeptides of the invention wherein the first ISV, such as a Nanobody, inhibits of impairs at least one function, preferably more than one, and most preferably all functions of said first target.

Preferably, the first ISV is directed against an interaction site of said first target, thereby impairing a function of said first target. A preferred interaction site for binding by the first ISV is a ligand binding site on the first target. For instance, binding of the anti-CXCR4 ISV may inhibit or displace binding of the cognate ligand, i.e. SDF-1 (also known as CXCL12) to CXCR4. Also, when the first target is part of a binding pair (for example, a receptor-ligand binding pair), the immunoglobulin single variable domains and polypeptides may be such that they compete with the cognate binding partners, *e.g*., SDF-1 for binding with CXCR4 or HGF for binding to c-Met, and/or such that they (fully or partially) neutralize binding of the cognate binding partner to the target. Also, when a ligand, e.g. SDF-1 associates with other proteins or polypeptides, such as to form protein complexes (*e.g*., with CXCR4) it is within the scope of the invention that the immunoglobulin single variable domains and polypeptides of the invention bind to the receptor associated with its ligand, e.g. SDF-1 associated with CXCR4, provided a function of the receptor is impaired. In all these cases, the immunoglobulin single variable domains and polypeptides of the invention may bind to such associated protein complexes with an affinity and/or specificity that may be the same as or different from *(i.e.,* higher than or lower than) the affinity and/or specificity with which the immunoglobulin single variable domains and polypeptides of the invention bind to the cellular target, e.g. receptor and in particular human CXCR4 in its non-associated state, again provided a function of the first target is inhibited.

Since various cell surface receptors require dimerization for activation, it is preferred that in such cases the first ISV binds to these dimerization sites, such as homo- or hetero-dimerization sites, thereby inhibiting or preventing dimerization and thus signalling by the receptor pair.

Furthermore, most receptors exist in various conformations, e.g. the relaxed conformation binds substrates readily, while upon binding of a substrate the conformation is changed allowing signalling. Accordingly, the first ISV may also impair the function of the first target by allosteric effects. For instance, binding of the first ISV prevents the first target from conformational changes, thereby inhibiting signalling.

Advantageously, since the bispecific constructs of the invention are directed against two different targets, inadvertent dimerization and thus signalling is precluded.

It is also expected that the immunoglobulin single variable domains and polypeptides of the invention will generally bind to all naturally occurring or synthetic analogs, variants, mutants, alleles, parts and fragments of its targets; or at least to those analogs, variants, mutants, alleles, parts and fragments of CXCR4 and in particular human CXCR4 that contain one or more antigenic determinants or epitopes that are essentially the same as the antigenic determinant(s) or epitope(s) to which the immunoglobulin single variable domains and polypeptides of the invention bind to CXCR4 and in particular to human CXCR4. Again, in such a case, the immunoglobulin single variable domains and polypeptides of the invention may bind to such analogs, variants, mutants, alleles, parts and fragments with an affinity and/or specificity that are the same as, or that are different from (*i.e.,* higher than or lower than), the affinity and specificity with which the immunoglobulin single variable domains bind to (wild-type) CXCR4, provided a function of CXCR4 is inhibited.

Inhibition of a function(s) of the first target can be determined by any suitable assay known by the person skilled in the art, such as ELISA, FACS, Scatchard analysis, Alphascreen, SPR, functional assays, etc.

The efficacy or potency of the immunoglobulin single variable domains and polypeptides of the invention, and of compositions comprising the same, can be tested using any suitable in vitro assay, cell-based assay, in vivo assay and/or animal model known *per se,* or any combination thereof, depending on the specific disease or disorder involved. Suitable assays and animal models will be clear to the skilled person, and for example include ligand displacement assays (Burgess et al., Cancer Res 2006 66:1721-9), dimerization assays (WO2009/007427A2, Goetsch, 2009), signaling assays (Burgess et al., Mol Cancer Ther 9:400-9), proliferation/survival assays (Pacchiana et al., J Biol Chem 2010 Sep M110.134031), cell adhesion assays (Holt et al., Haematologica 2005 90:479-88) and migration assays (Kong-Beltran et al., Cancer Cell 6:75-84), endothelial cell sprouting assays (Wang et al., J Immunol. 2009; 183:3204-11), and *in vivo* xenograft models (Jin et al., Cancer Res. 2008 68:4360-8), as well as the assays and animal models used in the experimental part below and in the prior art cited herein. A means to express the inhibition of said first target is by IC50.

Accordingly, the present invention relates to a polypeptide as defined by the claims, wherein said first ISV has an IC50 of between 200 nM and 1 nM, such as 2, 3, 4, 5, 6, 7, 8, 9, 10, 15, 20, 30, 40, 50, 60, 70, 80, 90, 100, 110, 120, 130, 140, 150, 160, 170, 180, 190 or 200 nM, for instance determined in a ligand competition assay, a functional cellular assay, such as inhibition of ligand-induced chemotaxis, an Alphascreen assay, etc.

Accordingly, the present invention relates to a polypeptide as defined by the claims, wherein said first ISV inhibits binding of a natural ligand to said first target, such as e.g. SDF-1 to CXCR4 by about 10%, 20%, 30%, 40%, 50%, 60%, 80%, 90% and preferably 95% or even 100%, e.g. relative to the inhibition in the absence of said first ISV.

Accordingly, the present invention relates to a polypeptide as defined by the claims, wherein said first ISV inhibits the pharmacologic effect e.g. anaplasia, invasiveness, metastasis, proliferation, differentiation, migration and/or survival, in which said first target is involved by about 20%, 30%, 40%, 50%, 60%, 80%, 90% and preferably 95% or even 100%, e.g. relative to the pharmacologic effect in the absence of said first ISV.

Accordingly, the present invention relates to a polypeptide as defined by the claims, wherein said first ISV increases apoptosis, cell killing and/or growth arrest of said cell, in which said first target is involved by about 20%, 30%, 40%, 50%, 60%, 80%, 90% and preferably 95% or even 100%, e.g. relative to the increase in the absence of said first ISV.

Accordingly, the present invention relates to a polypeptide as defined by the claims, wherein said first ISV displaces about 20%, 30%, 40%, 50%, 60%, 80%, 90% and preferably 95% or more of the natural ligand to said first target, e.g. relative to the displacement in the absence of said first ISV.

Accordingly, the present invention relates to a polypeptide as defined by the claims, wherein said first ISV inhibits signalling by said first target, e.g. kinase activity of said first target, by about 20%, 30%, 40%, 50%, 60%, 80%, 90% and preferably 95% or even 100%, e.g. relative to the inhibition in the absence of said first ISV.

Accordingly, the present invention relates to a polypeptide as defined by the claims, wherein said first ISV inhibits dimerisation of said first target by about 20%, 30%, 40%, 50%, 60%, 80%, 90% and preferably 95% or even 100%, e.g. relative to the inhibition in the absence of said first ISV.

Accordingly, the present invention relates to a polypeptide as defined by the claims, wherein said first ISV inhibits chemotaxis by about 20%, 30%, 40%, 50%, 60%, 80%, 90% and preferably 95% or even 100% in a chemotaxis assay, e.g. relative to the inhibition in the absence of said first ISV.

### Second building block (anchoring building block)

The second building block, which is an ISV, such as a Nanobody or VHH, has a high affinity for its --the second-- target. The second ISV may for example be directed against an antigenic determinant, epitope, part, domain, subunit or confirmation (where applicable) of said second target. The second ISV, such as Nanobody or VHH, is chosen for its high affinity for its target per se, disregarding the influence of any avidity effects.

Accordingly, the present invention relates to a polypeptide as defined by the claims, wherein said second ISV binds to a second target with an average KD value of between 10 nM and 0.1 pM, such as at an average KD value of 10 nM or less, even more preferably at an average KD value of 9 nM or less, such as less than 8, 7, 6, 5, 4, 3, 2, 1, 0.5 nM or even less, such as less than 400, 300, 200, 100, 50, 40, 30, 20, 10, 9, 8, 7, 6, 5, 4, 3, 2, 1, 0.5 pM, or even less such as less than 0.4 pM. The KD is determined by SPR.

Accordingly, the present invention relates to a polypeptide as defined by the claims, wherein said second ISV has a high affinity when measured as a monovalent.

Accordingly, the present invention relates to a polypeptide as defined by the claims, wherein said average KD is measured by surface plasmon resonance (SPR) on recombinant protein.

The present invention also relates to a polypeptide as defined by the claims, wherein said second ISV binds to a second target on the surface of a cell with an EC50 value of between 10 nM and 0.1 pM, such as at an average KD value of 10 nM or less, even more preferably at an average KD value of 9 nM or less, such as less than 8, 7, 6, 5, 4, 3, 2, 1, 0.5 nM or even less, such as less than 400, 300, 200, 100, 50, 40, 30, 20, 10, 9, 8, 7, 6, 5, 4, 3, 2, 1, 0.5 pM, or even less such as less than 0.4 pM.

Accordingly the present invention relates to a polypeptide as defined by the claims, wherein said average EC50 is measured on cells comprising said target 2 but substantially lacking said target 1.

Accordingly, the present invention relates to a polypeptide as defined by the claims, wherein said average KD is determined by Biacore on a monovalent second ISV, such as a Nanobody, or a polypeptide comprising a monovalent second ISV, such as a Nanobody.

It has been shown in the examples that the KD correlates well with the EC50.

Said second target can be any target on a cell as defined by the claims, e.g. CD123 (OMIM: 308385), provided it is different from said first target. Preferably, said second target is unique to said diseased cell, e.g. a cancer cell. For instance, said second target is not expressed on a normal, healthy cell. However, this will not be the case generally. In most cases, said second target will be present on both normal and diseased cells, e.g. cancer cells. Although the function of said second target might not be vital to said cells, inhibiting its function on normal cells may give rise to some toxicity and side-effects. The present invention further relates to high affinity binders comprised in the polypeptide of the invention which do not or only minimally impair or inhibit the function of normal cells. Accordingly the present invention relates to a polypeptide as defined by the claims, wherein said second ISV binds to an allosteric site of said second target.

Accordingly the present invention relates to a polypeptide as defined by the claims, wherein said second ISV does not substantially or only marginally inhibit a function of said second target, e.g. as a monovalent.

Accordingly the present invention relates to a polypeptide as defined by the claims, wherein said second ISV has an IC50 of between 100 nM and 10 µM, such as 200 nM, 500 nM, 1 µM or 5 µM, in an Alphascreen assay, competition ELISA, or FACS on cells as e.g., described in the experimental part.

Accordingly the present invention relates to a polypeptide as defined by the claims, wherein said second ISV inhibits binding of a natural ligand to said second target by less than about 50%, such as 40%, 30%, or 20% or even less than 10%, e.g. relative to the inhibition in the absence of said second ISV.

Accordingly the present invention relates to a polypeptide as defined by the claims,, wherein said second ISV inhibits the pharmacologic effect of said second target by less than about 50%, such as 40%, 30%, or 20% or even less than 10%, e.g. relative to the inhibition in the absence of said second ISV.

Accordingly the present invention relates to a polypeptide as defined by the claims, wherein said second ISV displaces the natural ligand to said second target by less than about 50%, such as 40%, 30%, or 20% or even less than 10%, e.g. relative to the displacement in the absence of said second ISV.

Accordingly the present invention relates to a polypeptide as defined by the claims, wherein said second ISV inhibits signalling by said second target by less than about 50%, such as 40%, 30%, or 20% or even less than 10%, e.g. relative to the inhibition in the absence of said second ISV.

Accordingly the present invention relates to a polypeptide as defined by the claims, wherein said second ISV inhibits dimerisation of said first target by less than about 50%, such as 40%, 30%, or 20% or even less than 10%, e.g. relative to the inhibition in the absence of said second ISV.

Accordingly the present invention relates to a polypeptide as defined by the claims, wherein said second ISV inhibits chemotaxis by less than about 50%, such as 40%, 30%, or 20% or even less than 10% in an chemotaxis assay, e.g. relative to the inhibition in the absence of said second ISV.

### Combinations

In order to increase specificity and thus minimize side-effects and/or toxicity, the second, anchoring target is preferably a tumor-associated antigen (TAA). TAA are typically antigens that are expressed on cells of particular tumors, but that are typically not expressed in normal cells. Often, TAA are antigens that are normally expressed in cells only at particular points in an organism's development (such as during fetal development) and that are being inappropriately expressed in the organism at the present point of development, or are antigens not expressed in normal tissues or cells of an organ now expressing the antigen. Preferred TAA as second, anchoring target include MART-1, carcinoembryonic antigen ("CEA"), gp100, MAGE-1, HER-2, and Lewis^{Y} antigens.

Cell surface antigens that are preferentially expressed on AML LSC compared with normal hematopoietic stem cells, and thus preferred as second target, include CD123, CD44, CLL-1, CD96, CD47, CD32, CXCR4, Tim-3 and CD25.

Other tumor-associated antigens suitable as the second target within the polypeptides of the invention include: TAG-72, Ep-CAM, PSMA, PSA, glycolipids such as GD2 and GD3.

The second, anchoring targets of the invention include also hematopoietic differentiation antigens, i.e. glycoproteins usually associated with cluster differentiation (CD) grouping, such as CD4, CD5, CD19, CD20, CD22, CD33, CD36, CD45, CD52, and CD147; and Cytokine receptors including Interleukin-2 receptor gamma chain (CD132 antigen); Interleukin-10 receptor alpha chain (IL-10R-A); Interleukin-10 receptor beta chain (IL-10R-B); Interleukin-12 receptor beta-1 chain (IL-12R-beta1); Interleukin-12 receptor beta-2 chain (IL-12 receptor beta-2); Interleukin-13 receptor alpha-1 chain (IL-13R-alpha-1) (CD213 al antigen); Interleukin-13 receptor alpha-2 chain (Interleukin-13 binding protein); Interleukin-17 receptor (IL-17 receptor); Interleukin-17B receptor (IL-17B receptor); Interleukin 21 receptor precursor (IL-21R); Interleukin-1 receptor, type I (IL-1R-1) (CD121a); Interleukin-1 receptor, type II (IL-1R-beta) (CDw121b); Interleukin-1 receptor antagonist protein (IL-1ra); Interleukin-2 receptor alpha chain (CD25 antigen); Interleukin-2 receptor beta chain (CD122 antigen); Interleukin-3 receptor alpha chain (IL-3R-alpha) (CD123 antigen)

Accordingly the present invention relates to a polypeptide as defined by the claims, wherein said second, anchoring target is chosen from the group consisting of MART-1, carcinoembryonic antigen ("CEA"), gp100, MAGE-1, HER-2, and Lewis^{Y} antigens, CD123, CD44, CLL-1, CD96, CD47, CD32, CXCR4, Tim-3, CD25, TAG-72, Ep-CAM, PSMA, PSA, GD2, GD3, CD4, CD5, CD19, CD20, CD22, CD33, CD36, CD45, CD52, and CD147; and Cytokine receptors including Interleukin-2 receptor gamma chain (CD132 antigen); Interleukin-10 receptor alpha chain (IL-10R-A); Interleukin-10 receptor beta chain (IL-10R-B); Interleukin-12 receptor beta-1 chain (IL-12R-beta1); Interleukin-12 receptor beta-2 chain (IL-12 receptor beta-2); Interleukin-13 receptor alpha-1 chain (IL-13R-alpha-1) (CD213 al antigen); Interleukin-13 receptor alpha-2 chain (Interleukin-13 binding protein); Interleukin-17 receptor (IL-17 receptor); Interleukin-17B receptor (IL-17B receptor); Interleukin 21 receptor precursor (IL-21R); Interleukin-1 receptor, type I (IL-1R-1) (CD121a); Interleukin-1 receptor, type II (IL-1R-beta) (CDw121b); Interleukin-1 receptor antagonist protein (IL-1ra); Interleukin-2 receptor alpha chain (CD25 antigen); Interleukin-2 receptor beta chain (CD122 antigen); Interleukin-3 receptor alpha chain (IL-3R-alpha) (CD123 antigen).

Accordingly the present invention relates to a polypeptide as defined by the claims, wherein said first, functional target is chosen from the group consisting of GPCRs, Receptor Tyrosine Kinases, DDR1, Discoidin I (CD167a antigen), DDR2, ErbB-1, C-erbB-2, FGFR-1, FGFR-3, CD135 antigen, CD 117 antigen, Protein tyrosine kinase-1, c-Met, CD148 antigen, C-ret, ROR1, ROR2, Tie-1, Tie-2, CD202b antigen, Trk-A, Trk-B, Trk-C, VEGFR-1, VEGFR-2, VEGFR-3, Notch receptor 1-4, FAS receptor, DR5, DR4, CD47, CX3CR1, CXCR-3, CXCR-4, CXCR-7, Chemokine binding protein 2, and CCR1, CCR2, CCR3, CCR4, CCR5, CCR6, CCR7, CCR8, CCR9, CCR10 and CCR11; and said second target is chosen from the group consisting of MART-1, carcinoembryonic antigen ("CEA"), gp100, MAGE-1, HER-2, and Lewis^{Y} antigens, CD123, CD44, CLL-1, CD96, CD47, CD32, CXCR4, Tim-3, CD25, TAG-72, Ep-CAM, PSMA, PSA, GD2, GD3, CD4, CD5, CD19, CD20, CD22, CD33, CD36, CD45, CD52, and CD147; and Cytokine receptors including Interleukin-2 receptor gamma chain (CD132 antigen); Interleukin-10 receptor alpha chain (IL-10R-A); Interleukin-10 receptor beta chain (IL-10R-B); Interleukin-12 receptor beta-1 chain (IL-12R-beta1); Interleukin-12 receptor beta-2 chain (IL-12 receptor beta-2); Interleukin-13 receptor alpha-1 chain (IL-13R-alpha-1) (CD213 al antigen); Interleukin-13 receptor alpha-2 chain (Interleukin-13 binding protein); Interleukin-17 receptor (IL-17 receptor); Interleukin-17B receptor (IL-17B receptor); Interleukin 21 receptor precursor (IL-21R); Interleukin-1 receptor, type I (IL-1R-1) (CD121a); Interleukin-1 receptor, type II (IL-1R-beta) (CDw121b); Interleukin-1 receptor antagonist protein (IL-1ra); Interleukin-2 receptor alpha chain (CD25 antigen); Interleukin-2 receptor beta chain (CD122 antigen); Interleukin-3 receptor alpha chain (IL-3R-alpha) (CD123 antigen).

As used herein "epidermal growth factor receptor" (EGFR, ErbB1, HER1) refers to naturally occurring or endogenous mammalian EGFR proteins and to proteins having an amino acid sequence which is the same as that of a naturally occurring or endogenous corresponding mammalian EGFR protein (e.g., recombinant proteins, synthetic proteins (i.e., produced using the methods of synthetic organic chemistry)). Accordingly, as defined herein, the term includes mature EGFR protein, polymorphic or allelic variants, and other isoforms of an EGFR (e.g., produced by alternative splicing or other cellular processes), and modified or unmodified forms of the foregoing (e.g., lipidated, glycosylated). Naturally occurring or endogenous EGFR include wild type proteins such as mature EGFR, polymorphic or allelic variants and other isoforms which occur naturally in mammals (e.g., humans, non-human primates). Such proteins can be recovered or isolated from a source which naturally produces EGFR, for example. These proteins and proteins having the same amino acid sequence as a naturally occurring or endogenous corresponding EGFR, are referred to by the name of the corresponding mammal. For example, where the corresponding mammal is a human, the protein is designated as a human EGFR. An ISV (e.g., Nanobody) that inhibits binding of EGF and/or TGF alpha to EGFR inhibits binding in the EGFR binding assay or EGFR kinase assay described herein with an IC50 of about 1 [mu]M or less, about 500 nM or less, about 100 nM or less, about 75 nM or less, about 50 nM or less, about 10 nM or less or about 1 nM or less.

Accordingly the present invention relates to a polypeptide as described herein, wherein said first target (functional target) and said second target (anchoring target) are chosen from the group consisting of

| functional target | anchoring target |
|---|---|
| RTK | TAA |
| GPCR | TAA |
| CXCR4 (OMIM: 162643) | CD123 (OMIM: 308385) |
| DR5 (OMIM: 603612) | EpCam (OMIM: 185535) |
| DR4 (OMIM: 126452) | EpCam (OMIM: 185535) |
| CD95 (OMIM: 134637) | EpCam (OMIM: 185535) |
| CD47 (OMIM: 601028) | CD123 (OMIM: 308385) |
| CD47 (OMIM: 601028) | EpCam (OMIM: 185535) |
| EGFR (OMIM: 131550) | CEA (OMIM: 114890) |
| CXCR4 (OMIM: 162643) | CD4 (OMIM/ 186940) |
| IL12Rβ1 (OMIM: 601604) | CD4 (OMIM/ 186940) |
| IL12Rβ2 (OMIM: 601642) | CD4 (OMIM/ 186940) |
| IL23R (OMIM: 605580) | CD4 (OMIM/ 186940) |

In particular, the present invention relates to a polypeptide according to the invention, wherein said first target and said second target are chosen from the group consisting of:
- Receptor Tyrosine Kinase as a first target and a tumor-associated antigen (TAA) as a second target;
- G-Protein-Coupled Receptor (GPCR) as a first target and a hematopoietic differentiation antigen as a second target;
- Receptor Tyrosine Kinase as a first target and a hematopoietic differentiation antigen as a second target;
- G-Protein-Coupled Receptor (GPCR) as a first target and a tumor-associated antigen (TAA) as a second target;
- CXCR4 as a first target and CD123 as a second target;
- DR5 as first target and EpCam as a second target;
- DR4 as first target and EpCam as a second target;
- CD95 as first target and EpCam as a second target;
- CD47as first target and CD123 as a second target;
- CD47 as first target and EpCam as a second target;
- EGFR as first target and CEA as a second target
- CD4 as first target and CXCR4 as a second target
- IL12Rβ1 as first target and CD4 as a second target
- IL12Rβ2 as first target and CD4 as a second target, and
- IL23R as first target and CD4 as a second target

The present inventors have also demonstrated that a first target can become a second target and vice versa, depending on the affinity and the functional properties of the respective ISVs (see e.g. ISVs binding CXCR4).

The present inventors further demonstrated that the absolute copy number of the first and second target, but also the ratio of the first target and second target, on the cell surface can be a determinant in the specificity of the eventual binding, and thus in the toxicity and/or side effects. Preferably, a low number of copies is present of said first, functional target. Preferably, a high number of copies is present of said second, anchoring target. Even more preferably, a low ratio of the first, functional target and second, anchoring target is present on the cell surface number.

Accordingly the present invention relates to a polypeptide as defined by the claims, wherein said cell comprises between 1,000 and 40,000 copies, such as between 10,000 and 20,000 copies of said first target on the surface of said cell.

Accordingly the present invention relates to a polypeptide as defined by the claims, wherein said cell comprises between 40,000 and 100,000 copies, such as between 60,000 and 80,000 copies of said second target on the surface of said cell.

Accordingly the present invention relates to a polypeptide as defined by the claims, wherein said cell comprises a ratio of 0.01 to 0.9 of said first, functional target and said second, anchoring target, even more preferably between 0.2 to 0.8, 0.3 to 0.7, 0.4 to 0.6, such as a ratio of 0.02, 0.05, 0.08, 0.1, 0.2, 0.3, 0.4, 0.5, 0.6, 0.7, 0.8, 0.9, preferably a ratio of 0.5.

As such, the polypeptides and compositions of the present invention can be used for the diagnosis, prevention and treatment of diseases and disorders of the present invention (herein also "diseases and disorders of the present disclosure") which include, but are not limited to cancer. The term "cancer" refers to the pathological condition in mammals that is typically characterized by dysregulated cellular proliferation or survival. Examples of cancer include, but are not limited to, carcinomas, gliomas, mesotheliomas, melanomas, lymphomas, leukemias, adenocarcinomas: breast cancer, ovarian cancer, cervical cancer, glioblastoma, multiple myeloma (including monoclonal gammopathy of undetermined significance, asymptomatic and symptomatic myeloma), prostate cancer, and Burkitt's lymphoma, head and neck cancer, colon cancer, colorectal cancer, non-small cell lung cancer, small cell lung cancer, cancer of the esophagus, stomach cancer, pancreatic cancer, hepatobiliary cancer, cancer of the gallbladder, cancer of the small intestine, rectal cancer, kidney cancer, bladder cancer, prostate cancer, penile cancer, urethral cancer, testicular cancer, vaginal cancer, uterine cancer, thyroid cancer, parathyroid cancer, adrenal cancer, pancreatic endocrine cancer, carcinoid cancer, bone cancer, skin cancer, retinoblastomas, Hodgkin's lymphoma, non-Hodgkin's lymphoma, Kaposi's sarcoma, multicentric Castleman's disease or AIDS-associated primary effusion lymphoma, neuroectodermal tumors, rhabdomyosarcoma (see *e.g*., Cancer, Principles and practice (DeVita, V.T. et al. eds 1997) for additional cancers); as well as any metastasis of any of the above cancers, as well as non-cancer indications such as nasal polyposis; as well as other disorders and diseases described herein. In particular, the polypeptides and compositions of the present invention can be used for the diagnosis, prevention and treatment of diseases involving EGFR mediated metastasis, chemotaxis, cell adhesion, trans endothelial migration, cell proliferation and/or survival. Cancers characterized by expression of EGFR on the surface of cancerous cells (EGFR-expressing cancers) include, for example, bladder cancer, ovarian cancer, colorectal cancer, breast cancer, lung cancer (e.g., non-small cell lung carcinoma), gastric cancer, pancreatic cancer, prostate cancer, head and neck cancer, renal cancer and gall bladder cancer..

For a general description of immunoglobulin single variable domains, reference is made to the further description below, as well as to the prior art cited herein. In this respect, it should however be noted that this description and the prior art mainly describes immunoglobulin single variable domains of the so-called "V_{H}3 class" (*i.e.,* immunoglobulin single variable domains with a high degree of sequence homology to human germline sequences of the V_{H}3 class such as DP-47, DP-51 or DP-29), which form a preferred aspect of this disclosure. It should, however, be noted that the disclosure in its broadest sense generally covers any type of immunoglobulin single variable domains and for example also covers the immunoglobulin single variable domains belonging to the so-called "V_{H}4 class" (*i.e.,* immunoglobulin single variable domains with a high degree of sequence homology to human germline sequences of the V_{H}4 class such as DP-78), as for example described in WO 07/118670.

Generally, immunoglobulin single variable domains (in particular V_{HH} sequences and sequence optimized immunoglobulin single variable domains) can in particular be characterized by the presence of one or more *"Hallmark residues"* (as described herein) in one or more of the framework sequences (again as further described herein).

Thus, generally, an immunoglobulin single variable domain can be defined as an amino acid sequence with the (general) structure (cf. formula 1 below)
FR1 - CDR1 - FR2 - CDR2 - FR3 - CDR3 - FR4 in which FR1 to FR4 refer to framework regions 1 to 4, respectively, and in which CDR1 to CDR3 refer to the complementarity determining regions 1 to 3, respectively.

In a preferred aspect, the invention provides polypeptides comprising at least an immunoglobulin single variable domain that is an amino acid sequence with the (general) structure
FR1 - CDR1 - FR2 - CDR2 - FR3 - CDR3 - FR4 in which FR1 to FR4 refer to framework regions 1 to 4, respectively, and in which CDR1 to CDR3 refer to the complementarity determining regions 1 to 3, respectively, and in which:
i) at least one of the amino acid residues at positions 11, 37, 44, 45, 47, 83, 84, 103, 104 and 108 according to the Kabat numbering are chosen from the Hallmark residues mentioned in Table A-1 below; and in which:
ii) said amino acid sequence has at least 80%, more preferably 90%, even more preferably 95% amino acid identity with at least one of the immunoglobulin single variable domains as shown in WO 2009/138519 (see SEQ ID NOs: 1 to 125 in WO 2009/138519), in which for the purposes of determining the degree of amino acid identity, the amino acid residues that form the CDR sequences (indicated with X in the sequences) are disregarded; and in which:
iii) the CDR sequences are generally as further defined herein (*e.g*., the CDR1, CDR2 and CDR3 in a combination as can be determined with the information provided herein, noting that the CDR definitions are calculated according to the Kabat numbering system).

**Table A-1: Hallmark Residues in VHHs**

| Position | Human V_{H}3 | Hallmark Residues |
|---|---|---|
| 11 | L, V; predominantly L | L, S, V, M, W, F, T, Q, E, A, R, G, K, Y, N, P, I; preferably L |
| 37 | V, I, F; usually V | F⁽¹⁾, Y, V, L, A, H, S, I, W, C, N, G, D, T, P, preferably F⁽¹⁾ or Y |
| 44⁽⁸⁾ | G | E⁽³⁾, Q⁽³⁾, G⁽²⁾, D, A, K, R, L, P, S, V, H, T, N, W, M, I; preferably G⁽²⁾, E⁽³⁾ or Q⁽³⁾; most preferably G⁽²⁾ or Q⁽³⁾. |
| 45⁽⁸⁾ | L | L⁽²⁾, R⁽³⁾, P, H, F, G, Q, S, E, T, Y, C, I, D, V; preferably L⁽²⁾ or R⁽³⁾ |
| 47⁽⁸⁾ | W, Y | F⁽¹⁾, L⁽¹⁾ or W⁽²⁾ G, I, S, A, V, M, R, Y, E, P, T, C, H, K, Q, N, D; preferably W⁽²⁾, L⁽¹⁾ or F⁽¹⁾ |
| 83 | R or K; usually R | R, K⁽⁵⁾, T, E⁽⁵⁾, Q, N, S, I, V, G, M, L, A, D, Y, H; preferably K or R; most preferably K |
| 84 | A, T, D; predominantly A | P⁽⁵⁾, S, H, L, A, V, I, T, F, D, R, Y, N, Q, G, E; preferably P |
| 103 | W | W⁽⁴⁾, R⁽⁶⁾, G, S, K, A, M, Y, L, F, T, N, V, Q, P⁽⁶⁾, E, C; preferably W |
| 104 | G | G, A, S, T, D, P, N, E, C, L; preferably G |
| 108 | L, M or T; predominantly L | Q, L⁽⁷⁾, R, P, E, K, S, T, M, A, H; preferably Q or L⁽⁷⁾ |
| Notes: | | |
| ⁽¹⁾ In particular, but not exclusively, in combination with KERE or KQRE at positions 43-46. | | |
| ⁽²⁾ Usually as GLEW at positions 44-47. | | |
| ⁽³⁾ Usually as KERE or KQRE at positions 43-46, e.g. as KEREL, KEREF, KQREL, KQREF, KEREG, KQREW or KQREG at positions 43-47. Alternatively, also sequences such as TERE (for example TEREL), TQRE (for example TQREL), KECE (for example KECEL or KECER), KQCE (for example KQCEL), RERE (for example REREG), RQRE (for example RQREL, RQREF or RQREW), QERE (for example QEREG), QQRE, (for example QQREW, QQREL or QQREF), KGRE (for example KGREG), KDRE (for example KDREV) are possible. Some other possible, but less preferred sequences include for example DECKL and NVCEL. | | |
| ⁽⁴⁾ With both GLEW at positions 44-47 and KERE or KQRE at positions 43-46. | | |
| ⁽⁵⁾ Often as KP or EP at positions 83-84 of naturally occurring V_{HH} domains. | | |
| ⁽⁶⁾ In particular, but not exclusively, in combination with GLEW at positions 44-47. | | |
| ⁽⁷⁾ With the proviso that when positions 44-47 are GLEW, position 108 is always Q in (non-humanized) V_{HH} sequences that also contain a W at 103. | | |
| ⁽⁸⁾ The GLEW group also contains GLEW-like sequences at positions 44-47, such as for example GVEW, EPEW, GLER, DQEW, DLEW, GIEW, ELEW, GPEW, EWLP, GPER, GLER and ELEW. | | |

Again, such immunoglobulin single variable domains may be derived in any suitable manner and from any suitable source, and may for example be naturally occurring V_{HH} sequences (*i.e.,* from a suitable species of Camelid, *e.g*., llama) or synthetic or semi-synthetic VHs or VLs (*e.g*., from human). Such immunoglobulin single variable domains may include "humanized" or otherwise "sequence optimized" VHHs, "camelized" immunoglobulin sequences (and in particular camelized heavy chain variable domain sequences, *i.e.,* camelized VHs), as well as human VHs, human VLs, camelid VHHs that have been altered by techniques such as affinity maturation (for example, starting from synthetic, random or naturally occurring immunoglobulin sequences), CDR grafting, veneering, combining fragments derived from different immunoglobulin sequences, PCR assembly using overlapping primers, and similar techniques for engineering immunoglobulin sequences well known to the skilled person; or any suitable combination of any of the foregoing as further described herein. As mentioned herein, a particularly preferred class of immunoglobulin single variable domains comprises immunoglobulin single variable domains with an amino acid sequence that corresponds to the amino acid sequence of a naturally occurring V_{HH} domain, but that has been "humanized", i.e. by replacing one or more amino acid residues in the amino acid sequence of said naturally occurring V_{HH} sequence (and in particular in the framework sequences) by one or more of the amino acid residues that occur at the corresponding position(s) in a V_{H} domain from a conventional 4-chain antibody from a human being (e.g. indicated above). This can be performed in a manner known per se, which will be clear to the skilled person, for example on the basis of the further description herein and the prior art on humanization referred to herein. Again, it should be noted that such humanized immunoglobulin single variable domains can be obtained in any suitable manner known per se and thus are not strictly limited to polypeptides that have been obtained using a polypeptide that comprises a naturally occurring V_{HH} domain as a starting material.

Another particularly preferred class of immunoglobulin single variable domains comprises immunoglobulin single variable domains with an amino acid sequence that corresponds to the amino acid sequence of a naturally occurring V_{H} domain, but that has been "camelized", i.e. by replacing one or more amino acid residues in the amino acid sequence of a naturally occurring V_{H} domain from a conventional 4-chain antibody by one or more of the amino acid residues that occur at the corresponding position(s) in a V_{HH} domain of a heavy chain antibody. This can be performed in a manner known per se, which will be clear to the skilled person, for example on the basis of the description herein. Such "camelizing" substitutions are preferably inserted at amino acid positions that form and/or are present at the V_{H}-V_{L} interface, and/or at the so-called Camelidae hallmark residues, as defined herein (see also for example WO 94/04678 and Davies and Riechmann (1994 and 1996)). Preferably, the V_{H} sequence that is used as a starting material or starting point for generating or designing the camelized immunoglobulin single variable domains is preferably a V_{H} sequence from a mammal, more preferably the V_{H} sequence of a human being, such as a V_{H}3 sequence. However, it should be noted that such camelized immunoglobulin single variable domains can be obtained in any suitable manner known per se and thus are not strictly limited to polypeptides that have been obtained using a polypeptide that comprises a naturally occurring V_{H} domain as a starting material.

For example, again as further described herein, both "humanization" and "camelization" can be performed by providing a nucleotide sequence that encodes a naturally occurring V_{HH} domain or V_{H} domain, respectively, and then changing, in a manner known per se, one or more codons in said nucleotide sequence in such a way that the new nucleotide sequence encodes a "humanized" or "camelized" immunoglobulin single variable domains, respectively. This nucleic acid can then be expressed in a manner known per se, so as to provide the desired immunoglobulin single variable domains. Alternatively, based on the amino acid sequence of a naturally occurring V_{HH} domain or V_{H} domain, respectively, the amino acid sequence of the desired humanized or camelized immunoglobulin single variable domains, respectively, can be designed and then synthesized *de novo* using techniques for peptide synthesis known per se. Also, based on the amino acid sequence or nucleotide sequence of a naturally occurring V_{HH} domain or V_{H} domain, respectively, a nucleotide sequence encoding the desired humanized or camelized immunoglobulin single variable domains, respectively, can be designed and then synthesized *de novo* using techniques for nucleic acid synthesis known per se, after which the nucleic acid thus obtained can be expressed in a manner known per se, so as to provide the desired immunoglobulin single variable domains.

Generally, proteins or polypeptides that comprise or essentially consist of a single building block, single immunoglobulin single variable domain or single Nanobody will be referred to herein as "monovalent" proteins or polypeptides or as "monovalent constructs", or as monovalent building block, monovalent immunoglobulin single variable domain or monovalent Nanobody, respectively. Proteins and polypeptides that comprise or essentially consist of two or more immunoglobulin single variable domains (such as at least two immunoglobulin single variable domains) will be referred to herein as "multivalent" proteins or polypeptides or as "multivalent constructs", and these provide certain advantages compared to the corresponding monovalent immunoglobulin single variable domains. Some non-limiting examples of such multivalent constructs will become clear from the further description herein. The polypeptides of the invention are "multivalent", i.e. comprising two or more building blocks or ISVs of which at least the first building block, which is an ISV, such as a Nanobody, and the second building block, which is an ISV, such as a Nanobody, are different, and directed against different targets as defined by the claims, such as antigens or antigenic determinants. Polypeptides of the invention that contain at least two ISVs, such as Nanobodies, in which at least one ISV, such as a Nanobody, is directed against a first antigen *(i.e.,* against the first target, such as e.g. CXCR4) and at least one ISV, such as a Nanobody, is directed against a second antigen (*i.e.,* against the second target which is different from the first target, such as e.g. CD123), will also be referred to as "multispecific" polypeptides of the invention, and the building blocks, ISVs, such as Nanobodies, present in such polypeptides will also be referred to herein as being in a "multivalent format". Thus, for example, a "bispecific" polypeptide of the invention is a polypeptide that comprises at least one ISV, such as a Nanobody, directed against a first target (e.g. CXCR4) and at least one further ISV, such as a Nanobody, directed against a second target (*i.e.,* directed against a second target different from said first target, e.g. CD123), whereas a "trispecific" polypeptide of the invention is a polypeptide that comprises at least one ISV, sucha s a Nanobody, directed against a first target (*e.g*., CXCR4), a second ISV, such as a Nanobody, directed against a second target different from said first target (e.g. CD123) and at least one further building block, ISV or Nanobody directed against a third antigen *(i.e.,* different from both the first and the second target), such as, for instance, serum albumin; etc. As will be clear from the description, the invention is not limited to bispecific polypeptides, in the sense that a multispecific polypeptide of the invention may comprise at least a first ISV, such as a Nanobody, against a first target, a second ISV, such as a Nanobody, against a second target and any number of building blocks, ISVs or Nanobodies directed against one or more targets, which may be the same or different from the first and/or second target, respectively. The building blocks, ISVs or Nanobodies can optionally be linked via linker sequences.

Accordingly, the present invention also relates to a trispecific or multispecific polypeptide, comprising or essentially consisting of at least three binding moieties, such as three ISVs, wherein at least one of said at least three binding moieties is an ISV directed against a first target with a low affinity, at least one of said at least three binding moieties is an ISV directed against a second target with a high affinity and at least a third binding moiety increasing half life, such as e.g. an Albumin binder.

As will be clear from the further description above and herein, the immunoglobulin single variable domains can be used as "building blocks" to form polypeptides of the invention, *e.g*., by suitably combining them with other groups, residues, moieties or binding units, in order to form compounds or constructs as described herein (such as, without limitations, the bi-/tri-/tetra-/ multivalent and bi-/tri-/tetra-/multispecific polypeptides of the invention described herein) which combine within one molecule one or more desired properties or biological functions.

The compounds or polypeptides of the invention can generally be prepared by a method which comprises at least one step of suitably linking the one or more immunoglobulin single variable domains to the one or more further groups, residues, moieties or binding units, optionally via the one or more suitable linkers, so as to provide the compound or polypeptide of the invention. Polypeptides of the invention can also be prepared by a method which generally comprises at least the steps of providing a nucleic acid that encodes a polypeptide of the invention, expressing said nucleic acid in a suitable manner, and recovering the expressed polypeptide of the invention. Such methods can be performed in a manner known *per se,* which will be clear to the skilled person, for example on the basis of the methods and techniques further described herein.

The process of designing/selecting and/or preparing a compound or polypeptide of the invention, starting from an amino acid sequence, is also referred to herein as *"formatting"* said amino acid sequence of the invention; and an amino acid that is made part of a compound or polypeptide of the invention is said to be *"formatted"* or to be *"in the format of"* said compound or polypeptide of the invention. Examples of ways in which an amino acid sequence can be formatted and examples of such formats will be clear to the skilled person based on the disclosure herein; and such formatted immunoglobulin single variable domains form a further aspect of the disclosure.

For example, such further groups, residues, moieties or binding units may be one or more additional immunoglobulin single variable domains, such that the compound or construct is a (fusion) protein or (fusion) polypeptide. In a preferred but non-limiting aspect, said one or more other groups, residues, moieties or binding units are immunoglobulin sequences. Even more preferably, said one or more other groups, residues, moieties or binding units are chosen from the group consisting of domain antibodies, immunoglobulin single variable domains that are suitable for use as a domain antibody, single domain antibodies, immunoglobulin single variable domains (ISVs) that are suitable for use as a single domain antibody, "dAb"'s, immunoglobulin single variable domains that are suitable for use as a dAb, or Nanobodies. Alternatively, such groups, residues, moieties or binding units may for example be chemical groups, residues, moieties, which may or may not by themselves be biologically and/or pharmacologically active. For example, and without limitation, such groups may be linked to the one or more immunoglobulin single variable domains so as to provide a "derivative" of an amino acid sequence or polypeptide of the invention, as further described herein.

Also within the scope of the present invention are compounds or constructs, which comprise or essentially consist of one or more derivatives as described herein, and optionally further comprise one or more other groups, residues, moieties or binding units, optionally linked via one or more linkers. Preferably, said one or more other groups, residues, moieties or binding units are immunoglobulin single variable domains. In the compounds or constructs described above, the one or more immunoglobulin single variable domains and the one or more groups, residues, moieties or binding units may be linked directly to each other and/or via one or more suitable linkers or spacers. For example, when the one or more groups, residues, moieties or binding units are immunoglobulin single variable domains, the linkers may also be immunoglobulin single variable domains, so that the resulting compound or construct is a fusion protein or fusion polypeptide.

In a specific, but non-limiting aspect of the invention, which will be further described herein, the polypeptides of the invention have an increased half-life in serum (as further described herein) compared to the immunoglobulin single variable domain from which they have been derived. For example, an immunoglobulin single variable domain may be linked (chemically or otherwise) to one or more groups or moieties that extend the half-life (such as PEG), so as to provide a derivative of an amino acid sequence with increased half-life.

In a specific aspect of the invention, a compound of the invention or a polypeptide of the invention may have an increased half-life, compared to the corresponding amino acid sequence of the invention. Some preferred, but non-limiting examples of such compounds and polypeptides will become clear to the skilled person based on the further disclosure herein, and for example comprise immunoglobulin single variable domains or polypeptides of the invention that have been chemically modified to increase the half-life thereof (for example, by means of pegylation); immunoglobulin single variable domains that comprise at least one additional binding site for binding to a serum protein (such as serum albumin); or polypeptides of the invention which comprise at least one amino acid sequence that is linked to at least one moiety (and in particular at least one amino acid sequence) which increases the half-life of the amino acid sequence. Examples of polypeptides of the invention which comprise such half-life extending moieties or immunoglobulin single variable domains will become clear to the skilled person based on the further disclosure herein; and for example include, without limitation, polypeptides in which the one or more immunoglobulin single variable domains are suitably linked to one or more serum proteins or fragments thereof (such as (human) serum albumin or suitable fragments thereof) or to one or more binding units that can bind to serum proteins (such as, for example, domain antibodies, immunoglobulin single variable domains that are suitable for use as a domain antibody, single domain antibodies, immunoglobulin single variable domains that are suitable for use as a single domain antibody, "dAb"'s, immunoglobulin single variable domains that are suitable for use as a dAb, or Nanobodies that can bind to serum proteins such as serum albumin (such as human serum albumin), serum immunoglobulins such as IgG, or transferrin; reference is made to the further description and references mentioned herein); polypeptides in which an amino acid sequence is linked to an Fc portion (such as a human Fc) or a suitable part or fragment thereof; or polypeptides in which the one or more immunoglobulin single variable domains are suitable linked to one or more small proteins or peptides that can bind to serum proteins, such as, without limitation, the proteins and peptides described in WO 91/01743, WO 01/45746, WO 02/076489, WO2008/068280, WO2009/127691 and PCT/EP2011/051559 (WO2011/095545).

Generally, the compounds or polypeptides of the invention with increased half-life preferably have a half-life that is at least 1.5 times, preferably at least 2 times, such as at least 5 times, for example at least 10 times or more than 20 times, greater than the half-life of the corresponding amino acid sequence per se. For example, the compounds or polypeptides of the invention with increased half-life may have a half-life e.g., in humans that is increased with more than 1 hours, preferably more than 2 hours, more preferably more than 6 hours, such as more than 12 hours, or even more than 24, 48 or 72 hours, compared to the corresponding amino acid sequence per se.

In a preferred, but non-limiting aspect of the invention, such compounds or polypeptides of the invention have a serum half-life e.g. in humans that is increased with more than 1 hours, preferably more than 2 hours, more preferably more than 6 hours, such as more than 12 hours, or even more than 24, 48 or 72 hours, compared to the corresponding amino acid sequence per se.

In another preferred, but non-limiting aspect of the invention, such compounds or polypeptides of the invention exhibit a serum half-life in human of at least about 12 hours, preferably at least 24 hours, more preferably at least 48 hours, even more preferably at least 72 hours or more. For example, compounds or polypeptides of the invention may have a half-life of at least 5 days (such as about 5 to 10 days), preferably at least 9 days (such as about 9 to 14 days), more preferably at least about 10 days (such as about 10 to 15 days), or at least about 11 days (such as about 11 to 16 days), more preferably at least about 12 days (such as about 12 to 18 days or more), or more than 14 days (such as about 14 to 19 days).

In a particularly preferred but non-limiting aspect of the invention, the invention provides a polypeptide of the invention comprising a first and a second immunoglobulin single variable domain (ISV), wherein said first ISV binds to a first target on the surface of a cell with a low affinity and when bound inhibits a function of said first target; and said second ISV binds to a second target on the surface of said cell with a high affinity, and preferably inhibits a function of said second target minimally, wherein said first target is different from said second target; and further comprising one or more (preferably one) serum albumin binding immunoglobulin single variable domain as described herein, e.g. the serum albumin binding immunoglobulin single variable domain of SEQ ID NO: 114 or 115 (Table B-4).

### Polypeptide-Drug Conjugates (PDCs)

In some embodiments, the polypeptides of the invention are conjugated with drugs to form polypeptide-drug conjugates (PDCs). Contemporaneous antibody-drug conjugates (ADCs) are used in oncology applications, where the use of antibody-drug conjugates for the local delivery of drugs, such as cytotoxic or cytostatic agents, toxin or toxin, moieties, allows for the targeted delivery of the drug moiety to tumors, which can allow higher efficacy, lower toxicity, etc. These ADCs have three components: (1) a monoclonal antibody conjugated through a (2) linker to a (3) toxin moiety or toxin. An overview of this technology is provided in Ducry et al., Bioconjugate Chem., 21:5-13 (2010), Carter et al., Cancer J. 14(3):154 (2008) and Senter, Current Opin. Chem. Biol. 13:235-244 (2009). The PDCs also have three components: (1) a polypeptide conjugated through a (2) linker to a (3) drug, such as a toxin moiety or toxin. The person skilled in the art will appreciate that the technology, methods, means, etc. of ADCs are equally applicable to PDCs.

The invention provides polypeptides of the invention comprising a drug, such as a toxin or toxin moiety.

The drug, e.g. toxin moiety or toxin can be linked or conjugated to the polypeptide using any suitable method. Generally, conjugation is done by covalent attachment to the polypeptide, as known in the art, and generally relies on a linker, often a peptide linkage. For example, the drug, such as toxin moiety or toxin can be covalently bonded to the polypeptide directly or through a suitable linker. Suitable linkers can include noncleavable or cleavable linkers, for example, pH cleavable linkers that comprise a cleavage site for a cellular enzyme (e.g., cellular esterases, cellular proteases such as cathepsin B). Such cleavable linkers can be used to prepare a ligand that can release a drug, such as a toxin moiety or toxin after the polypeptide is internalized. As will be appreciated by those in the art, the number of drug moieties per polypeptide can change, depending on the conditions of the reaction, and can vary from 1:1 to 10:1 drug:polypeptide. As will also be appreciated by those in the art, the actual number is an average. A variety of methods for linking or conjugating a drug, such as a toxin moiety or toxin to a polypeptide can be used. The particular method selected will depend on the drug, such as a toxin moiety or toxin and polypeptide to be linked or conjugated. If desired, linkers that contain terminal functional groups can be used to link the polypeptide and drug, e.g. a toxin moiety or toxin. Generally, conjugation is accomplished by reacting the drug, e.g. a toxin moiety or toxin that contains a reactive functional group (or is modified to contain a reactive functional group) with a linker or directly with a polypeptide. Covalent bonds formed by reacting a drug, e.g. a toxin moiety or toxin that contains (or is modified to contain) a chemical moiety or functional group that can, under appropriate conditions, react with a second chemical group thereby forming a covalent bond. If desired, a suitable reactive chemical group can be added to polypeptide or to a linker using any suitable method. (See, e.g., Hermanson, G. T., Bioconjugate Techniques, Academic Press: San Diego, CA (1996).) Many suitable reactive chemical group combinations are known in the art, for example an amine group can react with an electrophilic group such as tosylate, mesylate, halo (chloro, bromo, fluoro, iodo), N-hydroxysuccinimidyl ester (NHS), and the like. Thiols can react with maleimide, iodoacetyl, acrylolyl, pyridyl disulfides, 5-thiol-2-nitrobenzoic acid thiol (TNB-thiol), and the like. An aldehyde functional group can be coupled to amine- or hydrazide-containing molecules, and an azide group can react with a trivalent phosphorous group to form phosphoramidate or phosphorimide linkages. Suitable methods to introduce activating groups into molecules are known in the art (see for example, Hermanson, G. T., Bioconjugate Techniques, Academic Press: San Diego, CA (1996)).

As described below, the drug of the PDC can be any number of agents, including but not limited to cytostatic agents, cytotoxic agents such as chemotherapeutic agents, growth inhibitory agents, toxins (for example, an enzymatically active toxin of bacterial, fungal, plant, or animal origin, or fragments thereof), toxin moieties, or a radioactive isotope (that is, a radioconjugate) are provided. In other embodiments, the disclosure further provides methods of using the PDCs.

Drugs for use in the present invention include cytotoxic drugs, particularly those which are used for cancer therapy. Such drugs include, in general, DNA damaging agents, anti-metabolites, natural products and their analogs. Exemplary classes of cytotoxic agents include the enzyme inhibitors such as dihydrofolate reductase inhibitors, and thymidylate synthase inhibitors, DNA intercalators, DNA cleavers, topoisomerase inhibitors, the anthracycline family of drugs, the vinca drugs, the mitomycins, the bleomycins, the cytotoxic nucleosides, the pteridine family of drugs, diynenes, the podophyllotoxins, dolastatins, maytansinoids, differentiation inducers, and taxols.

Members of these classes include, for example, methotrexate, methopterin, dichloromethotrexate, 5-fluorouracil, 6-mercaptopurine, cytosine arabinoside, melphalan, leurosine, leurosideine, actinomycin, daunorubicin, doxorubicin, mitomycin C, mitomycin A, caminomycin, aminopterin, tallysomycin, podophyllotoxin and podophyllotoxin derivatives such as etoposide or etoposide phosphate, vinblastine, vincristine, vindesine, taxanes including taxol, taxotere retinoic acid, butyric acid, N8-acetyl spermidine, camptothecin, calicheamicin, esperamicin, ene-diynes, duocarmycin A, duocarmycin SA, calicheamicin, camptothecin, maytansinoids (including DM1), monomethylauristatin E (MMAE), monomethylauristatin F (MMAF), and maytansinoids (DM4) and their analogues.

Drugs, such as toxins may be used as polypeptides-toxin conjugates and include bacterial toxins such as diphtheria toxin, plant toxins such as ricin, small molecule toxins such as geldanamycin (Mandler et al (2000) J. Nat. Cancer Inst. 92(19):1573-1581; Mandler et al (2000) Bioorganic & Med. Chem. Letters 10:1025-1028; Mandler et al (2002) Bioconjugate Chem. 13:786-791), maytansinoids (EP 1391213; Liu et al., (1996) Proc. Natl. Acad. Sci. USA 93:8618-8623), and calicheamicin (Lode et al (1998) Cancer Res. 58:2928; Hinman et al (1993) Cancer Res. 53:3336-3342). Toxins may exert their cytotoxic and cytostatic effects by mechanisms including tubulin binding, DNA binding, or topoisomerase inhibition.

Conjugates of a polypeptide of the invention and one or more small molecule toxins, such as a maytansinoids, dolastatins, auristatins, a trichothecene, calicheamicin, and CC1065, and the derivatives of these toxins that have toxin activity, are contemplated.

Other drugs, such as antitumor agents that can be conjugated to the polypeptides of the invention include BCNU, streptozoicin, vincristine and 5-fluorouracil, the family of agents known collectively LL-E33288 complex described in U.S. Pat. Nos. 5,053,394, 5,770,710, as well as esperamicins (U.S. Pat. No. 5,877,296).

Drugs, such as enzymatically active toxins and fragments thereof which can be used include diphtheria A chain, nonbinding active fragments of diphtheria toxin, exotoxin A chain (from Pseudomonas aeruginosa), ricin A chain, abrin A chain, modeccin A chain, alpha-sarcin, Aleurites fordii proteins, dianthin proteins, Phytolaca americana proteins (PAPI, PAPII, and PAP-S), momordica charantia inhibitor, curcin, crotin, sapaonaria officinalis inhibitor, gelonin, mitogellin, restrictocin, phenomycin, enomycin and the tricothecenes. See, for example, WO 93/21232 published Oct. 28, 1993.

The present invention further contemplates a PDC formed between a polypeptide of the invention and a compound with nucleolytic activity (e.g., a ribonuclease or a DNA endonuclease such as a deoxyribonuclease; DNase).

For selective destruction of the tumor, the polypeptide of the invention may comprise a highly radioactive atom. A variety of radioactive isotopes are available for the production of radioconjugated antibodies. Examples include At211, 1131, 1125, Y90, Re186, Re188, Sm153, Bi212, P32, Pb212 and radioactive isotopes of Lu.

The radio- or other labels may be incorporated in the conjugate in known ways. For example, the peptide may be biosynthesized or may be synthesized by chemical amino acid synthesis using suitable amino acid precursors involving, for example, fluorine-19 in place of hydrogen. Labels such as Tc99m or 1123, Re186, Re188 and In111 can be attached via a cysteine residue in the peptide. Yttrium-90 can be attached via a lysine residue. The IODOGEN method (Fraker et al (1978) Biochem. Biophys. Res. Commun. 80: 49-57 can be used to incorporate Iodine-123. "Monoclonal Antibodies in Immunoscintigraphy" (Chatal, CRC Press 1989) describes other methods in detail.

The generation of polypeptide-drug conjugate compounds can be accomplished by any technique known to the skilled artisan in the field of ADCs. Briefly, the polypeptide-drug conjugate compounds can include polypeptide of the invention as the Antibody unit, a drug, and optionally a linker that joins the drug and the binding agent.

Methods of determining whether a drug or an antibody-drug conjugate exerts an effect, e.g. a cytostatic and/or cytotoxic effect on a cell are known. Generally, the effect, e.g. a cytotoxic or cytostatic activity of an Antibody Drug Conjugate can be measured by: exposing mammalian cells expressing a target protein of the Antibody Drug Conjugate in a cell culture medium; culturing the cells for a period from about 6 hours to about 5 days; and measuring cell viability. Cell-based in vitro assays can be used to measure viability (proliferation), cytotoxicity, and induction of apoptosis (caspase activation) of the Antibody Drug Conjugate. These methods are equally applicable to PDCs.

Accordingly the invention relates to a polypeptide of the invention further comprising a drug, such as a toxin or toxin moiety.

Accordingly, the present invention relates to a polypeptide according to the invention conjugated to a drug, such as a toxin or toxin moiety.

In view of the specificity, the polypeptides of the invention are also very suitable for conjugation to imaging agents. Suitable imaging agents for conjugating to antibodies are well known in the art, and similarly useful for conjugating to the polypeptides of the present invention. Suitable imaging agents include but are not limited to molecules preferably selected from the group consisting of organic molecules, enzyme labels, radioactive labels, colored labels, fluorescent labels, chromogenic labels, luminescent labels, haptens, digoxigenin, biotin, metal complexes, metals, colloidal gold, fluorescent label, metallic label, biotin, chemiluminescent, bioluminescent, chromophore and mixtures thereof.

Accordingly, the present invention relates to a polypeptide according to the invention, further comprising an imaging agent, including, but not limited to a molecule preferably selected from the group consisting of organic molecules, enzyme labels, radioactive labels, colored labels, fluorescent labels, chromogenic labels, luminescent labels, haptens, digoxigenin, biotin, metal complexes, metals, colloidal gold, fluorescent label, metallic label, biotin, chemiluminescent, bioluminescent, chromophore and mixtures thereof.

### Linkers

In the polypeptides of the invention, the two or more building blocks, ISVs or Nanobodies and the optionally one or more polypeptides one or more other groups, drugs, agents, residues, moieties or binding units may be directly linked to each other (as for example described in WO 99/23221) and/or may be linked to each other via one or more suitable spacers or linkers, or any combination thereof. Suitable spacers or linkers for use in multivalent and multispecific polypeptides will be clear to the skilled person, and may generally be any linker or spacer used in the art to link amino acid sequences. Preferably, said linker or spacer is suitable for use in constructing proteins or polypeptides that are intended for pharmaceutical use.

Some particularly preferred spacers include the spacers and linkers that are used in the art to link antibody fragments or antibody domains. These include the linkers mentioned in the general background art cited above, as well as for example linkers that are used in the art to construct diabodies or ScFv fragments (in this respect, however, its should be noted that, whereas in diabodies and in ScFv fragments, the linker sequence used should have a length, a degree of flexibility and other properties that allow the pertinent V_{H} and V_{L} domains to come together to form the complete antigen-binding site, there is no particular limitation on the length or the flexibility of the linker used in the polypeptide of the invention, since each Nanobody by itself forms a complete antigen-binding site).

For example, a linker may be a suitable amino acid sequence, and in particular amino acid sequences of between 1 and 50, preferably between 1 and 30, such as between 1 and 10 amino acid residues. Some preferred examples of such amino acid sequences include gly-ser linkers, for example of the type (glyₓser_{y})_{z}, such as (for example (gly₄ser)₃ or (gly₃ser₂)₃, as described in WO 99/42077 and the GS30, GS15, GS9 and GS7 linkers described in the applications by Ablynx mentioned herein (see for example WO 06/040153 and WO 06/122825), as well as hinge-like regions, such as the hinge regions of naturally occurring heavy chain antibodies or similar sequences (such as described in WO 94/04678). Preferred linkers are depicted in Table B-5.

Some other particularly preferred linkers are poly-alanine (such as AAA), as well as the linkers GS30 (SEQ ID NO: 85 in WO 06/122825) and GS9 (SEQ ID NO: 84 in WO 06/122825).

Other suitable linkers generally comprise organic compounds or polymers, in particular those suitable for use in proteins for pharmaceutical use. For instance, poly(ethyleneglycol) moieties have been used to link antibody domains, see for example WO 04/081026.

It is encompassed within the scope of the invention that the length, the degree of flexibility and/or other properties of the linker(s) used (although not critical, as it usually is for linkers used in ScFv fragments) may have some influence on the properties of the final polypeptide of the invention, including but not limited to the affinity, specificity or avidity for a chemokine, or for one or more of the other antigens. Based on the disclosure herein, the skilled person will be able to determine the optimal linker(s) for use in a specific polypeptide of the invention, optionally after some limited routine experiments.

For example, in multivalent polypeptides of the invention that comprise building blocks, ISVs or Nanobodies directed against a first and second target, the length and flexibility of the linker are preferably such that it allows each building block, ISV or Nanobody present in the polypeptide to bind to its cognate target, e.g. the antigenic determinant on each of the targets. Again, based on the disclosure herein, the skilled person will be able to determine the optimal linker(s) for use in a specific polypeptide of the invention, optionally after some limited routine experiments.

It is also within the scope of the invention that the linker(s) used confer one or more other favourable properties or functionality to the polypeptides of the invention, and/or provide one or more sites for the formation of derivatives and/or for the attachment of functional groups (e.g. as described herein for the derivatives of the Nanobodies). For example, linkers containing one or more charged amino acid residues can provide improved hydrophilic properties, whereas linkers that form or contain small epitopes or tags can be used for the purposes of detection, identification and/or purification. Again, based on the disclosure herein, the skilled person will be able to determine the optimal linkers for use in a specific polypeptide of the invention, optionally after some limited routine experiments.

Finally, when two or more linkers are used in the polypeptides of the invention, these linkers may be the same or different. Again, based on the disclosure herein, the skilled person will be able to determine the optimal linkers for use in a specific polypeptide of the invention, optionally after some limited routine experiments.

Usually, for easy of expression and production, a polypeptide of the invention will be a linear polypeptide. However, the invention in its broadest sense is not limited thereto. For example, when a polypeptide of the invention comprises three of more building blocks, ISV or Nanobodies, it is possible to link them by use of a linker with three or more "arms", which each "arm" being linked to a building block, ISV or Nanobody, so as to provide a "star-shaped" construct. It is also possible, although usually less preferred, to use circular constructs.

### Therapeutic and diagnostic compositions and uses

The invention provides compositions comprising the polypeptides of the invention, including PDCs of the invention, and a pharmaceutically acceptable carrier, diluent or excipient. Also disclosed are therapeutic and diagnostic methods that employ the polypeptides or compositions of the invention. The polypeptides, including PDCs, according to the method of the present disclosure may be employed in in vivo therapeutic and prophylactic applications, in vivo diagnostic applications and the like. Therapeutic and prophylactic uses of polypeptides, including PDCs, of the disclosure involve the administration of polypeptides, including PDCs, according to the invention to a recipient mammal, such as a human.

Substantially pure polypeptides and PDCs of at least 90 to 95% homogeneity are preferred for administration to a mammal, and 98 to 99% or more homogeneity is most preferred for pharmaceutical uses, especially when the mammal is a human. Once purified, partially or to homogeneity as desired, the polypeptides and PDCs may be used diagnostically or therapeutically (including extracorporeally) or in developing and performing assay procedures, immunofluorescent stainings and the like (Lefkovite and Pernis, (1979 and 1981) Immunological Methods, Volumes I and II, Academic Press, NY).

For example, the polypeptides and PDCs of the present invention will typically find use in preventing, suppressing or treating disease states. For example, polypeptides or PDCs can be administered to treat, suppress or prevent a chronic inflammatory disease, allergic hypersensitivity, cancer, bacterial or viral infection, autoimmune disorders (which include, but are not limited to, Type I diabetes, asthma, multiple sclerosis, rheumatoid arthritis, juvenile rheumatoid arthritis, psoriatic arthritis, spondylarthropathy {e.g., ankylosing spondylitis), systemic lupus erythematosus, inflammatory bowel disease {e.g., Crohn's disease, ulcerative colitis), myasthenia gravis and Behcet's syndrome, psoriasis, endometriosis, and abdominal adhesions {e.g., post abdominal surgery). The polypeptides and PDCs are useful for treating infectious diseases in which cells infected with an infectious agent contain higher levels of cell surface EGFR than uninfected cells or that contain one or more cell surface targets that are not present on non-infected cells, such as a protein that is encoded by the infectious agent {e.g., bacteria, virus). The polypeptides and PDCs of the present invention will typically find use in preventing, suppressing or treating a cancer. For example, polypeptides and PDCs can be administered to treat, suppress or prevent cancer, which include, but are not limited to, carcinomas, gliomas, mesotheliomas, melanomas, lymphomas, leukemias, adenocarcinomas: breast cancer, ovarian cancer, cervical cancer, glioblastoma, multiple myeloma (including monoclonal gammopathy of undetermined significance, asymptomatic and symptomatic myeloma), prostate cancer, and Burkitt's lymphoma, head and neck cancer, colon cancer, colorectal cancer, non-small cell lung cancer, small cell lung cancer, cancer of the esophagus, stomach cancer, pancreatic cancer, hepatobiliary cancer, cancer of the gallbladder, cancer of the small intestine, rectal cancer, kidney cancer, bladder cancer, prostate cancer, penile cancer, urethral cancer, testicular cancer, vaginal cancer, uterine cancer, thyroid cancer, parathyroid cancer, adrenal cancer, pancreatic endocrine cancer, carcinoid cancer, bone cancer, skin cancer, retinoblastomas, Hodgkin's lymphoma, non-Hodgkin's lymphoma, Kaposi's sarcoma, multicentric Castleman's disease or AIDS-associated primary effusion lymphoma, neuroectodermal tumors, rhabdomyosarcoma (see e.g., Cancer, Principles and practice (DeVita, V.T. et al. eds 1997) for additional cancers); as well as any metastasis of any of the above cancers, as well as non-cancer indications such as nasal polyposis; as well as other disorders and diseases described herein.

In the instant application, the term "prevention" involves administration of the protective composition prior to the induction of the disease. "Suppression" refers to administration of the composition after an inductive event, but prior to the clinical appearance of the disease. "Treatment" involves administration of the protective composition after disease symptoms become manifest. Treatment includes ameliorating symptoms associated with the disease, and also preventing or delaying the onset of the disease and also lessening the severity or frequency of symptoms of the disease.

Animal model systems which can be used to assess efficacy of the polypeptides and PDCs of the invention in preventing treating or suppressing disease (e.g., cancer) are available. Suitable models of cancer include, for example, xenograft and orthotopic models of human cancers in animal models, such as the SCID-hu myeloma model (Epstein J, and Yaccoby, S., Methods Mol Med. 773:183-90 (2005), Tassone P, et al, Clin Cancer Res. 11:4251-8 (2005)), mouse models of human lung cancer (e.g., Meuwissen R and Berns A, Genes Dev. CHECK:643-64 (2005)), and mouse models of metastatic cancers (e.g., Kubota J Cell Biochem. 56:4-8 (1994)).

Generally, the present polypeptides and PDCs will be utilized in purified form together with pharmacologically appropriate carriers. Typically, these carriers include aqueous or alcoholic/aqueous solutions, emulsions or suspensions, including saline and/or buffered media. Parenteral vehicles include sodium chloride solution, Ringer's dextrose, dextrose and sodium chloride and lactated Ringer's. Suitable physiologically-acceptable adjuvants, if necessary to keep a polypeptide- or PDC-complex in suspension, may be chosen from thickeners such as carboxymethylcellulose, polyvinylpyrrolidone, gelatin and alginates.

Intravenous vehicles include fluid and nutrient replenishers and electrolyte replenishers, such as those based on Ringer's dextrose. Preservatives and other additives, such as antimicrobials, antioxidants, chelating agents and inert gases, may also be present (Mack (1982) Remington's Pharmaceutical Sciences, 16th Edition). A variety of suitable formulations can be used, including extended release formulations.

The polypeptides and PDCs of the present invention may be used as separately administered compositions or in conjunction with other agents. The polypeptides and PDCs can be administered and or formulated together with one or more additional therapeutic or active agents. When a polypeptide or PDC is administered with an additional therapeutic agent, the polypeptide or PDC can be administered before, simultaneously with or subsequent to administration of the additional agent. Generally, the polypeptide or PDC and additional agent are administered in a manner that provides an overlap of therapeutic effect.

The polypeptides and PDCs of the invention can be co-administered (e.g., to treat cancer, an inflammatory disease or other disease) with a variety of suitable co-therapeutic agents, including cytokines, analgesics/antipyretics, antiemetics, and chemotherapeutics.

Thus the disclosure provides a method of treating cancer comprising administering to a patient in need thereof a therapeutically effective amount of a polypeptide or PDC of the invention and a chemotherapeutic agent, wherein the chemotherapeutic agent is administered at a low dose. Generally the amount of chemotherapeutic agent that is co-administered with a polypeptide of the invention is about 80%, or about 70%, or about 60%, or about 50%, or about 40%, or about 30%, or about 20%, or about 10% or less, of the dose of chemotherapeutic agent alone that is normally administered to a patient. Thus, cotherapy is particularly advantageous when the chemotherapeutic agent causes deleterious or undesirable side effects that may be reduced or eliminated at lower doses.

Pharmaceutical compositions can include "cocktails" of various cytotoxic or other agents in conjunction with polypeptides or PDCs of the present invention, or even combinations of polypeptides and PDCs according to the present invention having different specificities, such as polypeptides or PDCs selected using different target antigens or epitopes, whether or not they are pooled prior to administration.

The route of administration of pharmaceutical compositions according to the invention may be any suitable route, such as any of those commonly known to those of ordinary skill in the art. For therapy, including without limitation immunotherapy, the polypeptides and PDCs of the invention can be administered to any patient in accordance with standard techniques. The administration can be by any appropriate mode, including parenterally, intravenously, intramuscularly, intraperitoneally, transdermally, intrathecally, intraarticularly, via the pulmonary route, or also, appropriately, by direct infusion (e.g., with a catheter). The dosage and frequency of administration will depend on the age, sex and condition of the patient, concurrent administration of other drugs, counter-indications and other parameters to be taken into account by the clinician. Administration can be local (e.g., local delivery to the lung by pulmonary administration,(e.g. , intranasal administration) or local injection directly into a tumor) or systemic as indicated.

The polypeptides and PDCs of this invention can be lyophilised for storage and reconstituted in a suitable carrier prior to use. This technique has been shown to be effective with conventional immunoglobulins and art-known lyophilisation and reconstitution techniques can be employed. It will be appreciated by those skilled in the art that lyophilisation and reconstitution can lead to varying degrees of antibody activity loss (e.g. with conventional immunoglobulins, IgM antibodies tend to have greater activity loss than IgG antibodies) and that use levels may have to be adjusted upward to compensate.

The compositions containing the polypeptides or PDCs can be administered for prophylactic and/or therapeutic treatments. In certain therapeutic applications, an adequate amount to accomplish at least partial inhibition, suppression, modulation, killing, or some other measurable parameter, of a population of selected cells is defined as a "therapeutically-effective dose". Amounts needed to achieve this dosage will depend upon the severity of the disease and the general state of the patient's health, but generally range from 0.005 to 5.0 mg of ligand per kilogram of body weight, with doses of 0.05 to 2.0 mg/kg/dose being more commonly used. For prophylactic applications, compositions containing the present polypeptides and PDCs or cocktails thereof may also be administered in similar or slightly lower dosages, to prevent, inhibit or delay onset of disease {e.g., to sustain remission or quiescence, or to prevent acute phase). The skilled clinician will be able to determine the appropriate dosing interval to treat, suppress or prevent disease. When polypeptides or PDCs are administered to treat, suppress or prevent a disease, it can be administered up to four times per day, twice weekly, once weekly, once every two weeks, once a month, or once every two months, at a dose of, for example, about 10 [mu]g/kg to about 80 mg/kg, about 100 [mu]g/kg to about 80 mg/kg, about 1 mg/kg to about 80 mg/kg, about 1 mg/kg to about 70 mg/kg, about 1 mg/kg to about 60 mg/kg, about 1 mg/kg to about 50 mg/kg, about 1 mg/kg to about 40 mg/kg, about 1 mg/kg to about 30 mg/kg, about 1 mg/kg to about 20 mg/kg , about 1 mg/kg to about 10 mg/kg, about 10 [mu]g/kg to about 10 mg/kg, about 10 [mu]g/kg to about 5 mg/kg, about 10 [mu]g/kg to about 2.5 mg/kg, about 1 mg/kg, about 2 mg/kg, about 3 mg/kg, about 4 mg/kg, about 5 mg/kg, about 6 mg/kg, about 7 mg/kg, about 8 mg/kg, about 9 mg/kg or about 10 mg/kg.

In particular embodiments, the polypeptide and PDC of the invention is administered at a dose that provides saturation of the anchoring target or a desired serum concentration in vivo. The skilled physician can determine appropriate dosing to achieve saturation, for example by titrating the polypeptide and monitoring the amount of free binding sites of said anchoring target expressing cells or the serum concentration of the polypeptide. Therapeutic regiments that involve administering a therapeutic agent to achieve target saturation or a desired serum concentration of agent are common in the art, particularly in the field of oncology.

Treatment or therapy performed using the compositions described herein is considered "effective" if one or more symptoms are reduced (e.g., by at least 10% or at least one point on a clinical assessment scale), relative to such symptoms present before treatment, or relative to such symptoms in an individual (human or model animal) not treated with such composition or other suitable control. Symptoms will obviously vary depending upon the disease or disorder targeted, but can be measured by an ordinarily skilled clinician or technician. Such symptoms can be measured, for example, by monitoring the level of one or more biochemical indicators of the disease or disorder (e.g., levels of an enzyme or metabolite correlated with the disease, affected cell numbers, etc.), by monitoring physical manifestations (e.g., inflammation, tumor size, etc.), or by an accepted clinical assessment scale. A sustained (e.g., one day or more, preferably longer) reduction in disease or disorder symptoms by at least 10% or by one or more points on a given clinical scale is indicative of "effective" treatment. Similarly, prophylaxis performed using a composition as described herein is "effective" if the onset or severity of one or more symptoms is delayed, reduced or abolished relative to such symptoms in a similar individual (human or animal model) not treated with the composition.

A composition containing polypeptides and/or PDCs according to the present invention may be utilized in prophylactic and therapeutic settings to aid in the alteration, inactivation, killing or removal of a select target cell population in a mammal. In addition, the ligands and selected repertoires of polypeptides described herein may be used extracorporeally or in vitro selectively to kill, deplete or otherwise effectively remove a target cell population from a heterogeneous collection of cells. Blood from a mammal may be combined extracorporeally with the ligands, e.g. antibodies, cell- surface receptors or binding proteins thereof whereby the undesired cells are killed or otherwise removed from the blood for return to the mammal in accordance with standard techniques.

Accordingly, the present invention relates to a pharmaceutical composition comprising a polypeptide or PDC according to the invention.

Accordingly, the present disclosure relates to a method for delivering a prophylactic or therapeutic polypeptide, PDC or imaging agent to a specific location, tissue or cell type in the body, the method comprising the steps of administering to a subject a polypeptide according to the invention.

Accordingly, the present disclosure relates to a method for treating a subject in need thereof comprising administering a polypeptide or PDC according to the invention.

Accordingly, the present invention relates to a polypeptide or PDC according to the invention for use in treating a subject in need thereof.

The embodiments illustrated and discussed in this specification are intended only to teach those skilled in the art the best way known to the inventors to make and use the invention.

The invention will now be further described by means of the following non-limiting preferred aspects, examples and figures.

### EXPERIMENTAL SECTION

### Example 1 Preferential targeting of leukemic cells with CXCR4-CD123 bispecific polypeptides

### Example 1.1 Experimental set up for designing bispecific CXCR4 and CD123 polypeptides

With the generation of bispecific anti-CXCR4-CD123 Nanobodies we aimed to generate a high affinity and high potency antagonist for CXCR4 on cells that express both the CXCR4 and CD123 receptors, as a model system for cancer cells, but not on cells that express primarily CXCR4, which represent normal cells, all in order to minimize side-effects or toxicity.

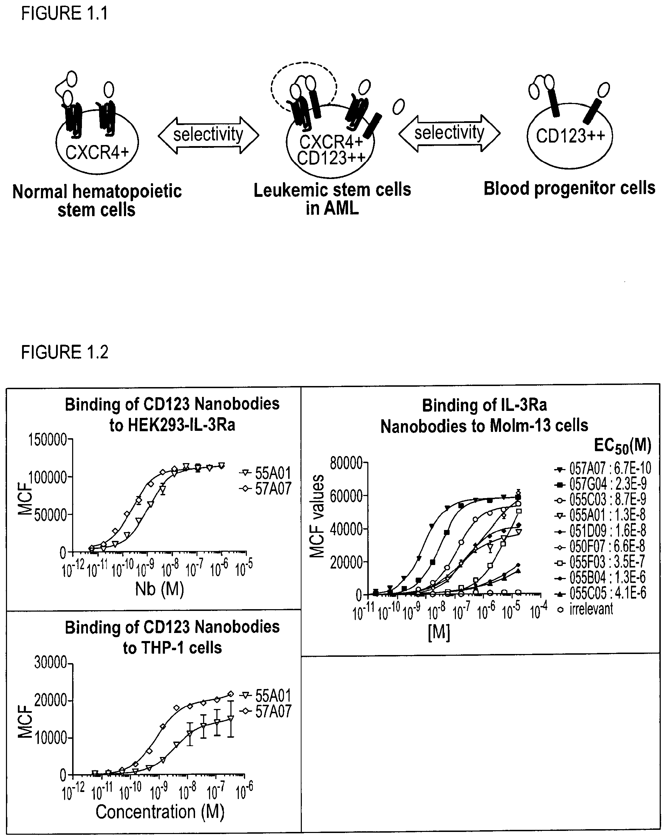

To reach this selectivity, it was hypothesized that the anti-CXCR4 Nanobody on one arm (the functional ISV) needs to be a full antagonist, but with only a low to moderate affinity. The anti-CD123 Nanobody on the other arm serves (the anchoring ISV) to increase the affinity and potency of the anti-CXCR4 Nanobody on cells which co-express both receptors by avidity. Simultaneous binding to 2 membrane receptors will increase the affinity of the bispecific over monovalent Nanobodies. For the CD123 arm, the Nanobody is preferentially a binder, but which does not affect its function, again in order to minimize side-effects or toxicity. Hence, a functional blockade of the CD123 receptor is not required. The model system as set out in Figure 1.1 was used to investigate the selective function of bispecific CD123-CXCR4 constructs, that bind with high avidity to cells expressing both receptors (i.e. leukemic stem cells), but that have only low affinity and potency for CXCR4+/CD123- cells (i.e. normal hematopoietic stem cells).

The affinity of each of the Nanobodies needed to obtain the increased avidity is a priori unknown; when the affinity is too high, the bispecific will also bind to cells that express only one receptor, which is not desired. Thereto we set out to design selection procedures for Nanobodies with different affinities to IL3Rα to be combined with low to moderate potency CXCR4 Nanobodies.

### Example 1.2: Production of monovalent Nanobodies

Monovalent CXCR4 and CD123-specific Nanobodies were produced in *E. coli* and expressed as C-terminal linked FLAG3, His6-tagged proteins in expression vector pAX129. The amino acid sequences are depicted in Tables 1 and 2 for monovalent CXCR4-building blocks and monovalent CD123-building blocks, respectively. Expression was induced by IPTG and allowed to continue for 4h at 37°C. After spinning the cell cultures, periplasmic extracts were prepared by freeze-thawing the pellets. Nanobodies were purified from these extracts using immobilized metal affinity chromatography (IMAC) and a buffer exchange to D-PBS. Purity and integrity was confirmed by SDS-PAGE.

### Example 1.3: Characteristics of anti-CD123 specific Nanobodies

In order to minimize potential side-effects and/or toxicity, the anti-CD123 Nanobodies do preferably not affect the function of the IL3Rα, which is also expressed on normal cells. Furthermore, in order to avoid any complication by the potential introduction of epitope diversity, and to ensure that any gain of function / selectivity in the Proof of Concept (PoC) study is defined only by the relative affinity (i.e. the affinity of the monovalent building block), we set out to identify Nanobodies binding to the same epitope but differing only in the relative affinity.

### Example 1.3.1 Binding of anti-CD123 Nanobodies to cells expressing IL-3Rα

Nanobody binding to membrane associated human IL-3Rα was analysed on HEK293T cells transfected with pcDNA3.1-IL3Rα (NM_002183.2) and non-transfected cells. Surface expression was confirmed by FACS using IL-3Rα specific antibodies (R&D MAB301 and BD Pharminogen 554528), followed by goat anti-mouse PE (Jackson Immuno Research 115-115-164). Briefly, serial dilutions of Nanobodies were allowed to associate for 30 minutes at 4°C in FACS buffer (PBS 1X + 10% FBS + 0.05 % azide). Following this, cells were washed by centrifugation and probed with 6.7 nM anti-FLAG for 30 minutes at 4°C, to detect bound Nanobody. Detection was done with anti-M13 for 30 minutes at 4°C. Cells were washed and incubated with TOPRO3 to stain for dead cells, which are then removed during the gating procedure. The cells were then analysed via a BD FACSArray. The results are depicted in Figure 1.2.

A clear interaction of the CD123 Nanobodies 55A01 and 57A07 with the Hek-IL-3Rα cells is demonstrated, while the lack of binding to HEK293T-wt cells confirmed the specificity of the Nanobodies for IL-3Rα (data not shown).

Binding of the CD123 Nanobodies was also assessed on leukemic cells that endogenously express both the IL-3Rα and IL-3Rβ chain, i.e. Molm-13 and THP-1 cells. These cells have a much lower IL-3Rα expression level than the transfected HEK-IL-3Ra cells, and with likely more representative expression levels of the receptor. Due to the lower potency of the clones selected for this project, the binding curves were incomplete with respect to saturation of binding. Binding curves and EC50 values are shown in Figure 1.2 and Table 3 respectively.

The binding studies confirmed that the Nanobodies are able to bind to IL3Rα but do not disrupt the heterodimeric receptor complex of IL3Rα with the IL3Rβ partner, which fulfils a prerequisite of evading a functional blockade of the CD123 receptor signalling.

### Example 1.3.2 Affinity determination of CD123 Nanobodies

The affinities of CD123 specific Nanobodies were further investigated via Surface Plasmon Resonance (SPR) at ProteOn. Immobilisation of recombinant IL-3Rα ectodomain (Sino Biologicals) was done until 761 RU. The Nanobodies were applied at a highest concentration of 1 µM, followed by a three-fold titration, covering 5 further concentrations ranging from 1 µM to 4.1 nM. These were then applied in a single injection cycle, utilising the ProteOn's specific one-shot kinetics approach for kinetic analysis. Evaluation of the association/dissociation data was performed by fitting a 1:1 interaction model (Langmuir binding model). A number of the clones failed to show saturation at the 1 µM concentration, due to the low affinity of the Nanobodies. For CD123 Nanobodies the obtained K_{D} values correlated well with the apparent affinities retrieved by cell binding EC50 values (see Table 3).

### Example 1.3.3 Competition of anti-CD123 Nanobodies with the anti-CD123 antibody 7G3

The functional high affinity human IL-3 receptor is a heterodimer consisting of a ligand binding α subunit and the β subunit. The β subunit does not bind the ligand IL-3 by itself but is required for the high affinity binding of IL-3 to the heterodimeric receptor complex.

Ligand displacement on Molm-13 cells could not be assessed, as the biotinylated ligand exerted too low binding. Since the IL-3 has only a low affinity to IL-3Rα in absence of the IL-3Rβ, transfected Hek-IL-3Rα cells could not be used either. To assess epitope information, CD123 Nanobodies were analysed in competition with the IL-3Rα-specific mAb 7G3 for binding to IL-3Rα ectodomain in ELISA. The humanised version of anti-IL-3Rα specific monoclonal antibody 7G3, CSL-360, was previously shown to lack functional efficacy in a Phase I clinical trial.

Briefly, antibody 7G3 (BD, 554527) was coated at 1ug/ml and blocked in casein (1%) in solution. Nanobodies and biotinylated-IL-3Rα ectodomain [R&D systems, 301-R3/CF] were added and allowed to reach equilibrium over four hours. The plate was then washed and 7G3 associated IL-3Rα was detected via extravidin peroxidase prior to development and subsequent analysis of absorption at OD₄₅₀ₙₘ. IC50 values are shown in Table 3.

CD123 Nanobodies were tested for their capacity to compete with the 7G3 antibody. Two anti-CD123 Nanobodies, i.e. 55A01 and 57A07, were binding to the same epitope as 7G3, but were having different relative affinities and potencies (see also Table 5). Subsequently, these Nanobodies were used for formatting into bispecific polypeptides with anti-CXCR4 Nanobodies (see Example 1.5)

### Example 1.4: Characteristics of anti-CXCR4 specific Nanobodies

In the present example, the inventors set out to identify and characterize anti-CXCR4 Nanobodies which on the one hand had a low affinity, and on the other hand still were able to act as functional antagonists. Since it is cumbersome to functionally test Nanobodies, which have low to moderate affinity, in particular the absence of any observed function must be due to the low affinity, but not due to binding to e.g. an irrelevant epitope, the inventors used an unconventional approach which is detailed below.

First a large series of available anti-CXCR4 Nanobodies were assessed for their capacity to antagonize CXCR4 signalling. In previous studies, functional antagonistic Nanobodies specific for CXCR4 were already identified. The present inventors then turned to family members of the functional antagonists, which had lower affinities.

Furthermore, the inventors observed that in some cases the position of a Nanobody in a bispecific polypeptide could decrease affinity. Without being bound to any theory, is was hypothesized that this may be due to steric hindrance. Hence, by positioning a Nanobody known to have a moderate affinity and having antagonistic activities, in an "unfavourable" location in the bispecific polypeptide, both the affinity and the functional effect could be decreased. As such, the avidity effect of the second Nanobody on the function of the low affinity anti-CXCR4 Nanobody could be discerned.

### Example 1.4.1 Identification of low affinity CXCR4 Nanobodies

For the generation of CXCR4-IL-3Ra bispecifics, Nanobodies with low to moderate affinities are needed, which recognise the correct epitope for functional blockade. In previous studies functional antagonistic Nanobodies specific for CXCR4 were identified. However, the primary aim during lead selection and identification procedure in those previous studies was to identify high potency candidates, and not the low affinity clones. As the screening cascade of previous studies was focussed on blockade of ligand binding, this hampered the identification of clones that have the correct epitope but low potencies due to low affinity as required in the present study. In case of CXCR4, which is to be embedded in the cellular membrane for correct conformation, no source of recombinant protein was available to specifically search for the low affinity Nanobodies by off-rate analysis in SPR, as done for the IL-3Ra Nanobodies.

To overcome this problem, the inventors zoomed in on family members of CXCR4 Nanobodies with proven ligand functional blockade of CXCR4 signalling. Nanobodies 14A02, 14E02 and 14D09 are members of the same family, as defined by a conserved CDR3 region. The high affine family member, CXCR4 Nanobody 14A02, has shown to be a potent antagonist of CXCR4 functionality in different cellular assays, including ligand-induced chemotaxis and inhibition of cAMP induction in CXCR4-expressing cells (Table 4).

### Example 1.4.2 Binding analysis of CXCR4 Nanobodies

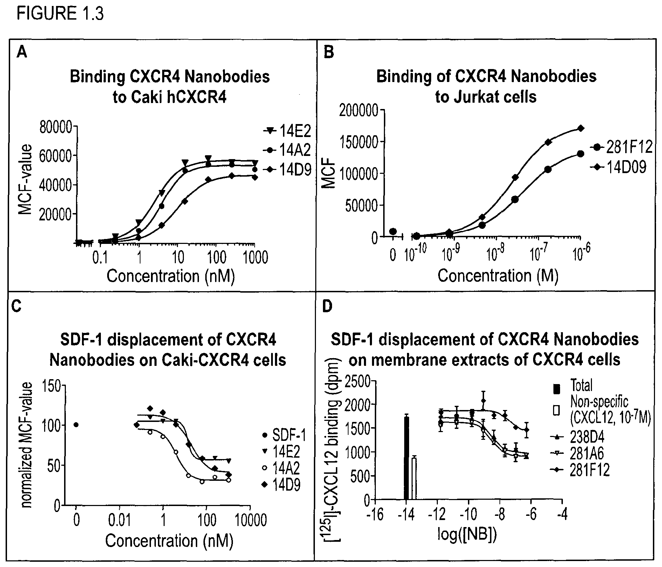

Binding of CXCR4 Nanobodies to CXCR4-expressing cells was assessed on different cell lines, to assess EC50 values. For CXCR4 the membrane insertion is needed for proper conformation and functionality of the receptor. Therefore CXCR4 Nanobodies were characterized for binding to viral lipoparticles (VLP; Molecular Integral) expressing CXCR4 versus control lipoparticles in ELISA. To this end VLPs were coated at 0.5 U/well overnight at 4°C using anti-myc antibodies for detection. Over all different binding assays, Nanobody 14D09 always exerted lower binding affinity than 14A02, as indicated by a shift in EC50 values. The results are depicted in Figure 1.3.

### Example 1.4.3 Ligand displacement of CXCR4 Nanobodies

CXCR4 Nanobodies were analysed for their ability to compete with the ligand CXCL12 (or SDF-1a) for receptor binding, by displacement of biotinylated SDF-1 on Caki-CXCR4 cells in flow cytometry. To this end serial dilutions of Nanobodies were incubated with 30 nM of biotinylated SDF-1 (R&D Systems Fluorokine kit) on cells, after which ligand binding was visualised using extravidin-PE. The biotin-SDF-1 competitor concentration used in this assay was below the EC50 value obtained in dose-titration, where IC50 values should reflect the Ki.

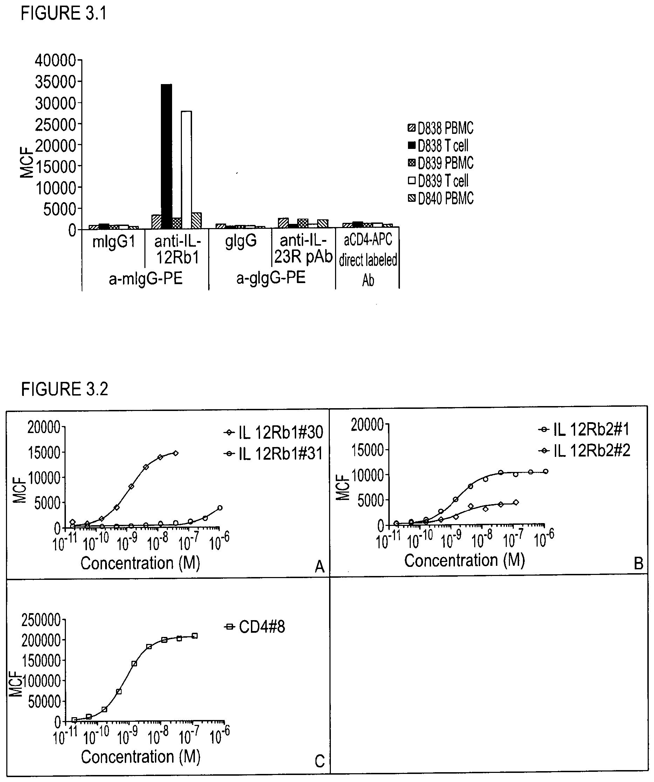

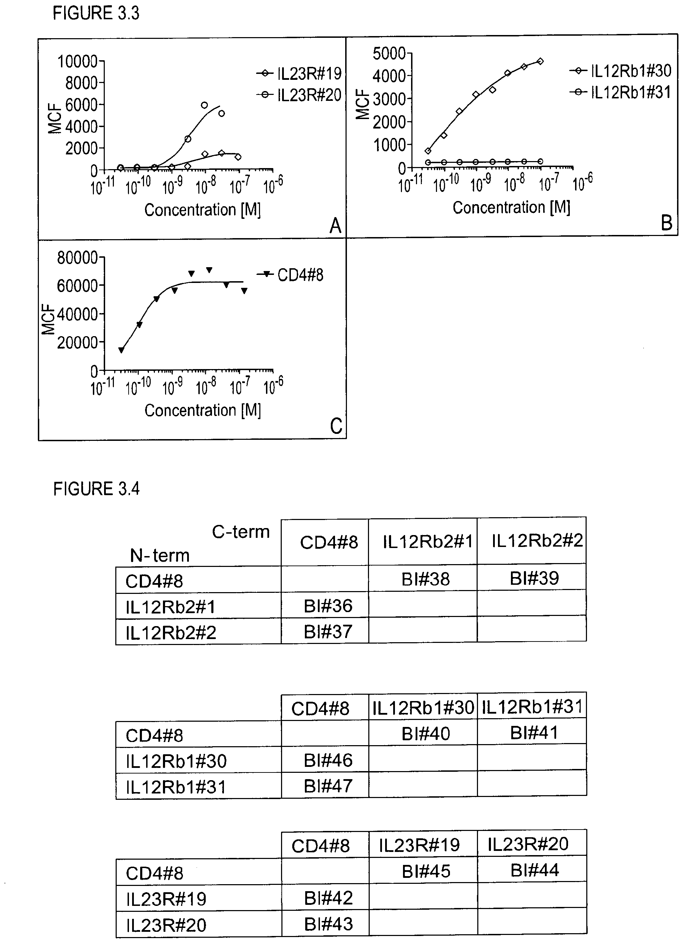

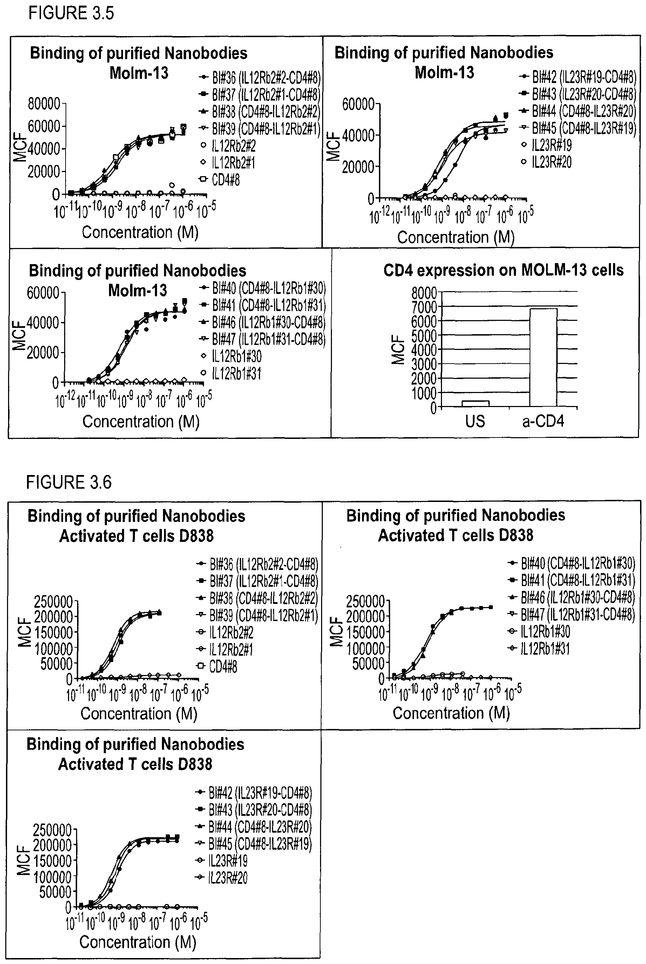

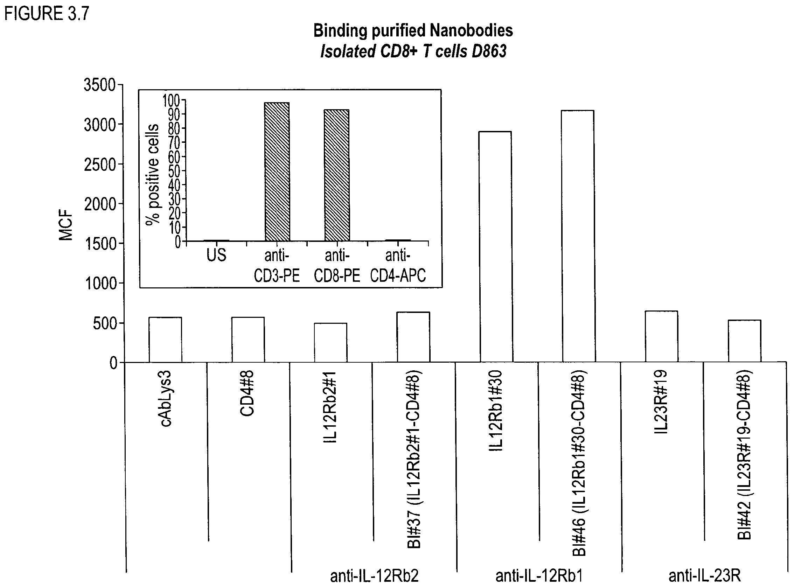

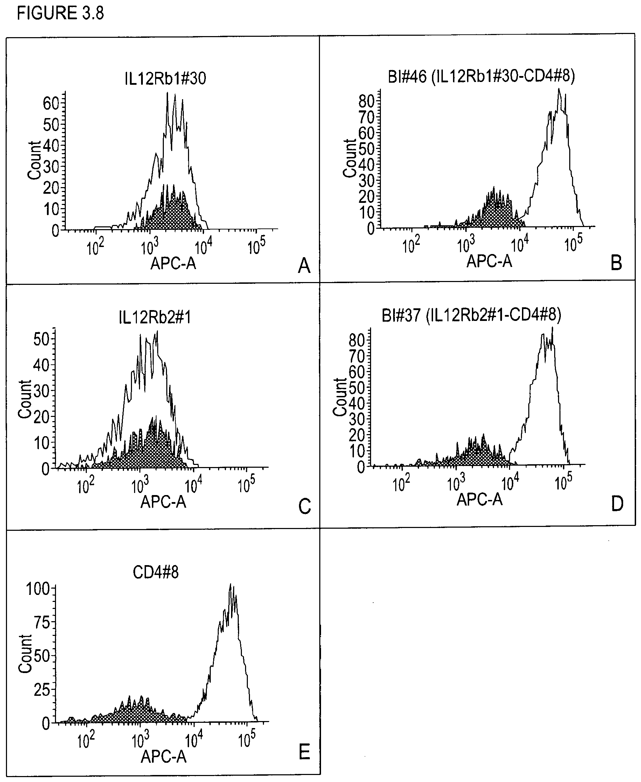

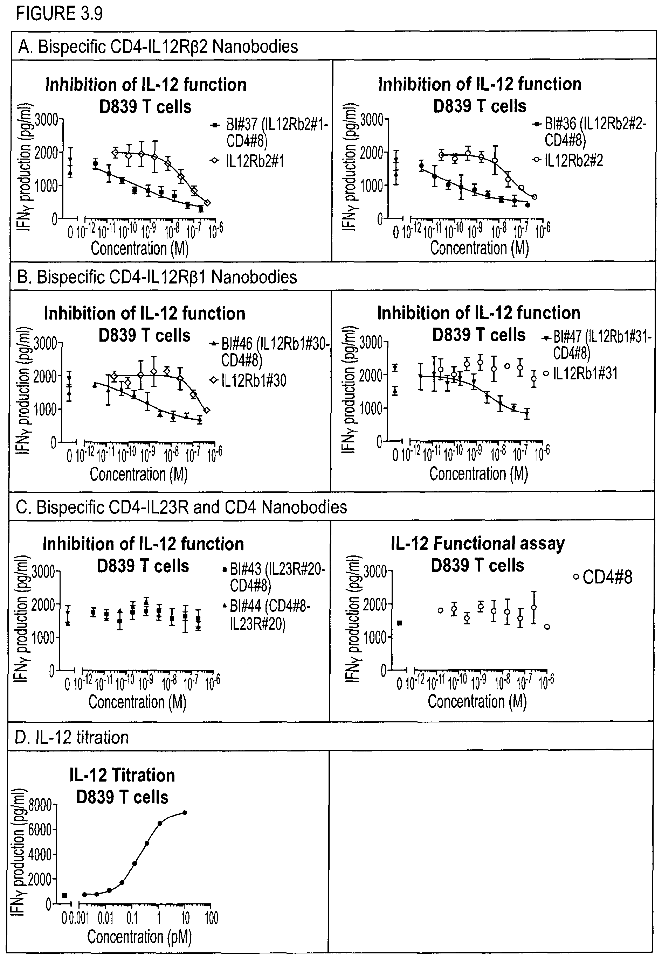

This assay confirmed that the difference in apparent affinities between the family members 14A09 and 14A02 translates into similar differences in capacity in ligand competition (Figure 3, panel C). In this manner we are confident that the 14D09 (also designated as 14D9) Nanobody is a ligand competitor and that improvement of its affinity can lead to better potencies (when lower potency fails to show efficient SDF-1 competition).

CXCR4 Nanobodies were analysed in radio-ligand displacement assay on membrane extracts of Hek-CXCR4 cells. The advantage of using the radiolabelled ligand is the increased sensitivity, and the low competitor concentration ensures the determination of Ki values (i.e. the real affinity constant) instead of measuring the IC50 value. This makes it possible to accurately determine the potencies of low affine Nanobodies, even though they may not reach full displacement.

To this end, membrane extracts of Hek293 cells transfected with CXCR4 were incubated with serial dilutions of purified Nanobodies and 75 pM of [¹²⁵I]-CXCL12. Non-specific binding was determined in presence of 100 nM cold SDF-1. As controls full blocking CXCR4 Nanobodies 238D4 and 281A6 were included. The assay was performed three times, and average percentages of SDF-1 inhibition were calculated.

In Figure 1.3 panel D is shown that Nanobody 281F12 had only a moderate potency, with a Ki of 27 nM, and only partial efficacy, while control CXCR4 Nanobodies 238D4 showed full efficacy. This makes 281F12 a suitable other candidate for use in formatting into bispecific constructs with IL-3Ra Nanobodies.

Table 4 lists the characteristics of CXCR4 Nanobodies of low to moderate affinity, as well as of their respective family members.

### Example 1.5: Bispecific polypeptides

In the present example, the inventors combined the different anti-CXCR4 and anti-CD123 Nanobodies which were identified and characterized in the previous experiments, and of which the characteristics are summarized in Table 5. The resulting bispecific polypeptides were subsequently tested for specificity. In particular, eight constructs were made, which are summarized in Table 6.

### Example 1.5.1 Cloning, production and physical characterisation

IL3Rα and CXCR4 Nanobodies were cloned in the production vector pAX138 and expressed as Myc - His6-tagged proteins to construct bispecific polypeptides. All eight combinations of the CXCR4 Nanobodies 14D09 (designated as CXCR4#1) and 281F12 (designated as CXCR4#2) and the IL-3Ra Nanobodies 57A07 (designated as CD123#1 and 55A01 (designated as CD123#2) were constructed (see Table 6). The Nanobodies were connected with a flexible, long linker of repetitive (GGGGS)₇. Individual Nanobodies were amplified in separate PCR reactions to generate N-terminal fragments and C-terminal fragments using primers containing appropriate restriction-sites. Fragments were sequentially inserted into the pAX138 expression vector for *E. coli* productions. The correct nucleotide sequence of all constructs was confirmed by sequence analysis (see Table 7, bispecific constructs). Subsequently the correct constructs were recloned into the pAX205 vector for production in *Pichia pastoris* as Flag3-His6-tagged proteins. Plasmids encoding bispecific constructs were linearized by digestion with restriction enzymes prior to the transformation into *P. pastoris* strain X-33. Small scale test expressions of *P. pastoris* transformants were done in to select for the clone with good expression levels. Hereto 4 ml scale expressions were performed of 4 clones of each construct in 24-wells deep well plates. Expression of Nanobodies in the medium was evaluated by SDS-PAGE. Medium fractions were collected and used as starting material for immobilized metal affinity chromatography (IMAC) using Nickel SepharoseTM 6 FF. Nanobodies were eluted from the column with 250 mM imidazole and subsequently desalted on Sephadex G-25 Superfine on the Atoll (ATO002) towards dPBS. The purity and integrity of Nanobodies was verified by SDS-PAGE and western blot using anti-VHH and anti-tag detection.

Monovalent CXCR4 and IL-3Ra-specific Nanobodies were produced in *E. coli* and expressed as C-terminal linked FLAG3, His6 -tagged proteins in expression vector pAX129 as set out in Example 1.2.

### Example 1.5.2 Characterisation of CXCR4-IL-3Ra bispecifics

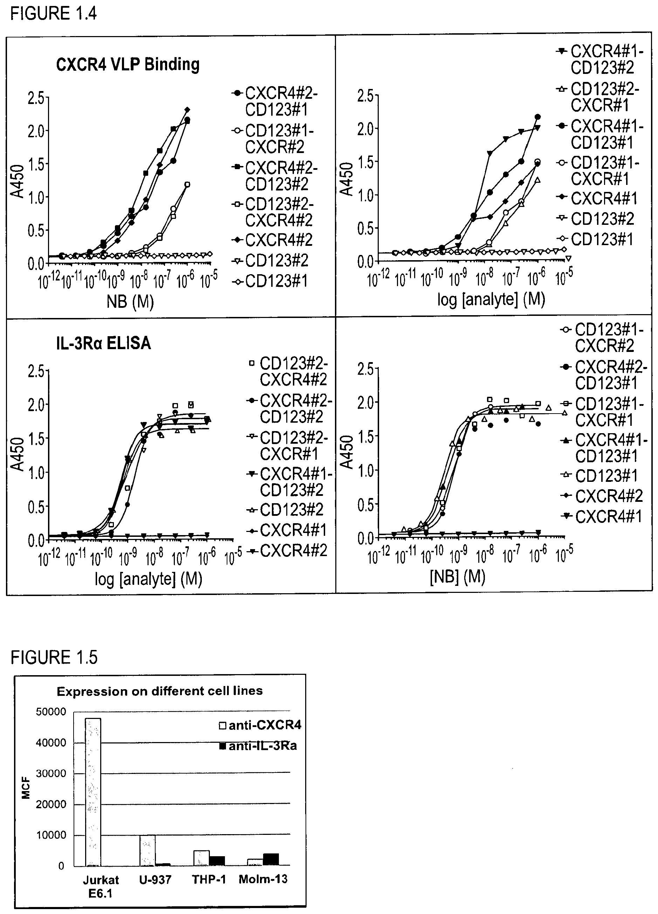

To assess if the formatting into bispecific constructs affected the target binding capacity of the individual Nanobodies, the bispecific Nanobodies were analysed for binding to recombinant IL-3Ra (R&D Systems) in ELISA and to CXCR4 viral lipoparticles (Integral Molecular). Figure 1.4 shows that the IL-3Ra binding ability of CD123#1 (57A07) and CD123#2 Nanobodies is retained in all bispecifics. However, CXCR4 binding of constructs with either CXCR4#1 or CXCR4#2 is retained only in one orientation, when the CXCR4 Nanobody is at the N-terminal position. The bispecific constructs where the CXCR4 Nanobody is positioned C-terminal show a 50-100-fold loss in binding to CXCR4-VLPs.

### Example 1.5.3 Leukemic cell lines expressing CXCR4 and Il-3Ra

Leukemic cell lines with different expression levels of CXCR4 and CD123 as well as Jurkat cells were used to assess the binding characteristics of the bispecific CXCR4-IL-3Ra polypeptides and their monovalent counterparts. Target expression was confirmed by FACS analysis with anti-hCXCR4 antibody 12G5 (R&D Systems MAB170) and anti-hIL-3Ra antibody 7G3 (BD Pharmingen, 554527), followed by secondary antibody goat-anti-mouse PE (Jackson Immuno Research).

The results are depicted in Figure 1.5.

MOLM-13 cells and THP-1 cells have different relative expression levels of the CXCR4 and Il3Ra, with hIL3Ra expression being higher compared to CXCR4 in Molm-13 than in THP-1 cells. U937 cells express the highest levels of CXCR4 and virtually no IL-3Ra.

### Example 1.5.4 Binding analysis of CXCR4-CD123

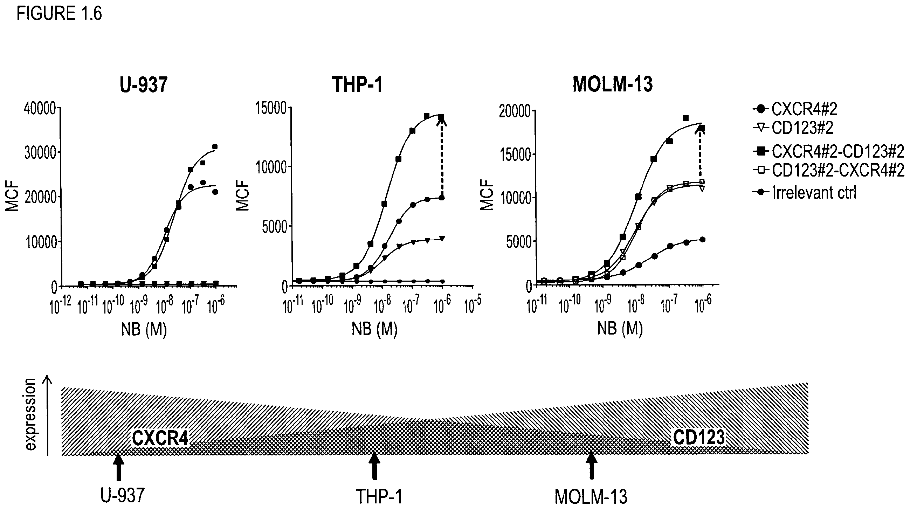

Binding of bispecific polypeptides in both orientations was analysed on U937 cells expressing only CXCR4, and MOLM-13 and THP-1 cells expressing both targets at different ratios Representative graphs are shown in Figure 1.6. In the CXCR4-IL-3Ra orientations, the affinity of the bispecific Nanobodies is improved on Molm-13 cells compared to the monovalent CXCR4 Nanobody, where the EC50 reflects those of the respective monovalent IL-3Ra Nanobody present in the construct. Beside a shift in EC50 value, also the total binding seems increased for the bispecifics in which the affinity for CXCR4 is maintained (CXCR4-IL-3Ra orientation). On Molm-13 cells the binding curves of constructs in which the CXCR4 binding is strongly reduced (IL-3Ra-CXCR4 orientation) are overlapping with the respective IL-3Ra Nanobody. This is in line with the higher expression levels of CD123 over CXCR4 in Molm-13 cells.

The differences in total fluorescence levels between THP-1 and MOLM-13 cells indicates that also the relative expression levels of the two antigens on the cell appear also to contribute to the binding behaviour of the CXCR4-IL-3Ra bispecific polypeptides (Figure 1.6).

### Example 1.5.5 Mixing of cell lines Jurkat E6-1 and MOLM-13

The ability of bispecific polypeptides to preferentially bind a cell that expresses both CXCR4 and CD123, rather than a cell expressing CXCR4 alone was evaluated. To this end, a FACS experiment with a mixed population of double-positive (MOLM-13) and CXCR4-only (Jurkat E6-1) cells was done, mimicking the real-life situation with heterogeneous cell populations. In order to distinguish both cell populations, prior to the incubation with the Nanobodies, MOLM-13 cells were labelled with 0.5 µM CFSE (Molecular Probes, Life Technologies) and Jurkat E6-1 with 0.5 µM PKH26 (Sigma-Aldrich), according to the manufacturer's instructions. After mixing both cell lines in the same well at a 1:1 ratio, they were incubated with 6-fold serial dilutions of the different bispecific polypeptides and corresponding monovalent building blocks. The dose-dependent binding of the Nanobodies was detected via the C-terminal FLAG tag using mouse anti-FLAG (Sigma-Aldrich), followed by anti-mouse IgG-APC (Jackson Immununoresearch) and measure with FACSCanto II (Becton, Dickinson and Company). As a control, Nanobody binding was also assessed on either cell line alone.

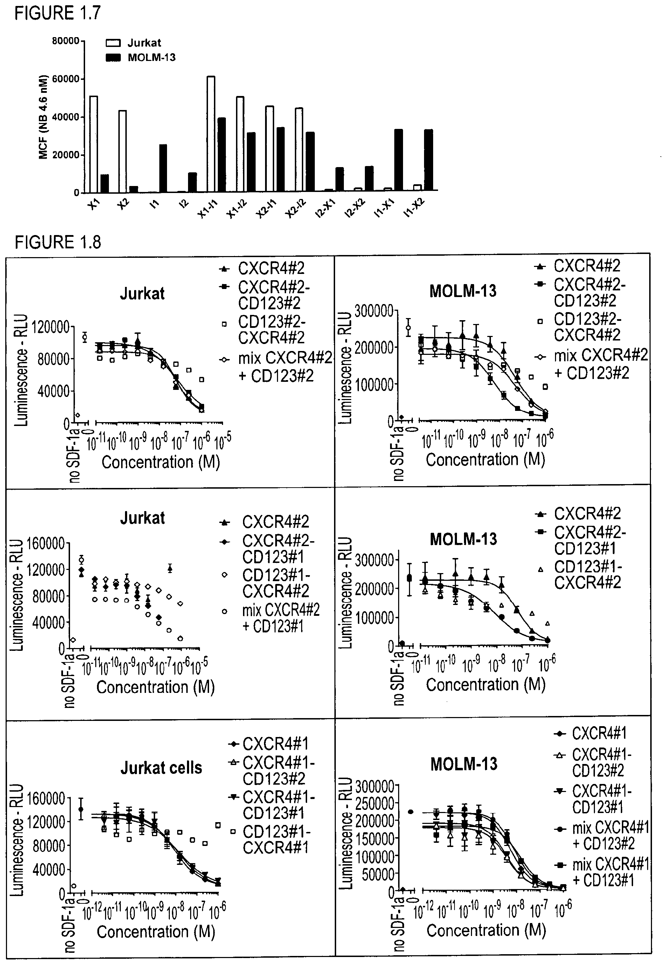

As a consequence of the low affinity of the bispecific polypeptide in the IL3Ra-CXCR4 orientation, no EC50 values could be obtained for these constructs. Therefore a direct comparison between the binding to MOLM-13 (CXCR4+/CD123+) versus Jurkat E6-1 (CXCR4+/CD123-) cells was made at one Nanobody concentration (4.6 nM). Figure 1.7 indicated that preferential binding to MOLM-13 cells was observed for bispecific constructs in the IL3Ra-CXCR4 (I-X) orientation, where the affinity for CXCR4 was compromised. Constructs with the inverse orientation, where CXCR4 monovalent is at N-terminal and its affinity is maintained, bound to both Jurkat E6-1 and MOLM-13 cells at the approximate same level, thus showing improvement in binding to MOLM-13 at this concentration.

This may indicate that the affinity of the currently used CXCR4 Nanobodies (i.e. EC50 around 10 nM) may still be too high to obtain a gain in selectivity via bispecific binding. To achieve this preferential binding, the result suggests that the affinity for CXCR4 may even be lower, e.g. to the level of the residual binding of the IL3Ra-CXCR4 constructs.

### Example 1.5.6 Inhibition of CXCR4-mediated chemotaxis

To verify if bispecific CXCR4-IL3Ra polypeptides show increased affinity and potency on cells expressing both receptors, a CXCR4-dependent functional assay was carried out. To this end SDF-1a dependent chemotaxis on Jurkat E6-1 (CXCR4+/IL3Ra-), and MOLM-13 cells (CXCR4+/IL3Ra+) was performed for direct comparison of cells expressing both or only one receptor. Since the functional blockade is only mediated via CXCR4, avidity by the simultaneous binding of the anti-IL3Ra Nanobody® is expected to translate into increased potency in inhibition of chemotaxis.

Bispecific polypeptides were analyzed for inhibition of CXCL12-induced chemotaxis on cells endogenously expressing CXCR4. As chemoattractant a concentration of 750pM SDF-1a was used on 100,000 cells/well for the Jurkat cell line, and 500,000 cells/well for the MOLM-13 cell line. On each plate the corresponding monovalent CXCR4 Nanobody was included as reference, allowing to calculate the fold increase of the bispecific within each plate. As additional control 1:1 mixtures of monovalent Nanobodies were included. Representative graphs of the different constructs during are shown in Figure 1.8. In Table 9 the respective IC50 values are shown (average of n=3 experiments).

These data show a clear gain in potency in inhibition of CXCR4 function for bispecifics in the CXCR4-IL-3Ra orientation on cells that express both antigens, but not on cells that express only CXCR4. This increase was not observed when a mixture of the two monovalent Nanobodies was used, hence depends on linking of the Nanobodies for simultaneous engagement of the targets. The potency enhancements for bispecific constructs of Nanobody CXCR4#2 on Molm-13 cells were 12-15 fold. There seemed no apparent difference between the two IL-3Ra Nanobodies, suggesting that the 8 nM affinity of the lower building block is already sufficient to serve as anchor. The gain in potency is less remarkable for the bispecific constructs of CXCR4#1 building block, where there is only a minor increase compared to the monovalent Nanobody. The potency of CXCR4#1 is higher than for CXCR4#2 (IC50 of 10 nM vs 84 nM), which may indicate it is too high to see an improvement after formatting into bispecific. Alternatively, it is also possible that this Nanobody binds to a different-less favourable- epitope on CXCR4 which limits the formatting potential.

Representative graphs of the different constructs are shown in Figure 1.8. In Table 8 the IC50 values are shown (n=2-3 experiments).

These data show that bispecifics show a gain in potencies, improving the potency of the CXCR4 Nanobodies to inhibit SDF-1 induced chemotaxis of MOLM-13 cells up to 12-15 fold.

### Example 2: Preferential targeting of T cells with CD4-CXCR4 bispecific polypeptides

### Example 2.1: Characteristics of monovalent Nanobodies for formatting

A panel of CD4 Nanobodies was previously identified from immune libraries with human peripheral blood lymphocytes. Besides its role on T cells, CD4 also serves as primary receptor for HIV1 entry. Therefore a panel of CD4 Nanobodies was analysed for the capacity to block the interaction with the viral gp120 protein. Briefly, CD4 Nanobodies were analysed for the ability to compete with gp120 protein binding to recombinant CD4 in ELISA. Briefly, plates were coated with 20ug/mL sheep anti-gp120 antibodies. 1ug/mL of HIV1 gp120 protein was captured for 1 hr at room temperature. Biotinylated recombinant human CD4 (Invitrogen) at 0.5 µg/mL was pre-incubated with 500 nM anti-CD4 Nanobodies, or control antibodies mouse anti-CD4 mAb B-A1 and F5 (Diaclone) and rabbit anti-CD4 pAb (ImmunoDiagnostic Inc) for 1hr, after which mixture was transferred to the coated plates and incubated for 1hr. Detection of bound CD4 was done with Extravidin-peroxidase conjugate. Figure 2.1 shows that only clone was found to inhibit the interaction with gp120, i.e. Nanobody 3F11. Binding to cell-expressed CD4 of 3F11 was demonstrated by flow cytometry on MOLM-13 cells, and human T-cells, using detection of the anti-flag-tag, with apparent affinities of 0.76 nM. Characteristics of Nanobody CD4 are found in Table 2.1.

**Table 2.1 Characteristics of monovalent CD4 Nanobody.**

| **Nanobody** | | **FACS binding** | | | **HIV-1 neutralization** |
|---|---|---|---|---|---|
| | **ID** | MOLM-13 EC50 (nM) | THP-1 EC50 (nM) | T cells EC50 (nM) | PMBCs + NL4.3 IC50 (nM) |
| **CD4#8** | **3F11** | 0.7 | 1.0 | 0.76 | 29.3 |

### Example 2.2 Construction of bispecific CXCR4-CD4 polypeptides

Constructs of the anti-CD4 Nanobody 3F11, designated as CD4#8, and anti-CXCR4 Nanobody 282F12, designated as CXCR4#2, were cloned in the production vector pAX100. This vector is derived from pUC119 and contains a LacZ promoter, a kanamycin resistance gene, a multiple cloning site, an OmpA leader sequence, a C-terminal c-myc tag and a (His)6 tag. Since both targets act as co-receptors for HIV-1 entry, they are expected to be in close proximity on the cell surface. For this reason bispecific polypeptides were generated with flexible spacers of different lengths for linking the two Nanobody building blocks: (Gly₄SerGly₄) (9GS), (Gly₄Ser)₅ (25GS), and (Gly₄Ser)₇ (35GS), respectively. Bispecific constructs were generated in both orientations, yielding 8 different bispecific constructs (Table 2.2). The correct nucleotide sequence of all constructs was confirmed by sequence analysis (see Table 10 for an overview of all sequences). Subsequently, the correct Nanobody constructs were recloned into the pAX205 vector for production in the yeast *Pichia pastoris* as FLAG3-His6-tagged proteins, as described in Example 1.2.

**Table 2.2**

| **Panel of CXCR4-CD4 Nanobodies** | |
|---|---|
| **CD4#8-CXCR4#2** | **03F11-9GS-281F12** |
| | **03F11-25GS-281F12** |
| | **03F11-35GS-281F12** |
| **CXCR4#2-CD4#8** | **281F12-9GS-03F11** |
| | **281F12-25GS-03F11** |
| | **281F12-35GS-03F11** |

### Example 2.3 Binding analysis of bispecific CXCR4-CD4 polypeptides

To assess if the formatting into bispecific constructs affected the binding of the CXCR4#2 Nanobody to CXCR4, the entire set of bispecific polypeptides was analysed for binding to CXCR4 on viral lipoparticles (Integral Molecular). Briefly 2 units of null VLPs and hCXCR4 VLPs were coated on maxisorp plates overnight at 4ºC. In the next day free binding sites were blocked using 4% marvel skimmed milk in PBS for 2h at room temperature. Then, after washing the plate 3x with PBS, 100nM, 10nM, 1nM and 0nM of purified polypeptides were added to the coated wells and incubated for 1h at room temperature. After washing 3x with PBS, bound polypeptides were detected with mouse anti-c-myc (Roche, cat# 11667149001) and rabbit anti-Mouse-HRP (DAKO, cat# P0260) antibodies both for 1h at room temperature. Binding was determined based on O.D. values and compared to controls: an irrelevant Nanobody, a non-coated well, both parental monovalent building blocks and a monoclonal anti CXCR4 antibody from R&D (clone:12G5, cat# MAB170). Figure 2.1 shows the results of the binding ELISA. An orientation effect for bispecific constructs with the CD4#8 Nanobody is observed, and CXCR4 binding was only retained with the CXCR4 Nanobody placed at the N-terminal position. A change in linker length could not overcome this loss of target binding of the CXCR4#2 Nanobody, except perhaps for the CD4#8-25GS-CXCR4#2 construct, which seemed to be less impaired than the two other bispecifics with the CXCR4 moiety in the C-terminal position.

The panel of CXCR4-CD4 bispecific polypeptides was analysed for dose-dependent binding to cell lines with different relative expression levels of the two targets in flow cytometry. Cells were incubated with Fc-blocking solution (Miltenyi Biotec cat# 130-059-901) for 30 minutes before staining with monoclonal anti-CXCR4 antibody 12G5 (R&D # MAB170) and monoclonal anti-CD4 antibody BA1 (Diaclone # 854030000). Bound polypeptides were detected with mouse anti-c-myc (AbD Serotec, cat# MCA2200) and Goat anti-Mouse-PE (Jackson Immunoreseach, cat# 115-115-171) antibodies both for 30min shaking at 4 °C. Binding was determined based on MCF values and compared to controls.

Expression levels of CD4 and CXCR4 on Jurkat cells, THP-1 cells and Molm-13 cells are depicted in Figure 2.3, as well as the binding curves of bispecific polypeptides to Jurkat and Molm-13 cells. Jurkat E6.1 cells show a heterogeneous population of cells expressing no or low levels of CD4. Monovalent Nanobody CD4#8 showed only a very low level of binding to these cells, although the EC50 value was similar to that on THP-1 and MOLM-13 cells (1.1 nM vs 1.0 nM vs 0.7 nM, respectively).

On Jurkat cells, the CXCR4-CD4 Nanobodies have similar EC50 values as monovalent CXCR4#2, in line with the high CXCR4 expression levels. Nanobodies have a slightly higher fluorescence level than monovalent CXCR4 Nanobodies. On double-positive THP1 cells, a clear shift in the curves of the CXCR4-CD4 bispecific Nanobodies is observed compared to both monovalents, and bispecifics reach much higher plateau levels. The difference in EC50 values between bispecifics and monovalents however is only moderate (0.67 nM vs 1.0 nM vs). On MOLM-13 cells the EC50 value of the bispecifics is similar to that of CD4#8, and also here increased plateau levels are observed. The binding curves of the inverse orientation, CD4-CXCR4 bispecifics are overlapping with the monovalent CD4#8 Nanobody.

This increase in total fluorescence in flow cytomety may represent additive binding (binding to each target alone), as well as simultaneous binding to both targets on the cell surface, but cell binding assays do not allow to discriminate between these binding modes.

### Example 2.4: Inhibition of CXCR4-mediated chemotaxis by CXCR4-CD4 bispecifics

To verify if bispecific CXCR4-IL-3Ra polypeptides show increased affinity and potency on cells expressing both receptors, a CXCR4-dependent functional assay was done. Since MOLM-13 cells express CD4 in conjunction with CXCR4 and CD123, the same experimental set-up was used as described for the CXCR4-CD123 bispecific Nanobodies (see: Example 1.5.5).

Dose-dependent inhibition of CXCL12-induced chemotaxis by the panel of bispecific CD4-CXCR4 Nanobodies was determined on Jurkat (CXCR4+/CD4 low), and Molm-13 cells (CXCR4++/CD4++). As reference anti-CXCR4 antibody 12G5 was included on each plate. Results of a representative example are shown in Figure 2.4, and IC50 values are presented in Table 2.3. Bispecific CXCR4#2-CD4#8 constructs showed strong potency enhancement (^{∼}150-fold) on double-positive cells compared to the monovalent CXCR4#2 Nanobody, whereas the CD4 Nanobody by itself did not have any affect. Remarkably, bispecific constructs in the inverse orientation were able to block CXCR4 function, despite their reduced affinity for CXCR4 due to the unfavourable position, although the blockade was partial. The much larger potency increases observed with Nanobodies targeting the CD4 and CXCR4 combination is most likely related to the higher relative expression levels of CD4 compared to CD123 on the Molm-13 cells.

**Table 2.3: Blockade of SDF-1 mediated chemotaxis by bispecific CXCR4-CD4 polypeptides.**

| **Nanobody** | | **CXCR4⁺/CD4⁺ MOLM-13 cells** | | | **CXCR4⁺/CD4^{low} Jurkat E6-1 cells** | | |
|---|---|---|---|---|---|---|---|
| **Nb1** | **Nb2** | Binding EC50 (nM) | Chemotaxis IC50 (nM) | Fold increase | Binding EC50 (nM) | Chemotaxis IC50 (nM) | Fold increase |
| **CXCR4#2** | | 5.2 | **86.0** | - | 7.0 | **84.2** | - |
| **CXCR4#2** | **CD4#8** | 1.1 | 0.59 | 146 | 11 | 110 | 0.8 |
| **CD4#8** | **CXCR4#2** | 0.7 | 1.29 | 67 | 1.1 | 460 | 0.2 |
| **CD4#8** | | 0.6 | - | - | 61 | - | - |

### Example 2.5: CXCR4 specificity in HIV1 infection assays

Besides its physiological role as homeostatic chemokine receptor, CXCR4 is also used as co-receptor for T-lymphotrophic HIV strains. For entry of the host cell, the viral gp120 protein interacts with CD4 and a co-receptor, which can be either CCR5 or CXCR4. HIV1 strains can be either dependent on CCR5 usage (R5), on CXCR4 usage (X4), or can be dual-tropic, being able to use either receptor for entry.

Modulation of either CD4 or the chemokine co-receptors are active strategies being tested in the clinic. A potential role for CXCR4 antagonists (e.g. AMD3100) in treatment of advanced stages of AIDS through inhibition of CXCR4 is anticipated, as X4 HIV-1 strains emerge late in this disease. To determine if the CXCR4#2 Nanobody is also capable of blocking the entry of CXCR4-using HIV1 strains, HIV-1 infection assays were performed with CXCR4 and CCR5 specific HIV clones. The specificity of the inhibitory effects of the monovalent and bispecific CXC4-CD4 Nanobodies was tested on CXCR4-using (X4) HIV-1 clone NL4.3 infecting MT-4 cells, or freshly isolated PBMCs (CD4+/CXCR4+/CCR5+), and the CCR5-using (R5) HIV-1 strain BaL infecting freshly isolated PBMCs (CD4+/CXCR4+/CCR5+).

### Example 2.5.1 HIV-1 infection assays

The anti-HIV-1 potencies of the entire panel of bispecific CD4-CXCR4 polypeptides and the monovalent CXCR4#2 and CD4#8 Nanobodies were determined by measuring the cytopathic effect of distinct HIV-1 strains in MT-4 and U87 cell lines, or by quantification of the viral p24 antigen production in the culture supernatant of PBMCs.

Viral strains used were the X4 HIV-1 clone NL4.3, R5 HIV-1 strain BaL, or the R5/X4 HIV-1 HE strain. Infection was done in MT-4 cells or phytohemagglutin-stimulated PBMCs from different healthy donors. The CXCR4-using (X4) HIV-1 clone NL4.3 was obtained from the National Institutes of Health NIAID AIDS Reagent program (Bethesda, MD), the CCR5-using (R5) HIV-1 strain BaL was obtained from the Medical Research Council AIDS reagent project (Herts, UK). The dual-tropic (R5/X4) HIV-1 HE strain was initially isolated from a patient at the University Hospital in Leuven. The MT-4 cells were seeded out in 96-well plate and the U87 cells in 24-well plates. Nanobodies were added at different concentrations together with HIV-1 and the plates were maintained at 37 °C in 10% CO₂. Cytopathic effect induced by the virus was monitored by daily microscopic evaluation of the virus-infected cell cultures. At day 4-5 after infection, when strong cytopathic effect was observed in the positive control (*i.e.,* untreated HIV-infected cells), the cell viability was assessed via the *in situ* reduction of the tetrazolium compound MTS, using the CellTiter 96® AQᵤₑₒᵤₛ One Solution Cell Proliferation Assay (Promega, Madison, WI). The absorbance was measured spectrophotometrically at 490 nm with a 96-well plate reader (Molecular Devices, Sunnyvale, CA) and compared with four cell control replicates (cells without virus and drugs) and four virus control wells (virus-infected cells without drugs). The IC₅₀, *i.e.,* the drug concentration that inhibits HIV-induced cell death by 50%, was calculated for each polypeptide from the dose-response curve. The CC₅₀ or 50% cytotoxic concentration of each of the polypeptides was determined from the reduction of viability of uninfected cells exposed to the agents, as measured by the MTS method described above.

Peripheral blood mononuclear cells (PBMCs) from healthy donors were isolated by density centrifugation (Lymphoprep; Nycomed Pharma, AS Diagnostics, Oslo, Norway) and stimulated with phytohemagglutin for 3 days. The activated cells were washed with PBS and viral infections were performed as described previously (Schols et al. J Exp Med 1997; 186:1383-1388). At 8-10 days after the start of the infection, viral p24 Ag was detected in the culture supernatant by an enzyme-linked immunosorbent assay (Perkin Elmer, Brussels, Belgium).

The HIV1 neutralisation results were depicted as IC₅₀ values in Table 2.4. In MT-4 cells infected with the NL4.3 strain, the CXCR4#2 Nanobody specifically inhibited anti-X4 HIV1 entry via CXCR4, but not binding to CCR5. The CD4#8 Nanobody was effectively blocking both X4 HIV1 infection, with a similar IC50 value as the CXCR4 monovalent. In this example the CD4 Nanobody is not exclusively serving as anchor, but also contributes to the functional blockade. Bispecific CXCR4#2-CD4#8 polypeptides were extremely potent in inhibiting HIV-1 X4 virus replication, especially when evaluated in PHA-stimulated PBMCs. Potency increases for the best bispecific CXCR4-CD4 construct with the shortest linker were between 250-320 fold compared to monovalent CXCR4#2 Nanobody. Bispecific Nanobodies in the inverse orientation, with the reduced affinity towards CXCR4, were less active in this functional assay. Thus, the simultaneous binding to both CXCR4 and CD4 of the bispecific CXCR4-CD4 Nanobodies results in strongly enhanced potencies in the neutralization of CXCR4-using HIV1.

**Table 2.4: Specificity for CXCR4-tropic HIV1 strain NL4.3 and the CCR5-tropic BaL.**

| IC50 (nM) n=3 | MT-4 + NL 4.3(X4) | U87 + NL 4.3 (X4) | PBMC + NL 4.3(X4) | PBMC + BaL(R5) |
|---|---|---|---|---|
| Nanobody | | | | |
| CD4#8 | 66.67 | >1333 | 580 | 610 |
| CXCR4#2 | 67.11 | >6666 | 29.3 | >1666 |
| CD4#8-9GS-CXCR4#2 | 14.89 | >3333 | 17.0 | >666 |
| CD4#8-25GS-CXCR4#2 | 9.22 | >3333 | 8.67 | 383.33 |
| CD4#8-35GS-CXCR4#2 | 11.67 | >3333 | 23.7 | 35.9 |
| CXCR4#2-9GS-CD4#8 | 0.20 | 0.53 | 0.03 | |
| CXCR4#2-25GS-CD4#8 | 0.21 | 2.67 | 0.12 | |
| CXCR4#2-35GS-CD4#8 | 0.24 | 2.67 | 0.37 | 2,46 |
| AMD3100 | 4.75 | 10 | 1.91 | |

### Example 2.5.2 Specificity

The potency of the CXCR4 Nanobody is specific for HIV1 strains that depend on CXCR4 usage for entry. One potential disadvantage of blockade of only one HIV1 co-receptors is that it may trigger the re-emergence of the HIV subtype that is not originally targeted. In case of the CCR5-dependent HIV BaL virus, only the CD4 Nanobody in the bispecific construct contributes to the virus neutralization in PBMCs. Since CXCR4 is expressed on PBMCs, in these cells the CXCR4 Nanobody in the bispecific can serve as anchor to enhance the inhibition potency of the CD4 Nanobody. Indeed bispecific CXCR4-CD4 with the longest linker has an IC50 values of 2.5 nM for BaL, around 200-fold enhancements relative to monovalent CD4#8, and are more potent inhibitors of infection than constructs in the inverse orientation, where the CXCR4 binding affinity is impaired. For the CD4-CXCR4 bispecifics a longer linker appears to give better inhibition, suggesting that this favours the binding to the CXCR4 as anchor.

### Example 2.5.3 Entry-inhibitor resistant HIV-1 NL4.3

To substantiate the contribution of the CXCR4 Nanobody as anchor in the bispecific polypeptide, blockade of HIV infection was assessed for a panel of HIV1 mutant that was made resistant for the CXCR4 small molecule inhibitor AMD3100, the CXCR-4 ligand, or the control antibody 12G2. In addition, viral escape mutants were generated for blockade of each of the monovalent Nanobodies, by culturing of NL4.3 in presence of polypeptides at IC90 concentration over multiple passages. Resistant viral clones that were thus identified were used for testing the potencies of bispecific polypeptides compared to the monovalent polypeptides. IC50 values are presented in Table 2.5. The IC50 values of the bispecific CXCR4-CD4 Nanobodies towards AMD3100 resistant virus are depicted in Figure 2.5. Monovalent CXCR4#2 showed a 100-fold loss in potency, similar as AMD3100, while the CD4 potency was unaffected. Each of the CXCR4-CD4 bispecific Nanobodies had retained potencies below 1nM for blocking infection of AMD3100 resistant virus, 20-fold better than the monovalent CD4 building block. Over the complete panel of resistant viruses, the CXC4#2-CD4#2 polypeptide retained strong neutralizing potency with IC50 values between 0.3-1.1 nM. Thus, the CXCR4-CD4 bispecific polypeptides seem relatively insensitive to mutants that no longer bind to one of the targets. Together these results indicate that bispecific polypeptides have a broad coverage in different HIV strains (see Table 2.6) and consistent high potency in blocking virus infections, and that functionality on only one of the arms of the bispecific CXCR4-CD4 polypeptides is already sufficient for the potent inhibition of these compounds in HIV entry.

**Table 2.5: Anti-HIV activity profile of Nanobodies towards entry-inhibitor resistant HIV-1 NL4.3 variants in MT-4 cells.**

| IC50 (M) | MT-4 | | | | | |
|---|---|---|---|---|---|---|
| Nanobody | NL4.3 wt | CD4#8 res. | CXCR4#2 res | AMD-3100 res. | CXCL-12res. | 2G12 res. |
| CD4#8 | 3.47E-08 | >6.7E-06 | >6.7 E-06 | 2.27E-08 | 1.53E-07 | 2.33E-08 |
| CXCR4#2 | 2.27E-08 | 8.73E-08 | 2.33E-06 | >1.67E-06 | 2.20E-07 | 1.73E-08 |
| CXCR4#2-35GS-CD4#8 | 1.87E-10 | 3.10E-10 | 1.40E-09 | 1.13E-09 | 4.33E-10 | 1.10E-10 |
| CD4#8-35GS-CXCR4#2 | 6.00E-09 | 9.57E-08 | >3.1E-07 | 1.40E-08 | 7.00E-08 | 3.00E-09 |
| AMD3100 | 4.28E-09 | 1.85E-08 | 3.99E-07 | 4.04E-07 | 5.03E-08 | |

**Table 2.6: Anti-HIV activity profile of Nanobodies towards distinct HIV strains on MT-4 cells.**

| IC50 (M) | MT-4 + | | |
|---|---|---|---|
| Nanobody | HIV-1 NL4.3 | HIV-1 HE | HIV-2 ROD |
| CD4#8 | 3.47E-08 | 1.00E-08 | 2.27E-08 |
| CXCR4#2 | 2.27E-08 | 1.00E-08 | 8.67E-08 |
| CXCR4#2-35GS-CD4#8 | 1.87E-10 | 9.06E-11 | 3.00E-10 |
| CD4#8-35GS-CXCR4#2 | 6.00E-09 | 2.00E-09 | 8.75E-09 |
| AMD3100 | 4.28E-09 | 3.90E-09 | 2.11E-08 |

### Example 3: Preferential targeting of T cell subsets with CD4-IL12Rβ2 and CD4-IL23R bispecific polypeptides

### Example 3.1: Characteristics of monovalent Nanobodies used for formatting

To generate bispecific polypeptides with the capacity to preferential block specific T cell subsets, Nanobodies directed against different subset-specific interleukin receptors were combined with a Nanobody directed against the CD4 glycoprotein. On the functional arm the IL-12Rβ2 was used as marker for the T_{H1} cell subset, and IL-23R as marker for T_{H17} cells. Both receptors belong to the same interleukin 12 receptor family and use the same co-receptor IL-12Rβ1 to form a functional heterodimer. For this reason also bispecific constructs of IL-12Rβ1 and CD4 were generated, as these are expected to target both T cell subsets and hence can serve as positive control. An anchoring Nanobody directed against the CD4 glycoprotein is used, to provide avidity and to prevent blockade of receptors on other immune cells, such as CD8+ T cells, B cells, natural killer cells and certain myeloid cells.

For this example, Nanobody 3F11 directed against the CD4 glycoprotein was used as common anchor. This Nanobody is specific for cell-expressed human CD4, and shows only low binding to recombinant CD4 protein, and was used in the generation of CXCR4-CD4 bispecifics (see Example 2). The Nanobodies specific for IL-23R, IL-12Rβ1 and IL-12Rβ2 were previously identified as ligand competing Nanobodies. To identify ligand-competing Nanobodies with sufficient low affinities for formatting, monovalent Nanobodies from families with multiple family members were characterised with respect to binding kinetics, ability to compete with ligand, and binding to cell-expressed receptors on primary cells.

### Example 3.1.1 SPR

The precise binding affinities of the purified Nanobodies were determined in a multi-cycle kinetic analysis using Surface Plasmon Resonance analysis (Biacore T100) on Fc-fusions of human IL12Rβ1, IL12Rβ2 and IL-23R extracellular domains (R&D Systems, #839-B1, #1959-B2, #1400-IR). Sensorchips CM5 were immobilized with anti-hlgG antibody (GE Healthcare, BR-1008-39), after which receptors were captured at 5 µg/ml protein and contact time of 120 seconds. Running buffer used was HBS-EP+ (GE Healthcare, BR-1006-69) at 25°C, with a flow-rate of 5 ml/min. For immobilization by amine coupling, EDC/NHS was used for activation and ethanolamine HCI for deactivation (Biacore, amine coupling kit). Nanobodies were evaluated at a concentration range between 1.37 nM and 3 µM. Nanobodies were allowed to associate for 2 min and to dissociate for 15 min at a flow rate of 45 ml/min. In between injections, the surfaces were regenerated with a 3 min pulse of 3M MgCl₂ and 2 min stabilization period. Evaluation of the association/dissociation data was performed by fitting a 1:1 interaction model (Langmuir binding model) by Biacore T100 software v2.0.3. The off-rates and affinity constants are shown in Table 3.1.

### Example 3.1.2 Competition for IL-12 and IL-23 binding in ELISA

The ability of monovalent Nanobodies to compete with binding of IL12 receptor-Fc proteins to IL-12 was assessed in a competition ELISA on coated human IL-12 (10 nM, Peprotech #200-12B) in a 384-well SpectraPlate HB microtiter plate (Perkin Elmer). Free binding sites were blocked with 1% casein in PBS. Serial dilutions of Nanobodies with a fixed concentration of either 2 nM IL12Rβ1-Fc or 3 nM IL12Rβ2-Fc were incubated for 1 hr. Concentration of competitors was based on dose-titration experiments, and final concentrations used were <EC₅₀ values. Residual binding of IL12Rβ1-Fc or IL12Rβ2-Fc was detected using a HRP-conjugated goat anti-hlgG antibody (1/3000, Jackson ImmunoResearch, Cat# 109-035-088) and a subsequent enzymatic reaction in the presence of the substrate esTMB (SDT reagents).

Similar assay set-ups were used for measuring the competition of IL-23R and IL12Rβ1 Nanobodies for binding to IL-23. A coating of human IL-23 (eBioscience 34-8239-82) at 20 nM was used for competition with 5nM IL-23R-Fc, a coating of 3 nM was used for competition of 2 nM IL12Rβ1-Fc. The IC50 values are shown in Table 3.1. The difference in ligand competition ability between the family members for each of the IL12 receptor subunits correlates well with the difference in K_{D} values measured.

### Example 3.1.3 Flow cytometry

Dose-dependent binding of monovalent Nanobodies to their cell-expressed receptor in the context of the heterodimeric complex was determined by flow cytometry on activated human T cells from distinct healthy donors.

Human T cells were isolated using the Human T Cell Enrichment Cocktail (RosetteSep #15061) and pre-activated for four days with Dynabeads® Human T-Activator CD3/CD28 (Gibco - Life Technologies #11131D) and one day with recombinant human IL-2 (Life Technologies - Gibco #PHC0027) to induce T_{H1} differentiation. Routinely, T cell markers surface expression and activation state was checked by FACS using anti-CD3 PE (eBioscience #12-0037-73), anti-CD8-PE (BD Bioscience #555367), anti-CD45RO-PE (BD Bioscience #555493), anti-CD45RA-APC (BD Bioscience #550855) anti-CD25-PE (BD Bioscience #557138) and anti-CD69-PE (BD Bioscience #557050). IL12R surface expression was confirmed by FACS using IL12Rβ1 antibody (R&D MAB839), followed by goat anti-mouse PE (Jackson Immuno Research 115-115-164). The expression of IL23R was checked by polyclonal goat anti-IL-23R (R&D AF1400). CD4 surface expression was confirmed by FACS using APC-labelled anti-CD4 (BD Bioscience #345771). In Figure 3.1 the expression levels of IL12Rβ1, IL23R and CD4 on T cells of one donor activated with this protocol with control antibodies are shown. For IL12Rβ2 none of the commercially available tools showed substantial binding.

As the expression of IL23R was very low in the T cell pool, the binding of monovalent IL23R Nanobodies was assessed on cells that were differentiated towards the Th17 phenotype by the incubation of PBMCs in the presence of a cytokine cocktail and IL-23, recombinant IL-6 (eBioscience #34-8069-82), recombinant TGF-b1 (R&D #240-B), anti-human IL-4 antibody (BD#554481), recombinant IL-1b (BD#554602)) and recombinant Human IL-23 (R&D Systems #219-IL-005) with co-stimulation of plate coated OKT-3 (eBioscience #16-0037-85), PeliCluster CD28 (Sanquin #M1650). Following this procedure, low but detectable IL23R expression levels were obtained. Optimization in the Th17 differentiation protocol could further increase these expression levels.

Dose-dependent binding of monovalent Nanobodies was assessed by flow cytometry on the respective Th1 or Th17 enriched T cell populations. Serial dilutions of antibody or Nanobodies were allowed to associate for 30 minutes at 4°C in FACS buffer (PBS supplemented with 10% FBS and 0.05 % azide). Cells were washed by centrifugation and probed with anti-FLAG antibodies (Sigma F1804) for 30 minutes at 4°C, to detect bound Nanobody. Detection was done with Goat anti-Mouse IgG-PE (Jackson ImmunoResearch #115-116-071) for 30 minutes at 4°C. Cells were washed and incubated with TOPRO3 to stain for dead cells, which are then removed during the gating procedure. The cells were then analysed via a BD FACSArray.

Specific Nanobody binding curves are shown in Figures 3.2 and 3.3. Monovalent Nanobodies are able to specifically bind to cell-expressed IL12Rβ1, respectively IL12Rβ2, in the presence of the heterodimeric receptor complex (Figure 3.2). The difference in binding affinity of the IL12Rβ1 family members clearly translates into different cell binding apparent affinities, while the EC₅₀ values of the two IL12Rβ2 family members on these cells are very similar. In each case the Nanobody with the faster off-rate typically reaches a lower plateau level. Due to its fast off-rate, binding curves were incomplete for IL12Rβ1#31 with respect to saturation of binding.

Specific binding of the IL-23R and IL12Rβ1 Nanobodies with the highest affinity was observed on the T_{H17}-enriched population, although the fluorescence signals were very low (Figure 3.3). This may indicate that the % of T_{H17} cells expressing IL23R in the T cell pool is still relatively low for obtaining dose response curves with low affinity monovalent Nanobodies.

The characteristics of the IL-23R, IL-12Rβ1 and IL-12Rβ2 Nanobodies selected for formatting into bispecific Nanobodies are presented in Table 3.1. We aimed to select Nanobodies with distinct off-rates belonging to the same family, i.e. with sequence conservation in their CDR3 regions, so that the epitope on the target was conserved and the effect of affinity could be addressed. Ideally Nanobodies with off-rates >10⁻⁴ s⁻¹ were chosen, to maximise the avidity effect provided by the anchoring Nanobody. The sequences of the two selected IL12Rβ2 Nanobodies differ in three amino acids in CDR1 and CDR2 regions, and show a 3.5-fold difference in K_{D} and ligand competition ability due to a difference in off-rate. The two selected IL12Rβ1 Nanobodies differ in six amino acids in the CDR1 and CDR3 regions, with a 6-7-fold difference in K_{D} and ligand competition. For the IL23R Nanobodies it proved not feasible to identify two family members with a substantial difference in off-rate. Therefore for this receptor two ligand competing Nanobodies with different fast off-rates from distinct families, hence with potentially different epitopes, were selected. Although cell binding could not always be accurately measured for the Nanobodies with fast off-rates (>1.E⁻⁰²), ligand competition assays demonstrated functional blocking with IC50 ranging between 10-16 nM for all selected monovalent Nanobodies.

### Example 3.2: Generation of bispecific Nanobodies

Formatting of bi-specific CD4-IL-12Rβ2, CD4-IL-12Rβ1 and CD-IL-23R polypeptides was done by genetic fusion of Nanobodies linked with a long flexible (GGGGS)7 linker, with the building blocks in both orientations. For each combination, two functional blocking receptor-specific Nanobodies were combined with one anti-CD4 Nanobody, CD4#8 3F11 (Figure 3.4). The correct nucleotide sequence of all constructs was confirmed by sequence analysis (see Table 12 for an overview of all sequences). Nanobodies were generated as flag3-His6-tagged proteins for expression in the yeast *Pichia pastoris* X-33, and purified from the culture medium using standard affinity chromatography, followed by size exclusion chromatography. All proteins were confirmed endotoxin-free for use in assays on primary cells.

### Example 3.3: Binding analysis of bispecific Nanobodies

### Example 3.3.1 Effect of formatting

To assess whether the orientation of the Nanobody after formatting affects the binding and functionality to the respective interleukin receptor, purified monovalent and bispecific Nanobodies were analysed for competition with either hIL-12Rβ1-Fc, hIL-12Rβ2 -Fc or IL-23R-Fc fusions for ligand binding (see above). Dose-dependent inhibition of both monovalent Nanobodies and bispecifics was carried out to determine IC₅₀ values for competition on plates coated with human IL-12. Similarly, a competition ELISA on plates coated with human IL-23 was performed to assess the functionality of bispecifics of the IL-23R and IL-12Rβ1 Nanobodies. The IC50 values are shown in Tables 3.2 and 3.3. In case of CD4, orientation effects were assessed by flow cytometry, comparing binding of monovalent Nanobodies and bispecific polypeptides to MOLM-13 cells that express CD4 but lack IL12R and IL23R. The CD4 expression was confirmed by FACS using the anti-human CD4 APC (BD Bioscience, #53384). Figure 3.5 shows that the formatting into bispecific polypeptides did not substantially affect the binding of the CD4 building block to cell-expressed CD4. Bispecific polypeptides showed binding comparable as the monovalent CD4#8 Nanobody, with the exception of IL23R#19-CD4#8 (BI#42), which showed a small drop in binding affinity. Neither of the monovalent IL12Rβ2, IL12Rβ1 and IL23R Nanobodies bound to MOLM-13 cells, confirming the absence of the IL12 and IL23 receptor expression.

### Example 3.3.2 Specificity

Dose-dependent binding of bispecific polypeptides was assessed on human T cells that were activated to increase expression levels of IL12R. Activated T cells showed relative moderate expression levels of the IL12R antigen, but very high CD4 expression, reflected in the high apparent affinity and high fluorescence signal of the anti-CD4 Nanobody. Simultaneous binding to the two target receptors is not apparent, as the binding curves of all bispecific Nanobodies overlap with those of the monovalent CD4 Nanobody, giving similar EC50 values (Figure 3.6).

The pool of activated T cells comprises both CD4+ T cells and CD8+ T cells. To confirm the specificity of the anti-CD4 Nanobody and to exclude binding to CD4-negative cells, binding was assessed to cytotoxic CD8+ T cells isolated from human PBMCs using the CD8+ T Cell Isolation Kit (Miltenyi Biotech, 130-096-495), resulting in 94% purity of CD8+ cells. Binding specificity experiments were carried out using Nanobodies at 250 nM. No binding was observed with the anti-CD4 Nanobody, while monovalent IL12Rb1#30 did bind to isolated CD8+ T cells (Figure 3.7). In addition, bispecific polypeptide IL12Rβ1#30-CD4#8 bound these cells to a similar level as monovalent Nanobody IL12Rβ1#30, without additional effect of the CD4 anchor. Similar data were obtained for the IL12Rβ2 and IL23R Nanobodies. These results indicate that the bispecifics of CD4 with the subset-specific receptors do not bind to cytotoxic CD8+ T cells but specifically interact with CD4+ T cells.

To elucidate if CD4-IL12R bispecific polypeptides preferentially bind to the CD4+/IL12R+ T_{H1} cell subset within the pool of T cells, Nanobody binding was analysed to a pool of activated T cells gated for either CD8 (detected by Anti-hu CD8 PE-Cy7 conjugated monoclonal antibody (BD 557746) or CD4 (detected by Anti-hu CD4 alexa Fluor 488-conjugated polyclonal antibody (R&D FAB8165G) in a multi-colour FACS experiment. Nanobody binding to the CD8+/CD4- gated cells and to CD4+ gated cells was determined using anti-flag-APC (Prozyme PJ255) detection. In this experiment T_{H1} activated T cells from the same donor (D838) as shown in Figure 3.6 were used. The CD4#8 Nanobody showed strong binding to the CD4+ gated population, as indicated by high fluorescence levels (Figure 3.8 panel E, light grey peak), while only a low signal was observed to the CD8+ population (dark grey peak). It was noticed that the CD4 Nanobody competed to a small extent with the anti-CD4 polyclonal Abs used for gating, which may have resulted in incomplete separation of the CD4+ and CD4- cells. For the monovalent IL12Rβ1#30 and IL12Rβ2#1 Nanobodies low fluorescence signals to both CD4+ and CD8+ cells were observed, indicating that Nanobodies bound weakly to both T cell subsets. The bispecific polypeptides IL12Rβ1#30-CD4#8 and IL12Rβ2#1-CD4#8 showed preferential binding to the CD4+ population over the CD8+ subset, indicating that these Nanobodies conferred the specificity of the CD4 Nanobody.

### Example 3.4: Functional Characterization of bispecific polypeptides

### Example 3.4.1: Cell-specific blockade of IL-12 function in human T cells

The ability of bispecific polypeptides to simultaneously engage both targets on the same cell was analysed in a IL-12 dependent functional assay, inhibition of IL-12 mediated IFN-γ release in activated human T cells. Since the functional blockade is only mediated via IL12R, avidity by the simultaneous binding of the CD4 Nanobody is expected to translate into increased potency of the bispecific in inhibition of cytokine release.

Isolated human T cells from buffycoats were activated for four days with Dynabeads® Human T-Activator CD3/CD28 (Gibco - Life Technologies #11131D) and one day with IL-2. To differentiate into Th1 subtype, T cells were cultured in presence of IL-12 with co-stimulation provided by plate coated CD3 at 0.5µg/ml (eBioscience #16-0037-85) and anti-CD28 (1µg/ml PeliCluster, Sanquin #M1650) in solution. Concentration of ligand used, 0.2 pM was based on dose -titration experiments, using concentration < EC50.As measure for IL-12 dependent signaling, release of the typical Th1 cytokine IFN-γ was measured after 72h in the presence or absence of the respectively Nanobodies by ELISA.

Dose-dependent blockade of IL-12 mediated IFNγ release was assessed for the bispecific IL-12Rβ2-CD4 and IL-12Rβ1-CD4 polypeptides in both orientations, and the corresponding monovalent Nanobodies. The IL-23R-CD4 bispecific polypeptides served as negative controls. Representative graphs of the bispecific IL12Rβ1-CD4, IL12Rβ2-CD4 and IL23R-CD4 polypeptides are shown in Figure 3.9, and IC50 values in Table 3.2. All four IL12Rβ2-CD4 bispecific polypeptides showed a shift in IC50 values between 74-1100 compared to their respective monovalent IL12Rβ2 Nanobody (Table 3.3), while the bispecific CD4-IL23R polypeptides were not blocking. Also ^{∼}500-fold potency differences were observed for the IL12Rβ1-CD4 bispecifics. Although bispecific constructs in both orientations show potency enhancements, the IL12Rβ2 Nanobody in the N-terminal position from CD4 gave stronger enhancements. Together these data show that both the IL12Rβ1-CD4 and the IL12Rβ2-CD4 bispecifics show a 400-1000 gain in potency on T_{H1} cells that express both antigens, and that CD4 binding by itself was not interfering.

To verify if this selective functionality of the bispecific polypeptides on T_{H1} cells was preserved in PBMCs, where also other immune cells were present, the same assay was performed using activated healthy human PBMCs. T cells within the PBMC pool were differentiated towards the T_{H1} subtype using 0.1pM IL-12. IFN-γ release in the presence or absence of the respectively Nanobodies was determined by ELISA after an incubation period of 6 days. A representative example of IL12 blockade of bispecific polypeptides in PBMCs is shown in Figure 3.10. Also in a PBMC-based assay a clear gain in potency for each of the IL12Rb1-CD4 and the IL12Rb2-CD4 bispecifics was observed, with shifts in IC50 values between 10-50 fold relative to the respective monovalent Nanobodies. PBMCs from two distinct donors were tested, with similar results. Monovalent CD4 Nanobody and CD4-IL23R bispecific polypeptides have no effect, indicating that also in the PBMC context selective functional blockade is obtained by bispecific polypeptides in a T cell subset-specific manner.

### Example 3.4.2: Cell-specific blockade of IL-23 function

To verify if bispecific polypeptides targeting the functional IL23 receptor showed increased affinity and potency on cells that co-express CD4 and IL23R, the ability of Nanobodies to inhibit IL23-dependent release of the Tₕ₁₇ type cytokine IL17 was measured. In this assay set-up human PBMCs were cultured in the presence of soluble IL23 to allow differentiation of T cells towards the Tₕ₁₇ phenotype. Cells were seeded onto OKT-3 (eBioscience #16-0037-85) coated plates in the presence of recombinant human IL-23 (eBioscience #14-8239) and PeliCluster CD28 (Sanquin #M1650) in solution. Cytokine (IL17) release in the presence or absence of the respectively Nanobodies was determined by ELISA after an incubation period of 9 days.

Dose-dependent inhibition of the panel of bispecific IL23R-CD4 and IL12Rβ1-CD4 polypeptides was assessed in comparison to the respective monovalent Nanobodies, with in this case the IL12Rβ1-CD4 specific polypeptides serving as negative controls. Figure 3.11 shows that the bispecific IL12Rβ1-CD4 polypeptides strongly inhibit the IL23 mediated IL17 release in a dose-dependent manner, with between 500-1700-fold enhanced potencies relative to the monovalent IL12Rβ1 Nanobodies (Table 3.3). There is a preference for the IL12Rβ1 building block in the C-terminal position from CD4 in this assay. There is a clear difference in potency between the two IL12Rβ1 family members, corresponding to the different binding kinetics and affinities, and this difference is preserved in the potency of the bispecific constructs. No inhibition is observed for the IL12Rβ2-CD4 bispecific polypeptides, nor for the anti-CD4 Nanobody, indicating that the blockade was subset specific.

For the bispecific constructs of IL23R and CD4 there is also a difference in potency observed between monovalent Nanobodies and bispecific polypeptides (Figure 3.11, panel C), although IC50 values cannot be determined for all Nanobodies. The difference in affinity of the monovalent Nanobodies is reflected in the potencies of the monovalent Nanobodies in the IL23 functional assay, but this difference is not as clear for the bispecific constructs. The IL23R Nanobodies are not family members, and the epitope of the Nanobody IL23R#19 may be less optimal than IL23R#20 for simultaneous binding to CD4 on the cell membrane. As the % of Tₕ₁₇ T cells obtained with in the PBMC pool was rather low, further optimization of the Tₕ₁₇ differentiation protocol could further substantiate the observed differences. In addition, PBMCs derived from patients suffering typical T_{H17} inflammatory disease, such as psoriasis, could provide a better IL23 response. These PBMCs represent a physiological mixture of T cell subsets, with expression levels of IL23R and IL12R to be expected in a relevant Tₕ₁₇ disease setting.

Taken together, these results indicate that T_{H1}-subset specific CD4-IL12Rβ2 and T_{H17}-Subset specific CD4-IL23R polypeptides show selective functional blockade in a T cell subset-specific manner, in assays with heterogeneous T cells as well as PBMCs. Furthermore, selective binding of the bispecific polypeptides to CD4+ T cell subsets was shown, whereas monovalent IL12Rβ2 Nanobodies showed only poor binding to CD4 and CD8 T cells.

With respect to affinities, even low affinity Nanobodies on the functional arm gave potency enhancements of 2-3 logs upon formatting with a high affinity anchoring CD4 Nanobody.

**Table 3.1: Characteristics of monovalent IL-12Rb2, IL-12Rb2 and IL-23R-specific Nanobodies**

| **Nanobody ID** | **Binding kinetics (SPR)** | | | **Inhibition of ligand binding (ELISA)** | **Binding T cells (FACS)** |
|---|---|---|---|---|---|
| | **kₐ (1/Ms)** | **k_{d} (1/s)** | **K_{D} (M)** | **IC₅₀ (M)** | **EC₅₀ (M)** |
| **IL12Rb2#1:135B08** | **2.1E+05** | **5.7E-04** | **2.7E-09** | **4.2E-09** | **1.5E-09** |
| **IL12Rb2#2:135A07** | **2.7E+05** | **1.8E-03** | **6.9E-09** | **1.5E-08** | **1.8E-09** |
| **IL12Rb1#30:148C09** | **4.5E+05** | **1.5E-03** | **3.3E-09** | **3.7E-09 (IL-12), 1.5E-9 (IL-23)** | **1.3E-09** |
| **IL12Rb1#31:148F09** | **7.2E+05** | **1.7E-02** | **2.3E-08** | **2.2E-08 (IL-12), 1.0E-8 (IL-23)** | *No fit* |
| **IL23R#19:150D02** | **7.3E+05** | **8.1E-03** | **1.1E-08** | **4.7E-09** | **2E-08** |
| **IL23R#20:150H07** | **3.0E+06** | **2.3E-01** | **7.8E-08** | **1.6E-08** | *No fit* |

**Table 3.2: Inhibition of IL-12 function by panel of monovalent and bispecific IL12Rb1-CD4, IL12Rb2-CD4, and IL23R-CD4 Nanobodies.**

| | | | **Inhibition of IL-12** | | | | | |
|---|---|---|---|---|---|---|---|---|
| **Nanobody ID** | **Nb1** | **Nb2** | **comp ELISA IC50 (M)** | **fold ctrl** | **IFNγ release T cells D839 IC50 (M)** | **fold ctrl** | **IFNγ release PBMC D840 IC50 (M)** | **fold ctrl** |
| CD4#8 | | | - | | - | | - | |
| IL23R#19 | | | - | | | | | |
| BI#42 | IL23R#19 | CD4#8 | | | | | | |
| BI#45 | CD4#8 | IL23R#19 | - | | | | | |
| IL23R#20 | | | - | | | | | |
| BI#43 | IL23R#20 | CD4#8 | - | | - | | - | |
| BI#44 | CD4#8 | IL23R#20 | - | | - | | - | |
| IL12Rb1#30 | | | **3.70E-09** | | **1.40E-07** | | **1.70E-08** | |
| BI#46 | IL12Rb1#30 | CD4#8 | 6.20E-09 | 0.6 | 2.9E-10 | **552** | 4.5E-10 | **38** |
| BI#40 | CD4#8 | IL12Rb1#30 | 7.30E-09 | 0.5 | 2.8E-10 | **429** | 3.6E-10 | **47** |
| IL12Rb1#31 | | | **2.20E-08** | | ***no fit*** | | **9.35E-08** | |
| BI#47 | IL12Rb1#31 | CD4#8 | 2.40E-08 | 0.9 | 4.1E-09 | | 1.9E-09 | **41** |
| BI#41 | CD4#8 | IL12Rb1#31 | 2.80E-08 | 0.8 | 1.7E-09 | | 2.2E-09 | **50** |
| IL12Rb2#1 | | | **4.20E-09** | | **5.10E-08** | | **1.35E-08** | |
| BI#37 | IL12Rb2#1 | CD4#8 | 4.80E-09 | 0.9 | 1.10E-10 | **400** | 1.1E-09 | **11** |
| BI#39 | CD4#8 | IL12Rb2#1 | 1.00E-08 | 0.4 | 7.8E-10 | **74** | 1.9E-09 | **8** |
| IL12Rb2#2 | | | **1.50E-08** | | **6.70E-08** | | **4.00E-08** | |
| BI#36 | IL12Rb2#2 | CD4#8 | 1.10E-08 | 1.4 | 3.1E-11 | **1129** | 1.6E-09 | **30** |
| BI#38 | CD4#8 | IL12Rb2#2 | 2.50E-08 | 0.6 | 7.6E-10 | **130** | 2.1E-09 | **8** |

**Table 3.3: Inhibition of IL-23 function by panel of monovalent and bispecific IL23R-CD4, IL12Rb1-CD4, and IL12Rb2-CD4 Nanobodies.**

| | | | **Inhibition of IL-23** | | | |
|---|---|---|---|---|---|---|
| **Nanobody ID** | **Nb1** | **Nb2** | **IL-23 comp ELISA IC50 (M)** | **fold ctrl** | **IL-17 release PBMC D840 IC50 (M)** | **fold ctrl** |
| CD4#8 | | | - | | - | |
| IL23R#19 | | | 1.70E-09 | | 1.00E-07 | |
| BI#42 | IL23R#19 | CD4#8 | 6.10E-09 | 0.3 | 5.89E-08 | **1.7** |
| BI#45 | CD4#8 | IL23R#19 | 6.60E-09 | 0.3 | 2.42E-08 | **4.1** |
| IL23R#20 | | | 1.60E-08 | | no fit | |
| BI#43 | IL23R#20 | CD4#8 | 1.10E-08 | 1.5 | no fit | |
| BI#44 | CD4#8 | IL23R#20 | 1.50E-08 | 1.1 | 2.20E-08 | |
| IL12Rb1#30 | | | 1.50E-09 | | 3.55E-08 | |
| BI#46 | IL12Rb1#30 | CD4#8 | 3.00E-09 | 0.5 | 1.6E-11 | **875** |
| BI#40 | CD4#8 | IL12Rb1#30 | 3.60E-09 | 0.4 | 3.5E-11 | **1629** |
| IL12Rb1#31 | | | 1.00E-08 | | 2.10E-07 | |
| BI#47 | IL12Rb1#31 | CD4#8 | 1.30E-08 | 0.8 | 2.3E-10 | **565** |
| BI#41 | CD4#8 | IL12Rb1#31 | 1.50E-08 | 0.7 | 1.7E-10 | **1706** |
| IL12Rb2#1 | | | | | | |
| BI#37 | IL12Rb2#1 | CD4#8 | | | | |
| BI#39 | CD4#8 | IL12Rb2#1 | | | | |
| IL12Rb2#2 | | | | | | |
| BI#36 | IL12Rb2#2 | CD4#8 | - | | - | |
| BI#38 | CD4#8 | IL12Rb2#2 | - | | - | |

### Example 4: EGFR-CEA bispecific polypeptides

### Example 4.1: Characteristics of monovalent Nanobodies used for formatting

Previous examples indicated that the cell-specific avidity of bispecific polypeptides can be measured by potency increase in functional assays, where bispecific polypeptides will block receptor function specifically on cells when they can simultaneously engage both targets *in cis.* To demonstrate the therapeutic window, functional cellular assays were done on cells that co-express the two targets ("double-positive cells"), and cells that only express the functional target ("single-positive cells") representing normal cells.

Our previous examples also indicated that for the cell-specific blockade monovalent functional Nanobodies are needed with low affinities and potencies, to ensure that monospecific Nanobodies are not sufficiently potent on normal cells. To obtain selectivity very low affinities were needed, where the bispecific merely resembles the anchor, indicating there is a delicate trade-off between selectivity and sufficient functional potency. In the current example we further addressed the effect of affinity for Nanobodies on the functional arm, to determine if there is a threshold affinity for selective blockade. The tyrosine kinase receptor EGFR is used as model antigen on the functional arm, for which recombinant protein is available to allow the precise determination of the affinities and kinetic parameters by SPR.

The second target, carcinoembryonic antigen (CEA, also known as CEACAM5), is a well-known tumour specific antigen expressed on many tumour types. CEA is a glycosylphosphatidylinisotol (GPI)-anchored cell surface glycoprotein that plays a role in cellular adhesion. It is an established tumour-associated marker for gastrointestinal tract cancers and also found in breast and lung cancers. Co-expression of EGFR and CEA has been reported for gastric and colorectal cancers, in primary tumours and in peritoneal metastasis, with in most cases higher membrane expression of CEA than EGFR (Ito *et al.* 2013, Tiernan *et al.* 2013). This makes CEA a useful target to serve as anchor for combining with EGFR for functional blockade in a tumour-selective manner.

Ligand-blocking Nanobodies against EGFR were previously generated in-house and well described by Roovers *et al.* (2011). Nanobody 7D12 binds to the ligand binding site on domain III of the extracellular domain of EGFR, overlapping with the epitope of cetuximab. The reported affinity of [¹²⁵I] radiolabelled 7D12 was 10.4 and 25.7 nM for HER14 and A431 cells, respectively. Its family member 7C12 differs in 5 amino acid residues.

To assess the effect of affinity while ensuring that the epitope on EGFR was preserved, a panel of EGFR 7D12 and 7C12 variants with reduced affinities was generated for use in formatting. Based on the co-crystal structure of Nanobody 7D12 with the EGFR ectodomain (Schmitz et al., 2013), amino acids in the receptor interface in 7D12 were substituted with residues that were expected to reduce the off-rates in a step-wise manner (Table 4.1).

On the anchoring arm, a CEACAM5-specific Nanobody designated NbCEA5 was used with a reported high affinity of K_{D} 0.3 nM by Cortez-Ramiras et al. (2004). A variant of this Nanobody has been described with a 30-fold reduction in its affinity due to introduction of the CDR regions into a human scaffold (Vaneycken et al., 2011). Both Nanobodies as well as additional CEA variants were generated with a number of amino acid substitutions, to reduce the affinity but safe-guard a sufficiently high Nanobody expression (Table 4.2).

The panel of monovalent EGFR 7D12 variants and NbCEA5 variants with decreased affinities was characterised with respect to binding kinetics, and binding to cell-expressed receptors.

### Example 4.1.1 SPR

To determine the precise binding affinities of the purified EGFR variants, a multi-cycle kinetic analysis was performed using Surface Plasmon Resonance analysis (Biacore T100) on directly immobilized hEGFR extracellular domain (Sino Biological, #10001-H08H). Around 1000RU of hEGFR was immobilized on a CM5 sensor chip. Running buffer used was HBS-EP+ (GE Healthcare, BR-1006-69) at 25 °C, with a flow-rate of 5µl/min. For immobilization by amine coupling, EDC/NHS was used for activation and ethanolamine HCl for deactivation (Biacore, amine coupling kit). Nanobodies were evaluated at a concentration range between 1.37 nM and 3 µM. Nanobodies were allowed to associate for 2 min and to dissociate for 15 min at a flow rate of 45 µl/min. In between injections, the surfaces were regenerated with a 5 sec pulse of 50mM NaOH and 1 min stabilization period. Evaluation of the association/dissociation data was performed by fitting a 1:1 interaction model (Langmuir binding model) by Biacore T100 software v2.0.3. Interactions which did not meet the acceptance criteria for the 1:1 interaction model, were fitted using the heterogeneous ligand fit model. The affinity constant K_{D} was calculated from resulting association and dissociation rate constants kₐ and k_{d}, and are shown in Table 4.1. The introduction of defined amino acid substitutions clearly reduced the off-rate of the EGFR Nanobody, while on-rates were similar.

The binding affinities of the purified CEA Nanobodies were obtained using similar experimental conditions on directly immobilized hCEACAM-5 (R&D Systems, # 4128-CM) up to 1000RU on a CM5 sensor chip. In between injections, the surfaces were regenerated with a 5 sec pulse of 10mM Glycine-HCl pH1.5 and 1 min stabilization period. Evaluation of the association/dissociation data was performed by fitting a 1:1 interaction model (Langmuir binding model) by Biacore T100 software v2.0.3. The affinity constant K_{D} was calculated from resulting association and dissociation rate constants kₐ and k_{d} and are shown in Table 4.2. The observed affinity of the NbCEA5 Nanobody (designated as CEA#1) and humanised variant (CEA#2) was in line with the reported value.

### Example 4.1.2 Binding to recombinant EGFR and CEACAM5 proteins in ELISA

All purified Nanobodies were shown to bind to the recombinant EGFR ectodomain and to recombinant CEACAM5 protein in a dose dependent manner in binding ELISA. In short, 0.25 µg/ml of human EGFR ECD (Sino Biological, Cat#10001-H08H) or 0.125 µg/ml recombinant human CEACAM5 (R&D Systems, Cat #4128-CM) were coated directly on 384-well SpectraPlate-HB microtiter plates (Perkin Elmer). Free binding sites were blocked with 1% casein in PBS. Serial dilutions of purified Nanobodies were allowed to bind the antigen for 1 hour. Nanobody binding was detected using HRP conjugated mouse-anti-FLAG M2 antibody (Sigma, Cat#A8592) and a subsequent enzymatic reaction in the presence of the substrate esTMB (SDT reagents, Cat#esTMB). Binding specificity was determined based on OD values compared to irrelevant Nanobody controls. The EC50 values are shown in Tables 4.1 and 4.2.

### Example 4.1.3 FACS binding

The colon carcinoma cell lines LoVo and HT-29 co-express EGFR and CEA with different relative expression levels (Figure 4.1). Since LoVo cells had higher CEA levels compared to HT-29, LoVo cells were used for binding analysis of the panel of monovalent EGFR and CEA variants to cell-expressed receptors. Binding to cell-expressed EGFR and CEA was confirmed by flow cytometry on EGFR+/CEA+ LoVo cells, and to HER14 cells, murine NIH-3T3 cells stably expressing human EGFR. Bound Nanobody was detected via a flag-tag-specific antibody, as described. Results are shown in Figure 4.1. For the EGFR variants saturation was not reached, hence no accurate EC50 could be determined, but the differences in off-rates were visible in shifted curves. Specificity of CEA Nanobodies was confirmed by lack of binding to HER14 cells (data not shown).

The binding characteristics of the monovalent EGFR 7D12 variants and CEA variants are presented in Tables 4.1 and 4.2, respectively. For the generation of EGFR-CEA bispecific Nanobodies, four EGFR variants were selected with differences in off-rates resulting in gradual decreased K_{D} values (ranging between 120-860 nM). The gap in off-rate between the highest and lowest affinity EGFR variant was 8-fold. When measured in ELISA, the difference was enlarged to ^{∼}80-fold, due to dissociation of the Nanobodies with the fast off-rates during the washing. Compared to the highest affinity variant EGFR#1, variant EGFR#11 has two amino acid substitutions, whereas EGFR#33 and EGFR#32 have three amino acid differences. For the anchoring arm, besides the original CEA Nanobody (CEA#1), also CEA variant#5 was selected for use in formatting, with four amino acid substitutions, as this Nanobody had the largest difference in off-rate compared to the original Nanobody.

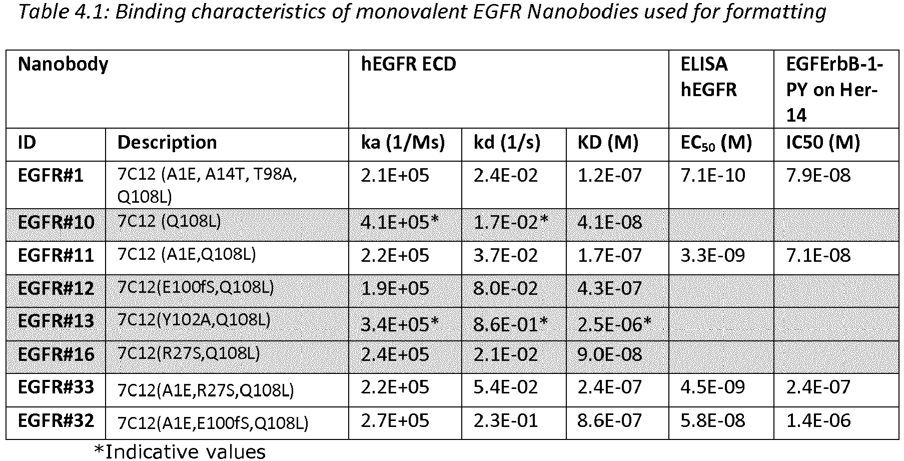

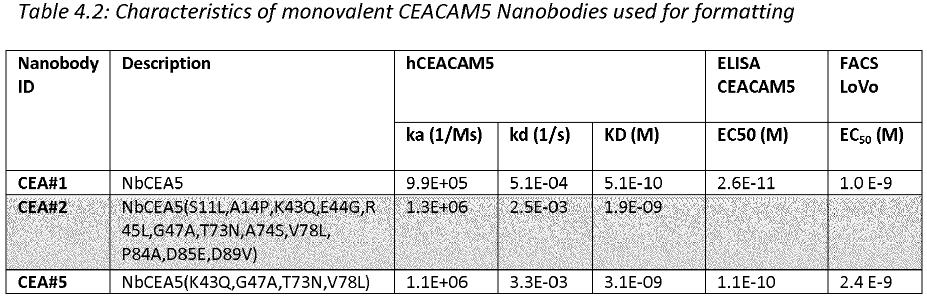

### Example 4.2: Generation of bispecific polypeptides

Formatting of bispecific EGFR-CEA polypeptides was accomplished by genetic fusion of Nanobodies linked with a flexible 35GS linker, with both building blocks in both orientations. Four different EGFR variants with distinct off-rates were combined with two distinct CEA Nanobodies with K_{D} values of 0.5 and 3 nM, respectively (Figure 4.2). In addition, each Nanobody was constructed with an irrelevant control Nanobody (cAblys3, directed to lysozyme), to preserve the valency in the monospecific reference molecules. The correct nucleotide sequence of all constructs was confirmed by sequence analysis (see Table 11 for an overview of all sequences). All polypeptides were generated as flag₃-His₆-tagged proteins in the yeast *Pichia pastoris.* Purification was done using standard affinity chromatography.

### Example 4.3: Binding analysis of bispecific EGFR-CEA Nanobodies

### Example 4.3.1 Effect of formatting

To verify if the formatting affected the ability of each of the building blocks to bind their respective target, binding of the purified bispecific Nanobodies was assessed by means of binding ELISA on recombinant EGFR ectodomain or CEACAM5, as described above. The EC50 values of all monospecific Nanobodies and bispecific polypeptides comprising EGFR-CEA are shown in Table 4.3.

For all EGFR-CEA bispecifics, the CEA Nanobody retained similar binding as the respective monovalent Nanobody. In contrast, the EGFR Nanobodies were sensitive to the position within the bispecific construct, and only in the N-terminal position the interaction with EGFR is preserved (Figure 4.3). For these constructs the measured apparent affinities are following the affinity differences observed for the monovalent Nanobodies. When EGFR was positioned C-terminal from the CEA Nanobody, a ^{∼}30 fold lower binding affinity was measured. Similar sensitivity to orientation was previously reported for wild-type 7D12 formatted with a distinct EGFR Nanobody directed against a different epitope (Roovers *et al.* 2011).

### Example 4.3.2 Binding specificity

Binding specificities of the monospecific and bispecific EGFR-CEA Nanobody constructs were analysed by flow cytometry on EGFR+/CEA- HER14 and HeLa cells, and double-positive LoVo and HT-29 cells, respectively. EC50 values are presented in Table 4.3. Results for LoVo cells are shown in Figure 4.4.

Bispecific polypeptides efficiently bound to cells in a dose-dependent manner. In line with the ELISA data, the bispecific polypeptides with the EGFR#1 Nanobody in C-terminal position lost substantial binding affinity on both HER14 and LoVo cells. When comparing the monospecific EGFR Nanobodies, the differences in off-rates between the distinct EGFR variants are less pronounced on cell-expressed EGFR, especially when EGFR expression levels are not so high, such as on LoVo and HeLa cells (Figure 4.5). On these cells the constructs of the 3 EGFR variants with the highest affinities all had very similar EC50 values between 2.5-5.5 nM (Table 4.3).

On LoVo cells, bispecific polypeptides in the EGFR-CEA orientation showed increased fluorescence levels and a slight shift in EC50 values compared to the respective EGFR control Nanobodies (Figure 4.4 panels A, B, C), except for the bispecifics of EGFR#32 with the lowest affinity for EGFR. Here the bispecific constructs showed virtually identical binding to the respective anchor CEA#1 or CEA#5 control Nanobodies, indicating that there is no contribution on the EGFR arm (Fig 4.4 panel D). This confirms earlier results obtained with CXCR4-CD4 and CXCR4-IL3Ra bispecific polypeptides, where the increase in fluorescence signal is only observed when there is sufficient binding to each of the targets.

**Table 4.3: Binding analysis of monospecific and bispecific EGFR-CEA bispecific Nanobodies.**

| **ID** | **Description** | **EGFR ELISA** | **CEA ELISA** | **Her-14 EGFR++/CEA-** | **HeLa EGFR+/CEA-** | **LoVo EGFR+/CEA+** |
|---|---|---|---|---|---|---|
| | | **EC50 (M)** | **EC50 (M)** | **EC50 (M)** | **EC50 (M)** | **EC50 (M)** |
| **BI#52** | **EGFR#1-ctrl** | 1.30E-09 | - | 8.6E-09 | 3.9E-09 | 4.3E-09 |
| **BI#26** | **EGFR#1-CEA#1** | 6.70E-10 | 4.90E-11 | 7.5E-09 | 1.9E-09 | 1.5E-09 |
| **BI#27** | **EGFR#1-CEA#5** | 3.50E-10 | 1.20E-10 | 2.0E-09 | 2.1E-09 | 3.4E-09 |
| **BI#28** | **CEA#1-EGFR#1** | 2.30E-08 | 5.60E-11 | 3.4E-08 | 2.4E-07 | 1.1E-09 |
| **BI#29** | **CEA#5-EGFR#1** | 1.30E-08 | 1.00E-10 | 3.6E-08 | 1.3E-07 | 3.0E-09 |
| **BI#49** | **EGFR#11-ctrl** | 1.70E-09 | - | 9.1E-09 | 2.9E-09 | 5.0E-09 |
| **BI#22** | **EGFR#11-CEA#1** | 9.35E-10 | 4.70E-11 | 7.1E-09 | 2.5E-09 | 1.5E-09 |
| **BI#24** | **EGFR#11-CEA#5** | 8.30E-10 | 1.40E-10 | 4.3E-09 | 3.6E-09 | 3.8E-09 |
| **BI#53** | **EGFR#33-ctrl** | 4.00E-09 | - | 1.7E-08 | 3.5E-09 | 4.5E-09 |
| **BI#34** | **EGFR#33-CEA#1** | 1.30E-09 | 5.30E-11 | 1.1E-08 | 2.7E-08 | 2.1E-09 |
| BI#35 | EGFR#33-CEA#5 | 6.10E-10 | 8.90E-11 | 3.6E-09 | 4.0E-09 | 3.7E-09 |
| **BI#50** | **EGFR#32-ctrl** | 1.60E-08 | - | 2.0E-08 | 5.7E-08 | 5.6E-08 |
| **BI#23** | **EGFR#32-CEA#1** | 1.10E-08 | 4.60E-11 | 1.4E-08 | 7.8E-08 | 1.8E-09 |
| **BI#25** | **EGFR#32-CEA#5** | 6.00E-09 | 1.20E-10 | 1.5E-08 | 1.1E-07 | 4.9E-09 |
| **BI#48** | **CEA#1-ctrl** | - | 2.60E-11 | - | - | 9.2E-10 |
| **BI#51** | **CEA#5-ctrl** | - | 1.10E-10 | - | - | 2.6E-09 |

### Example 4.4: Inhibition of EGFR function by bispecific EGFR-CEA polypeptides

To verify if bispecific polypeptides could enhance the potency of the EGFR Nanobodies by simultaneously engagement of EGFR and CEA on the cell surface, the panel of bispecific EGFR-CEA polypeptides and corresponding monospecific Nanobodies was analysed in a functional EGFR assay.

Dose-dependent inhibition of EGFR phosphorylation was assessed on HER14 cells expressing only EGFR, and EGFR+/CEA+ LoVo cells. Since the functional phosphorylation is only mediated via EGFR, avidity by the simultaneous binding of the CEA Nanobody is expected to translate into increased inhibition of EGFR phosphorylation in a cell-specific manner.

Briefly, LoVo cells were seeded in duplicate into 96-well culture plates at 2 × 10⁴ cells per well in F12-K medium supplemented with 10% FCS. HER14 cells were seeded in duplicate into 0.1% gelatin coated 96-well culture plates and grown in DMEM culture medium containing 10% FBS/BS for 24h. The next day, cells were serum-starved in medium supplemented with 0.1% FCS for 24 hrs and then incubated with Nanobodies followed by stimulation for 10 minutes with 0.5 nM of recombinant human EGF (R&D Systems, cat# 236-EG) for HER14 and 1nM for LoVo cells. EGF concentrations were based on the EC50 obtained in LoVo (EC50= 5.9 ng/ml) and HER14 cells (EC50=3.5 ng/ml). In each plate anti-EGFR mAb cetuximab (Erbitux Merck-Serono) and irrelevant control Nanobodies were included as reference. Monolayers were rinsed twice with ice-cold dPBS, and subsequently lysed in ice cold RIPA buffer substituted with 1 mM PMSF. EGF-dependent receptor activation in cell lysates was measured using a Phospho(Tyr1173)/Total EGFR Whole Cell Lysate Kit (Meso Scale Discovery - K15104D). Plates were loaded with 30 µl of lysate, incubated 1h at RT with shaking and processed according to the manufacturer's protocol. Plates were read on the Sector Imager 2400 (Meso Scale Discovery). The percentage of phospho-protein over total protein was calculated using the formula: (2 × p-protein)/(p-protein + total protein) × 100.

Representative graphs are shown in Figure 4.6, and average IC50 values of two and three independent assays are listed in Table 4.4. Nanobodies show dose-dependent inhibition of EGFR phosphorylation on both cells. On HER14 cells, all EGFR-CEA bispecific polypeptides showed equal inhibition of EGFR phosphorylation as the corresponding monospecific controls. The measured potency differences between the monospecific EGFR controls follow the off-rates of the monovalent EGFR building blocks, with IC50 values of 65-52-150-690 nM (HER14), and 75-150-467-2333nM (LoVo), respectively.

On EGFR+/CEA+ LoVo cells, about 5 fold difference in potency between monospecific and bispecific EGFR-CEA polypeptides was observed for constructs with EGFR#1 and EGFR#33 combined with the CEA#1 Nanobody as anchor. Constructs with the lowest affinity EGFR#32 variant could not block EGFR function, and the additional presence of CEA Nanobody could not enhance its potency.

Taken together, these results show that potency enhancements were obtained with bispecific polypeptides for the EGFR and CEACAM5 target combination, exclusively on cells that co-express both receptors. The relative small potency increase of EGFR-CEA bispecific polypeptides observed on LoVo cells in the phosphorylation assay may be related to a suboptimal ratio between CEA and EGFR expression on this cells, but it is also possible that the potency effects will be larger in assays that measure functional responses of EGF, such as proliferation and survival. Besides the effect on receptor phosphorylation at one timepoint, as assessed in the current assay, the Nanobody could have differential effects on the receptor inactivation and degradation kinetics, which are not be assessed in a signal transduction assay. It is also possible that the selected Nanobodies for this example had sterical limitations with respect to the epitope on the target, which may restrict simultaneous engagement of both targets on the cell surface.

A gain in potency was observed for the current combination tested but bispecific EGFR-CEA polypeptides directed towards other epitopes may show larger in cell-specific potency enhancements

**Table 4.4: Inhibition of EGF-mediated EGFR phosphorylation by EGFR-CEA bispecific Nanobodies compared to monospecific control Nanobodies.**

| | | **HER-14 (n=2) EGFR+/CEA-** | | **LoVo (n=2-3) EGFR+/CEA+** | |
|---|---|---|---|---|---|
| **ID** | **Description** | **IC50 (M)** | **fold increase*** | **IC50 (M)** | **fold increase** |
| BI#52 | EGFR#1-ctrl | 6.55E-08 | | 7.53E-08 | |
| BI#26 | EGFR#1-CEA#1 | 5.80E-08 | 1.1 | 1.50E-08 | 5.0 |
| BI#27 | EGFR#1-CEA#5 | 3.65E-08 | 1.8 | 2.50E-08 | 3.0 |
| BI#28 | CEA#1-EGFR#1 | 1.75E-06 | | 4.83E-06 | |
| BI#29 | CEA#5- EGFR#1 | 1.95E-06 | | 3.10E-06 | |
| BI#49 | EGFR#11-ctrl | 5.25E-08 | | 1.50E-07 | |
| BI#22 | EGFR#11-CEA#1 | 6.25E-08 | 0.8 | 4.30E-08 | 3.5 |
| BI#24 | EGFR#11-CEA#5 | 5.00E-08 | 1.1 | 4.15E-08 | 3.6 |
| BI#53 | EGFR#33- ctrl | 1.50E-07 | | 4.63E-07 | |
| BI#34 | EGFR#33-CEA#1 | 6.05E-08 | 2.5 | 5.98E-08 | 7.7 |
| BI#35 | EGFR#33-CEA#5 | 1.23E-07 | 1.2 | 8.90E-08 | 5.2 |
| BI#50 | EGFR#32-ctrl | 6.90E-07 | | 2.85E-06 | |
| BI#23 | EGFR#32-CEA#1 | 1.50E-06 | 0.7 | 8.85E-07 | 3.2 |
| BI#25 | EGFR#32-CEA#5 | 8.10E-07 | 1.3 | 8.08E-07 | 3.5 |
| BI#48 | CEA#1-ctrl | - | | - | |
| | erbitux | 1.25E-09 | | 4.4E-10 | |

| | | | | | |
|---|---|---|---|---|---|
| *IC50 ratio relative to respective monospecific EGFR Nanobodies on same cell line. | | | | | |

**Table 1: CXCR4 building blocks**

| | | |
|---|---|---|
| 14D09 | | EVQLVESGGGLVQAGGSLRLSCVASGISSSKRNMGWYRQAPGKQRESVATISSGGN KDYTDAVKDRFTISRDTTKNTVYLQMNSLKPEDTAVYYCKIEAGTGWATRRGYTYW GQGTQVTVSS |
| 14A09 | | EVQLVESGGGLVQAGGSLRLSCVASGISSSIRNSGWYRQAPGKQRESVATISSGGN KDYTDAVKDRFTISRDTTKNTVYLQMNSLKPEDTAVYYCKIEAGTGWATRRGYTYW GQGTQVTVSS |
| 281F12 (Q108L) | | EVQLVESGGGLVQAGDSLRLSCAASGRAFSRYAMGWFRQAPGKEREFVAAIGWGPS KTNYADSVKGRFTISRDNAKNTVYLQMNTLKPEDTAVYSCAAKFVNTDSTWSRSEM YTYWGQGTLVTVSS |
| 14A02 | | EVQLVESGGGLVQAGGSLRLSCVASGISSSIRNMGWYRQAPGKQRESVATISSGGN KDYTDAVKDRFTISRDTTKNTVYLQMSSLKPEDTAVYYCKIEAGTGWATRRGYTYW GQGTQVTVSS |
| 14E02 | | EVQLVESGGGLVQAGGSLRLSCVASGISSSIRNMGWYRQAPGKQRESVATISSGGN KDYTDAVKDRFTISRDTTKNTVYLQMNSLKPEDTAVYYCKIEAGTGWATRRGYTYW GQGTQVTVSS |
| 14D09 (Q108L) | | EVQLVESGGGLVQAGGSLRLSCVASGISSSKRNMGWYRQAPGKQRESVATISSGGN KDYTDAVKDRFTISRDTTKNTVYLQMNSLKPEDTAVYYCKIEAGTGWATRRGYTYW GQGTLVTVSS |
| 281F12 (TAG) | 4CXCR281F1 2-FLAG3-HIS6 | EVQLVESGGGLVQAGDSLRLSCAASGRAFSRYAMGWFRQAPGKEREFVAAIGWGPS KTNYADSVKGRFTISRDNAKNTVYLQMNTLKPEDTAVYSCAAKFVNTDSTWSRSEM YTYWGQGTLVTVSSAAADYKDHDGDYKDHDIDYKDDDDKGAAHHHHHH |
| 14D09 (TAG) | 4CXCR014D0 9-FLAG3-HIS6 | EVQLVESGGGLVQAGGSLRLSCVASGISSSKRNMGWYRQAPGKQRESVATISSGGN KDYTDAVKDRFTISRDTTKNTVYLQMNSLKPEDTAVYYCKIEAGTGWATRRGYTYW GQGTLVTVSSAAADYKDHDGDYKDHDIDYKDDDDKGAAHHHHHH |

**Table 2: CD123 building blocks**

| | |
|---|---|
| 55B04 | EVQLVESGGGLVQPGGSLRLSCAASGINFRFNSMGWWRRRAPGKEREWVAAITSGD ITNYRDSVRGRFTISRDNVKNTVYLQMNTLKLEDTAVYYCNTFPPIADYWGLGTQV TVSS |
| 51D09 | EVQLVESGGGLVQPGGSLRLSCAASGSIFSGNTMGWYRQAPGKQRELVAAISSGGS TDYADSVKGRFTISRDNSKNTVYLQMNSLRPEDTAVYYCNAAILLYRLYGYEEGDY WGLGTLVTVSS |
| 55C05 | EVQLVESGGGLVPAGDSLRLSCVASGRSLNTYTMGWFRQAPGKECEFVAAINWNGV YRDYADSAKGRFTASRDNAMNTVFLQMNSLKPEDTAVYFCATATQGWDRHTEPSDF GSWGLGTQVTVSS |
| 50F07 | EVQLVESGGGLVQPGGSLRLSCTGSGSTFSINAMGWYRQAPGKQRELVAAITSGGR TNYADSVKGRFTISRDNSKNTVYLQMNSLRPEDTAVYYCNARISAGTAFWLWSDYE YWGLGTLVTVSS |
| 55F03 | EVQLVESGGGLVQAGGPLRLSCAASGRTFSSYVMGWFRQAPGKEREFVAAIYWSNG KTQYTDSVKGRFTISGDNAKNTVYLQMNSLNPEDTAVYYCVADKDETGFRTLPIAY DYWGLGTQVTVSS |
| 55A01 | EVQLVESGGGSVQAGGSLRLSCTTSGRALNMYVMGWFRQAPGNEREFVAATSSSGG STSYPDSVKGRFTISRDNAKNTVYLQMNSLKPEDTAAYRCAASPYVSTPTMNILEE YRYWGLGTQVTVSS |
| 57A07 | EVQLVESGGGLVQAGGSLRLSCAASGSIFSGNVMGWYRRQAPGKEREWVAAIASGG SIYYRDSVKGRFTISRDNAKNTVYLQMNSLKPEDTAVYYCNSHPPTLPYWGLGTQV TVSS |

**Table 3: Characteristics of monovalent IL-3Ra Nanobodies**

| Nanobody | | | SPR-IL-3Ra | | | FACS binding | | | mAb 7G3 competition |
|---|---|---|---|---|---|---|---|---|---|
| | ID | Germ-line | ka (1/Ms) | kd (1/s) | KD [M] | MOLM-13 EC50 (M) | THP-1 EC50 (M) | Hek-IL-R3a EC50 (M) | IC50 (M) |
| CD123#1 | 57A07 | VHH2 | 1.0E+06 | 8.1E-04 | 7.83E-10 | 6.6E-10 | 1.3E-9 | 2.4E-10 | 1.20E-09 |
| CD123#2 | 55A01 | VHH3 | 8.4E+04 | 1.4E-03 | 1.71E-08 | 8.2E-9 | 1.1E-8 | 1.10E-09 | 4.00E-08 |
| | 55B04 | VHH2 | 5.04E+05 | 7.94E-03 | 1.58E-08 | 5.12E-08 | | | 5.50E-08 |
| | 51D09 | VHH2 | 3.78E+04 | 5.16E-04 | 1.36E-08 | 1.53E-08 | | | |
| | 55C05 | VHH3 | 1.26E+05 | 7.41E-03 | 5.90E-08 | 3.12E-08 | | | 1.90E-07 |
| | 50F07 | VHH2 | 1.02E+05 | 7.58E-03 | 7.42E-08 | 1.46E-08 | | | 2.30E-07 |
| | 55F03 | VHH3 | 4.25E+04 | 4.87E-03 | 1.15E-07 | 1.13E-07 | | | |

**Table 4: Characteristics of monovalent CXCR4 Nanobodies**

| **Nanobody** | | | **Ligand competition** | | | **CXCR4 Binding** | | |
|---|---|---|---|---|---|---|---|---|
| **Description** | **ID** | **Fam** | **Biotin-SDF-1 IC50 (nM)** | **[¹²⁵I]-SDF-1 # Ki (nM)** | **Chemotaxis Jurkat IC50 (nM)** | **Caki-CXCR4 EC50 (nM)** | **Jurkat EC50 (nM)** | **CXCR4-VLP EC50 (nM)** |
| CXCR4#2 | **281F12** | 3 | | 26.9 | 68 | nd | 7.8 | nd |
| CXCR4#1 | **14D09** | 57 | 18.4 | | 11 | 9.9 | 11 | 7.28 |
| | **14A02** | 57 | 4.1 | | 0.95 | 4.0 | 1.15 | 0.73 |
| | **14E02** | 57 | 13.5 | | | 2.6 | nd | 0.78 |
| | **238D4** | 17 | 2.1 | 5.4 | 7.6 | 3.4 | nd | nd |

| | | | | | | | | |
|---|---|---|---|---|---|---|---|---|
| # Determined with [¹²⁵I]-SDF-1 on membrane extracts of Hek-CXCR4 cells. | | | | | | | | |

**Table 5: Summary of selected Nanobodies**

| **Nanobody function** | **Target** | **High affinity:** | **Medium affinity:** | **Medium/low potency Ligand inhibition IC50 >=10 nM** |
|---|---|---|---|---|
| | | **K_{D} <=1 nM** | **EC50/kD 1< x <=10 nM** | |
| | | **EC₅₀ <=1 nM** | **Ligand inhibition IC50 1<x <10 nM** | |
| Functional | CXCR4 | | CXCR4#1: 14D09 | CXCR4#2: 281F12 |
| Anchor | CD123 | CD123#1: 57A07 | CD123#2: 55A01 | |
| Anchor | CD4 | CD4#8: 3F11 | | |
| Functional | IL12Rβ1 | | IL12Rβ1#30: 148C09 | IL12Rβ1#31: 148F09 |
| Functional | IL12Rβ2 | | IL12Rβ2#1: 135B08 | IL12Rβ2#2: 135A07 |
| Functional | IL-23R | | IL23R#19: 150D02 | IL23R#20: 150H07 |
| Functional | EGFR | | | EGFR#1/11/33/32: 7D12 variants |
| Anchor | CEACAM5 | CEA#1: NbCEA5 | CEA#5: NbCEA5 variant | |
| | | | | |

| **Table 9Nanobody function** | **Target** | **High affinity:** | **Medium affinity:** | |
|---|---|---|---|---|
| | | **K_{D} <=1 nM** | **EC50 1< x <=10 nM** | |
| | | **EC₅₀ <=1 nM** | | |
| Anchor | CD123 | CD123#1: 57A07 | CD123#2: 55A01 | |
| Anchor | CD4 | CD4#8: 3F11 | | |
| Anchor | CEACAM5 | CEA#1: NbCEA5 | CEA#5: NbCEA5 variant | |

**Table 6: Summary of bispecific constructs**

| | |
|---|---|
| 57A07 - 14D09 | 55A01 - 14D09 |
| 57A07 - 281F12 | 55A01 - 281F12 |
| 14D09 - 57A07 | 14D09 - 55A01 |
| 281F12 - 57A07 | 281F12 - 55A01 |

**Table 7: bispecific constructs (all with c-myc HIS6 tag)**

| | | |
|---|---|---|
| 57A07 - 14D09 | A0110057A0 7-35GS-4CXCR014D0 9(Q108L) | EVQLVESGGGLVQAGGSLRLSCAASGSIFSGNVMGWYRRQAPGKEREWVAAIASGG SIYYRDSVKGRFTISRDNAKNTVYLQMNSLKPEDTAVYYCNSHPPTLPYWGQGTLV TVSSGGGGSGGGGSGGGGSGGGGSGGGGSGGGGSGGGGSEVQLVESGGGLVQAGGS LRLSCVASGISSSKRNMGWYRQAPGKQRESVATISSGGNKDYTDAVKDRFTISRDT TKNTVYLQMNSLKPEDTAVYYCKIEAGTGWATRRGYTYWGQGTLVTVSSAAAEQKL ISEEDLNGAAHHHHHH |
| 57A07 - 281F12 | A0110057A0 7-35GS-4CXCR281F1 2(Q108L) | EVQLVESGGGLVQAGGSLRLSCAASGSIFSGNVMGWYRRQAPGKEREWVAAIASGG SIYYRDSVKGRFTISRDNAKNTVYLQMNSLKPEDTAVYYCNSHPPTLPYWGQGTLV TVSSGGGGSGGGGSGGGGSGGGGSGGGGSGGGGSGGGGSEVQLVESGGGLVQAGDS LRLSCAASGRAFSRYAMGWFRQAPGKEREFVAAIGWGPSKTNYADSVKGRFTISRD NAKNTVYLQMNTLKPEDTAVYSCAAKFVNTDSTWSRSEMYTYWGQGTLVTVSSAAA EQKLISEEDLNGAAHHHHHH |
| 14D09 - 57A07 | 4CXCR014D0 9(Q108L)-35GS-A0110057A0 7- | EVQLVESGGGLVQAGGSLRLSCVASGISSSKRNMGWYRQAPGKQRESVATISSGGN KDYTDAVKDRFTISRDTTKNTVYLQMNSLKPEDTAVYYCKIEAGTGWATRRGYTYW GQGTLVTVSSGGGGSGGGGSGGGGSGGGGSGGGGSGGGGSGGGGSEVQLVESGGGL VQAGGSLRLSCAASGSIFSGNVMGWYRRQAPGKEREWVAAIASGGSIYYRDSVKGR FTISRDNAKNTVYLQMNSLKPEDTAVYYCNSHPPTLPYWGQGTLVTVSSAAAEQKL ISEEDLNGAAHHHHHH |
| 281F12 - 57A07 | 4CXCR281F1 2(Q108L)-35GS-A0110057A07- | EVQLVESGGGLVQAGDSLRLSCAASGRAFSRYAMGWFRQAPGKEREFVAAIGWGPS KTNYADSVKGRFTISRDNAKNTVYLQMNTLKPEDTAVYSCAAKFVNTDSTWSRSEM YTYWGQGTLVTVSSGGGGSGGGGSGGGGSGGGGSGGGGSGGGGSGGGGSEVQLVES GGGLVQAGGSLRLSCAASGSIFSGNVMGWYRRQAPGKEREWVAAIASGGSIYYRDS VKGRFTISRDNAKNTVYLQMNSLKPEDTAVYYCNSHPPTLPYWGQGTLVTVSSAAA EQKLISEEDLNGAAHHHHHH |
| | | |
| 55A01-14D09 | A0110055A0 1-35GS-4CXCR014D0 9(Q108L) | EVQLVESGGGSVQAGGSLRLSCTTSGRALNMYVMGWFRQAPGNEREFVAATSSSGG STSYPDSVKGRFTISRDNAKNTVYLQMNSLKPEDTAAYRCAASPYVSTPTMNILEE YRYWGQGTLVTVSSGGGGSGGGGSGGGGSGGGGSGGGGSGGGGSGGGGSEVQLVES GGGLVQAGGSLRLSCVASGISSSKRNMGWYRQAPGKQRESVATISSGGNKDYTDAV KDRFTISRDTTKNTVYLQMNSLKPEDTAVYYCKIEAGTGWATRRGYTYWGQGTLVT VSSAAAEQKLISEEDLNGAAHHHHHH |
| 55A01-281F12 | A0110055A01-35GS-4CXCR281F1 2(Q108L) | EVQLVESGGGSVQAGGSLRLSCTTSGRALNMYVMGWFRQAPGNEREFVAATSSSGG STSYPDSVKGRFTISRDNAKNTVYLQMNSLKPEDTAAYRCAASPYVSTPTMNILEE YRYWGQGTLVTVSSGGGGSGGGGSGGGGSGGGGSGGGGSGGGGSGGGGSEVQLVES GGGLVQAGDSLRLSCAASGRAFSRYAMGWFRQAPGKEREFVAAIGWGPSKTNYADS VKGRFTISRDNAKNTVYLQMNTLKPEDTAVYSCAAKFVNTDSTWSRSEMYTYWGQG TLVTVSSAAAEQKLISEEDLNGAAHHHHHH |
| 14D09 - 55A01 | 4CXCR014D0 9(Q108L)-35GS-A0110055A01 | EVQLVESGGGLVQAGGSLRLSCVASGISSSKRNMGWYRQAPGKQRESVATISSGGN KDYTDAVKDRFTISRDTTKNTVYLQMNSLKPEDTAVYYCKIEAGTGWATRRGYTYW GQGTLVTVSSGGGGSGGGGSGGGGSGGGGSGGGGSGGGGSGGGGSEVQLVESGGGS VQAGGSLRLSCTTSGRALNMYVMGWFRQAPGNEREFVAATSSSGGSTSYPDSVKGR FTISRDNAKNTVYLQMNSLKPEDTAAYRCAASPYVSTPTMNILEEYRYWGQGTLVT VSSAAAEQKLISEEDLNGAAHHHHHH |
| 281F12 - 55A01 | 4CXCR281F1 2(Q108L)-35GS-A0110055A01 | EVQLVESGGGLVQAGDSLRLSCAASGRAFSRYAMGWFRQAPGKEREFVAAIGWGPS KTNYADSVKGRFTISRDNAKNTVYLQMNTLKPEDTAVYSCAAKFVNTDSTWSRSEM YTYWGQGTLVTVSSGGGGSGGGGSGGGGSGGGGSGGGGSGGGGSGGGGSEVQLVES GGGSVQAGGSLRLSCTTSGRALNMYVMGWFRQAPGNEREFVAATSSSGGSTSYPDS VKGRFTISRDNAKNTVYLQMNSLKPEDTAAYRCAASPYVSTPTMNILEEYRYWGQG TLVTVSSAAAEQKLISEEDLNGAAHHHHHH |

**Table 8: Potencies of monovalent and bispecific CXCR4-IL3Ra Nanobodies® to inhibit CXCL-12 induced chemotaxis.**

| | | | **Jurkat E6-1** | | | | **MOLM-13** | | | |
|---|---|---|---|---|---|---|---|---|---|---|
| **Abbreviation** | **N-terminal** | **C-terminal** | **IC50** | **95% LCI** | **95% UCI** | **Fold inc.** | **IC50** | **95% LCI** | **95% UCI** | **Fold inc.** |
| CXCR4#1 | 14D09 | - | 1.04E-08 | 7.08E-09 | 1.56E-08 | - | 8.62E-09 | 5.33E-09 | 1.61E-08 | - |
| CXCR4#1-CD123#1 | 14D09 | 57A07 | 1.50E-08 | 1.00E-08 | 2.35E-08 | 0.69 | 3.60E-09 | 2.50E-09 | 5.35E-09 | 2.39 |
| CXCR4#1-CD123#2 | 14D09 | 55A01 | 1.20E-08 | 8.20E-09 | 1.70E-08 | 0.87 | 3.60E-09 | 2.25E-09 | 5.70E-09 | 2.39 |
| CD123#1-CXCR4#1 | 57A07 | 014D09 | 2.10E-07 | 1.60E-07 | 2.80E-07 | 0.05 | - | - | - | - |
| CD123#2-CXCR4#1 | 55A01 | 014D09 | 8.50E-08 | 3.90E-08 | 1.90E-07 | 0.12 | 2.90E-07 | 1.70E-07 | 4.90E-07 | 0.03 |
| CXCR4#2 | 281F12 | - | 9.68E-08 | 7.08E-08 | 1.33E-07 | - | 8.60E-08 | 5.11E-08 | 1.51E-07 | - |
| CXCR4#2-CD123# 1 | 281F12 | 57A07 | 3.80E-08 | 2.40E-08 | 6.00E-08 | 2.55 | 6.83E-09 | 4.30E-09 | 1.20E-08 | 12.58 |
| CXCR4#2-I CD123#2 | 281F12 | 55A01 | 8.60E-08 | 4.50E-08 | 1.73E-07 | 1.13 | 7.85E-09 | 5.55E-09 | 1.30E-08 | 10.95 |
| CD123#1-CXCR4#2 | 57A07 | 281F12 | - | - | - | - | - | - | - | - |
| CD123#2-CXCR4#2 | 55A01 | 281F12 | - | - | - | - | - | - | - | - |

| | | | | | | | | | | |
|---|---|---|---|---|---|---|---|---|---|---|
| Legend: IC50 - average of the respective IC50 in 2-3 independent experiments LCI - Lower limit of 95% confidence interval (average from 2-3 independent experiments) UCI - Upper limit of 95% confidence interval (average from 2-3 independent experiments) Fold inc - fold increase of the bispecific construct compared to the respective anti-CXCR4 building block | | | | | | | | | | |

**Table 10 CXCR4-CD4 sequences**

| **Nanobody ID** | **Code used in text** | |
|---|---|---|
| **281F12** | **CXCR4#2** | EVQLVESGGGLVQAGDSLRLSCAASGRAFSRYAMGWFRQAP GKEREFVAAIGWGPSKTNYADSVKGRFTISRDNAKNTVYLQ MNTLKPEDTAVYSCAAKFVNTDSTWSRSEMYTYWGQGTQV TVSS |
| **03F11** | **CD4#8** | EVQLVESGGGSVQPGGSLTLSCGTSGRTFNVMGWFRQAPGK EREFVAAVRWSSTGIYYTQYADSVKSRFTISRDNAKNTVYLE MNSLKPEDTAVYYCAADTYNSNPARWDGYDFRGQGTQVTV SS |
| **03F11-9GS- 281F12** | **CD4#8-9GS-CXCR4#2** | EVQLVESGGGSVQPGGSLTLSCGTSGRTFNVMGWFRQAPGK EREFVAAVRWSSTGIYYTQYADSVKSRFTISRDNAKNTVYLE MNSLKPEDTAVYYCAADTYNSNPARWDGYDFRGQGTQVTV SSGGGGSGGGGSEVQLVESGGGLVQAGDSLRLSCAASGRAF SRYAMGWFRQAPGKEREFVAAIGWGPSKTNYADSVKGRFTI SRDNAKNTVYLQMNTLKPEDTAVYSCAAKFVNTDSTWSRSE MYTYWGQGTQVTVSS |
| **03F11-25GS- 281F12** | **CD4#8-25GS-CXCR4#2** | EVQLVESGGGSVQPGGSLTLSCGTSGRTFNVMGWFRQAPGK EREFVAAVRWSSTGIYYTQYADSVKSRFTISRDNAKNTVYLE MNSLKPEDTAVYYCAADTYNSNPARWDGYDFRGQGTQVTV SSGGGGSGGGGSGGGGSGGGGSGGGGSEVQLVESGGGLVQ AGDSLRLSCAASGRAFSRYAMGWFRQAPGKEREFVAAIGW GPSKTNYADSVKGRFTISRDNAKNTVYLQMNTLKPEDTAVYS CAAKFVNTDSTWSRSEMYTYWGQGTQVTVSS |
| **03F11-35GS- 281F12** | **CD4#8-35GS-CXCR4#2** | EVQLVESGGGSVQPGGSLTLSCGTSGRTFNVMGWFRQAPGK EREFVAAVRWSSTGIYYTQYADSVKSRFTISRDNAKNTVYLE MNSLKPEDTAVYYCAADTYNSNPARWDGYDFRGQGTQVTV SSGGGGSGGGGSGGGGSGGGGSGGGGSGGGGSGGGGSEV QLVESGGGLVQAGDSLRLSCAASGRAFSRYAMGWFRQAPG KEREFVAAIGWGPSKTNYADSVKGRFTISRDNAKNTVYLQM NTLKPEDTAVYSCAAKFVNTDSTWSRSEMYTYWGQGTQVT VSS |
| **281F12-9GS- 03F11** | **CXCR4#2-9GS-CD4#8** | EVQLVESGGGLVQAGDSLRLSCAASGRAFSRYAMGWFRQAP GKEREFVAAIGWGPSKTNYADSVKGRFTISRDNAKNTVYLQ MNTLKPEDTAVYSCAAKFVNTDSTWSRSEMYTYWGQGTQV TVSSGGGSGGGGSEVQLVESGGGSVQPGGSLTLSCGTSGRTF NVMGWFRQAPGKEREFVAAVRWSSTGIYYTQYADSVKSRFT ISRDNAKNTVYLEMNSLKPEDTAVYYCAADTYNSNPARWDG YDFRGQGTQVTVSS |
| **281F12-25GS- 03F11** | **CXCR4#2-25GS-CD4#8** | EVQLVESGGGLVQAGDSLRLSCAASGRAFSRYAMGWFRQAP GKEREFVAAIGWGPSKTNYADSVKGRFTISRDNAKNTVYLQ MNTLKPEDTAVYSCAAKFVNTDSTWSRSEMYTYWGQGTQV TVSSGGGGSGGGGSGGGGSGGGGSGGGGSEVQLVESGGGS VQPGGSLTLSCGTSGRTFNVMGWFRQAPGKEREFVAAVRW SSTGIYYTQYADSVKSRFTISRDNAKNTVYLEMNSLKPEDTAV YYCAADTYNSNPARWDGYDFRGQGTQVTVSS |
| **281F12-35GS- 03F11** | **CXCR4#2-35GS-CD4#8** | EVQLVESGGGLVQAGDSLRLSCAASGRAFSRYAMGWFRQAP GKEREFVAAIGWGPSKTNYADSVKGRFTISRDNAKNTVYLQ MNTLKPEDTAVYSCAAKFVNTDSTWSRSEMYTYWGQGTQV TVSSGGGGSGGGGSGGGGSGGGGSGGGGSGGGGSGGGGS EVQLVESGGGSVQPGGSLTLSCGTSGRTFNVMGWFRQAPGK EREFVAAVRWSSTGIYYTQYADSVKSRFTISRDNAKNTVYLE MNSLKPEDTAVYYCAADTYNSNPARWDGYDFRGQGTQVTV SS |
| | | |
| **A011000025** | **4CXCR281F12(L108Q)-35GS- 4CD003F11(L108Q)-FLAG3-HIS6** | |
| **A011000026** | **4CD003F11(L108Q)-35GS- 4CXCR281F12(L108Q)-FLAG3-HIS6** | |

**Table 11 EGFR-CEA sequences**

| **Nb ID** | **Code used:** | **sequence** | | | |
|---|---|---|---|---|---|
| **NbCEA5** | **CEA#1** | EVQLVESGGGSVQAGGSLRLSCAASGDTYGSYWMGWFRQAPGKEREG VAAINRGGGYTVYADSVKGRFTISRDTAKNTVYLQMNSLRPDDTADYYC AASGVLGGLHEDWFNYWGQGTLVTVSS | | | |
| **T023200002** | **CEA#2** | EVQLVESGGGLVQPGGSLRLSCAASGDTYGSYWMGWFRQAPGQGLEA VAAINRGGGYTVYADSVKGRFTISRDNSKNTLYLQMNSLRAEDTAVYYC AASGVLGGLHEDWFNYWGQGTLVTVSS | | | |
| **T023200003** | **CEA#3** | EVQLVESGGGSVQAGGSLRLSCAASGDTYGSYWMGWFRQAPGKEREG VAAINRGGGYTVYADSVKGRFTISRDNAKNTLYLQMNSLRPDDTADYYC AASGVLGGLHEDWFNYWGQGTLVTVSS | | | |
| **T023200004** | **CEA#4** | EVQLVESGGGSVQAGGSLRLSCAASGDTYGSYWMGWFRQAPGQERE GVAAINRGGGYTVYADSVKGRFTISRDNAKNTLYLQMNSLRPDDTADY YCAASGVLGGLHEDWFNYWGQGTLVTVSS | | | |
| **T023200005** | **CEA#5** | EVQLVESGGGSVQAGGSLRLSCAASGDTYGSYWMGWFRQAPGQERE AVAAINRGGGYTVYADSVKGRFTISRDNAKNTLYLQMNSLRPDDTADY YCAASGVLGGLHEDWFNYWGQGTLVTVSS | | | |
| **T023200006** | **CEA#6** | EVQLVESGGGSVQAGGSLRLSCAASGDTYGSYWMGWFRQAPGQELEA VAAINRGGGYTVYADSVKGRFTISRDNAKNTLYLQMNSLRPDDTADYYC AASGVLGGLHEDWFNYWGQGTLVTVSS | | | |
| **T023200007** | **CEA#7** | EVQLVESGGGSVQAGGSLRLSCAASGDTYGSYWMGWFRQAPGQGLE AVAAINRGGGYTVYADSVKGRFTISRDNAKNTLYLQMNSLRPDDTADY YCAASGVLGGLHEDWFNYWGQGTLVTVSS | | | |
| | | | | | |
| **7D12** | **EGFR#1** | EVQLVESGGGSVQTGGSLRLTCAASGRTSRSYGMGWFRQAPGKEREFV SGISWRGDSTGYADSVKGRFTISRDNAKNTVDLQMNSLKPEDTAIYYCA AAAGSAWYGTLYEYDYWGQGTLVTVSS | | | |
| **T023200010** | **EGFR#10** | AVQLVESGGGSVQAGGSLRLTCAASGRTSRSYGMGWFRQAPGKEREF VSGISWRGDSTGYADSVKGRFTISRDNAKNTVDLQMNSLKPEDTAIYYC AAAAGSTWYGTLYEYDYWGQGTLVTVSS | | | |
| **T023200011** | **EGFR#11** | EVQLVESGGGSVQAGGSLRLTCAASGRTSRSYGMGWFRQAPGKEREFV SGISWRGDSTGYADSVKGRFTISRDNAKNTVDLQMNSLKPEDTAIYYCA AAAGSTWYGTLYEYDYWGQGTLVTVSS | | | |
| **T023200012** | **EGFR#12** | AVQLVESGGGSVQAGGSLRLTCAASGRTSRSYGMGWFRQAPGKEREF VSGISWRGDSTGYADSVKGRFTISRDNAKNTVDLQMNSLKPEDTAIYYC AAAAGSTWYGTLYSYDYWGQGTLVTVSS | | | |
| **T023200013** | **EGFR#13** | AVQLVESGGGSVQAGGSLRLTCAASGRTSRSYGMGWFRQAPGKEREF VSGISWRGDSTGYADSVKGRFTISRDNAKNTVDLQMNSLKPEDTAIYYC AAAAGSTWYGTLYEYDAWGQGTLVTVSS | | | |
| **T023200032** | **EGFR#32** | EVQLVESGGGSVQAGGSLRLTCAASGRTSRSYGMGWFRQAPGKEREFV SGISWRGDSTGYADSVKGRFTISRDNAKNTVDLQMNSLKPEDTAIYYCA AAAGSTWYGTLYSYDYWGQGTLVTVSS | | | |
| **T023200033** | **EGFR#33** | EVQLVESGGGSVQAGGSLRLTCAASGSTSRSYGMGWFRQAPGKEREFV SGISWRGDSTGYADSVKGRFTISRDNAKNTVDLQMNSLKPEDTAIYYCA | | | |
| | | AAAGSTWYGTLYEYDYWGQGTLVTVSS | | | |
| | | | | | |
| **T023200022** | **EGFR#11-CEA#1** | EVQLVESGGGSVQAGGSLRLTCAASGRTSRSYGMGWFRQAPGKEREFV SGISWRGDSTGYADSVKGRFTISRDNAKNTVDLQMNSLKPEDTAIYYCA AAAGSTWYGTLYEYDYWGQGTLVTVSSGGGGSGGGGSGGGGSGGGG SGGGGSGGGGSGGGGSEVQLVESGGGSVQAGGSLRLSCAASGDTYGSY WMGWFRQAPGKEREGVAAINRGGGYTVYADSVKGRFTISRDTAKNTV YLQMNSLRPDDTADYYCAASGVLGGLHEDWFNYWGQGTLVTVSS | | | |
| **T023200023** | **EGFR#32-CEA#1** | EVQLVESGGGSVQAGGSLRLTCAASGRTSRSYGMGWFRQAPGKEREFV SGISWRGDSTGYADSVKGRFTISRDNAKNTVDLQMNSLKPEDTAIYYCA AAAGSTWYGTLYSYDYWGQGTLVTVSSGGGGSGGGGSGGGGSGGGG SGGGGSGGGGSGGGGSEVQLVESGGGSVQAGGSLRLSCAASGDTYGSY WMGWFRQAPGKEREGVAAINRGGGYTVYADSVKGRFTISRDTAKNTV YLQMNSLRPDDTADYYCAASGVLGGLHEDWFNYWGQGTLVTVSS | | | |
| **T023200024** | **EGFR#11-CEA#5** | EVQLVESGGGSVQAGGSLRLTCAASGRTSRSYGMGWFRQAPGKEREFV SGISWRGDSTGYADSVKGRFTISRDNAKNTVDLQMNSLKPEDTAIYYCA AAAGSTWYGTLYEYDYWGQGTLVTVSSGGGGSGGGGSGGGGSGGGG SGGGGSGGGGSGGGGSEVQLVESGGGSVQAGGSLRLSCAASGDTYGSY WMGWFRQAPGQEREAVAAINRGGGYTVYADSVKGRFTISRDNAKNTL YLQMNSLRPDDTADYYCAASGVLGGLHEDWFNYWGQGTLVTVSS | | | |
| **T023200025** | **EGFR#32-CEA#5** | EVQLVESGGGSVQAGGSLRLTCAASGRTSRSYGMGWFRQAPGKEREFV SGISWRGDSTGYADSVKGRFTISRDNAKNTVDLQMNSLKPEDTAIYYCA AAAGSTWYGTLYSYDYWGQGTLVTVSSGGGGSGGGGSGGGGSGGGG SGGGGSGGGGSGGGGSEVQLVESGGGSVQAGGSLRLSCAASGDTYGSY WMGWFRQAPGQEREAVAAINRGGGYTVYADSVKGRFTISRDNAKNTL YLQMNSLRPDDTADYYCAASGVLGGLHEDWFNYWGQGTLVTVSS | | | |
| **T023200026** | **EGFR#1-CEA#1** | EVQLVESGGGSVQTGGSLRLTCAASGRTSRSYGMGWFRQAPGKEREFV SGISWRGDSTGYADSVKGRFTISRDNAKNTVDLQMNSLKPEDTAIYYCA AAAGSAWYGTLYEYDYWGQGTLVTVSSGGGGSGGGGSGGGGSGGGG SGGGGSGGGGSGGGGSEVQLVESGGGSVQAGGSLRLSCAASGDTYGSY WMGWFRQAPGKEREGVAAINRGGGYTVYADSVKGRFTISRDTAKNTV YLQMNSLRPDDTADYYCAASGVLGGLHEDWFNYWGQGTLVTVSS | | | |
| **T023200027** | **EGFR#1-CEA#5** | EVQLVESGGGSVQTGGSLRLTCAASGRTSRSYGMGWFRQAPGKEREFV SGISWRGDSTGYADSVKGRFTISRDNAKNTVDLQMNSLKPEDTAIYYCA AAAGSAWYGTLYEYDYWGQGTLVTVSSGGGGSGGGGSGGGGSGGGG SGGGGSGGGGSGGGGSEVQLVESGGGSVQAGGSLRLSCAASGDTYGSY WMGWFRQAPGQEREAVAAINRGGGYTVYADSVKGRFTISRDNAKNTL YLQMNSLRPDDTADYYCAASGVLGGLHEDWFNYWGQGTLVTVSS | | | |
| **T023200034** | **EGFR#33-CEA#1** | EVQLVESGGGSVQAGGSLRLTCAASGSTSRSYGMGWFRQAPGKEREFV SGISWRGDSTGYADSVKGRFTISRDNAKNTVDLQMNSLKPEDTAIYYCA AAAGSTWYGTLYEYDYWGQGTLVTVSSGGGGSGGGGSGGGGSGGGG SGGGGSGGGGSGGGGSEVQLVESGGGSVQAGGSLRLSCAASGDTYGSY WMGWFRQAPGKEREGVAAINRGGGYTVYADSVKGRFTISRDTAKNTV YLQMNSLRPDDTADYYCAASGVLGGLHEDWFNYWGQGTLVTVSS | | | |
| **T023200035** | **EGFR#33-CEA#5** | EVQLVESGGGSVQAGGSLRLTCAASGSTSRSYGMGWFRQAPGKEREFV SGISWRGDSTGYADSVKGRFTISRDNAKNTVDLQMNSLKPEDTAIYYCA AAAGSTWYGTLYEYDYWGQGTLVTVSSGGGGSGGGGSGGGGSGGGG SGGGGSGGGGSGGGGSEVQLVESGGGSVQAGGSLRLSCAASGDTYGSY WMGWFRQAPGQEREAVAAINRGGGYTVYADSVKGRFTISRDNAKNTL YLQMNSLRPDDTADYYCAASGVLGGLHEDWFNYWGQGTLVTVSS | | | |
| **T023200028** | **CEA#1-EGFR#1** | EVQLVESGGGSVQAGGSLRLSCAASGDTYGSYWMGWFRQAPGKEREG VAAINRGGGYTVYADSVKGRFTISRDTAKNTVYLQMNSLRPDDTADYYC AASGVLGGLHEDWFNYWGQGTLVTVSSGGGGSGGGGSGGGGSGGGG SGGGGSGGGGSGGGGSEVQLVESGGGSVQTGGSLRLTCAASGRTSRSY GMGWFRQAPGKEREFVSGISWRGDSTGYADSVKGRFTISRDNAKNTV DLQMNSLKPEDTAIYYCAAAAGSAWYGTLYEYDYWGQGTLVTVSS | | | |
| **T023200029** | **CEA#5-EGFR#1** | EVQLVESGGGSVQAGGSLRLSCAASGDTYGSYWMGWFRQAPGQERE AVAAINRGGGYTVYADSVKGRFTISRDNAKNTLYLQMNSLRPDDTADY YCAASGVLGGLHEDWFNYWGQGTLVTVSSGGGGSGGGGSGGGGSGG GGSGGGGSGGGGSGGGGSEVQLVESGGGSVQTGGSLRLTCAASGRTSR SYGMGWFRQAPGKEREFVSGISWRGDSTGYADSVKGRFTISRDNAKNT VDLQMNSLKPEDTAIYYCAAAAGSAWYGTLYEYDYWGQGTLVTVSS | | | |
| **T023200048** | **CEA#1-ctrl** | EVQLVESGGGSVQAGGSLRLSCAASGDTYGSYWMGWFRQAPGKEREG VAAINRGGGYTVYADSVKGRFTISRDTAKNTVYLQMNSLRPDDTADYYC AASGVLGGLHEDWFNYWGQGTLVTVSSGGGGSGGGGSGGGGSGGGG SGGGGSGGGGSGGGGSDVQLQASGGGSVQAGGSLRLSCAASGYTIGPY CMGWFRQAPGKEREGVAAINMGGGITYYADSVKGRFTISQDNAKNTV YLLMNSLEPEDTAIYYCAADSTIYASYYECGHGLSTGGYGYDSWGQGTQ VTVSS | | | |
| **T023200051** | **CEA#5-ctrl** | EVQLVESGGGSVQAGGSLRLSCAASGDTYGSYWMGWFRQAPGQERE AVAAINRGGGYTVYADSVKGRFTISRDNAKNTLYLQMNSLRPDDTADY YCAASGVLGGLHEDWFNYWGQGTLVTVSSGGGGSGGGGSGGGGSGG GGSGGGGSGGGGSGGGGSDVQLQASGGGSVQAGGSLRLSCAASGYTI GPYCMGWFRQAPGKEREGVAAINMGGGITYYADSVKGRFTISQDNAK NTVYLLMNSLEPEDTAIYYCAADSTIYASYYECGHGLSTGGYGYDSWGQ GTQVTVSS | | | |
| **T023200049** | **EGFR#11-ctrl** | EVQLVESGGGSVQAGGSLRLTCAASGRTSRSYGMGWFRQAPGKEREFV SGISWRGDSTGYADSVKGRFTISRDNAKNTVDLQMNSLKPEDTAIYYCA AAAGSTWYGTLYEYDYWGQGTLVTVSSGGGGSGGGGSGGGGSGGGG SGGGGSGGGGSGGGGSDVQLQASGGGSVQAGGSLRLSCAASGYTIGPY CMGWFRQAPGKEREGVAAINMGGGITYYADSVKGRFTISQDNAKNTV YLLMNSLEPEDTAIYYCAADSTIYASYYECGHGLSTGGYGYDSWGQGTQ VTVSS | | | |
| **T023200050** | **EGFR#32-ctrl** | EVQLVESGGGSVQAGGSLRLTCAASGRTSRSYGMGWFRQAPGKEREFV SGISWRGDSTGYADSVKGRFTISRDNAKNTVDLQMNSLKPEDTAIYYCA AAAGSTWYGTLYSYDYWGQGTLVTVSSGGGGSGGGGSGGGGSGGGG SGGGGSGGGGSGGGGSDVQLQASGGGSVQAGGSLRLSCAASGYTIGPY CMGWFRQAPGKEREGVAAINMGGGITYYADSVKGRFTISQDNAKNTV YLLMNSLEPEDTAIYYCAADSTIYASYYECGHGLSTGGYGYDSWGQGTQ | | | |
| | | VTVSS | | | |
| **T023200052** | **EGFR#1-ctrl** | EVQLVESGGGSVQTGGSLRLTCAASGRTSRSYGMGWFRQAPGKEREFV SGISWRGDSTGYADSVKGRFTISRDNAKNTVDLQMNSLKPEDTAIYYCA AAAGSAWYGTLYEYDYWGQGTLVTVSSGGGGSGGGGSGGGGSGGGG SGGGGSGGGGSGGGGSDVQLQASGGGSVQAGGSLRLSCAASGYTIGPY CMGWFRQAPGKEREGVAAINMGGGITYYADSVKGRFTISQDNAKNTV YLLMNSLEPEDTAIYYCAADSTIYASYYECGHGLSTGGYGYDSWGQGTQ VTVSS | | | |
| **T023200053** | **EGFR#33-ctrl** | EVQLVESGGGSVQAGGSLRLTCAASGSTSRSYGMGWFRQAPGKEREFV SGISWRGDSTGYADSVKGRFTISRDNAKNTVDLQMNSLKPEDTAIYYCA AAAGSTWYGTLYEYDYWGQGTLVTVSSGGGGSGGGGSGGGGSGGGG SGGGGSGGGGSGGGGSDVQLQASGGGSVQAGGSLRLSCAASGYTIGPY CMGWFRQAPGKEREGVAAINMGGGITYYADSVKGRFTISQDNAKNTV YLLMNSLEPEDTAIYYCAADSTIYASYYECGHGLSTGGYGYDSWGQGTQ VTVSS | | | |

| | | **c- terminal** | | | |
|---|---|---|---|---|---|
| | | **CEA#1** | **CEA#5** | **EGFR#1** | **control** |
| **n-terminal** | **EGFR#1** | **BI#26** | **BI#27** | | **BI#52** |
| | **EGFR#11** | **BI#22** | **BI#24** | | **BI#49** |
| | **EGFR#32** | **BI#23** | **BI#25** | | **BI#50** |
| | **EGFR#33** | **BI#34** | **BI#35** | | **BI#53** |
| | **CEA#1** | | | **BI#28** | **BI#48** |
| | **CEA#5** | | | **BI#29** | **BI#51** |

**Table 12 CD4-IL12R CD4-IL23R sequences**

| **Nb ID** | **Code used in text** | **Sequence** | | |
|---|---|---|---|---|
| **03F11** | **CD4#8** | EVQLVESGGGSVQPGGSLTLSCGTSGRTFNVMGWFRQAPGKER EFVAAVRWSSTGIYYTQYADSVKSRFTISRDNAKNTVYLEMNSLK PEDTAVYYCAADTYNSNPARWDGYDFRGQGTLVTVSS | | |
| **LG150C02** | **IL23R#18** | EVQLVESGGGLVQSGGSLRLSCAASEGTFTIYPLGWFRQAPGKDR KFVAALPWSAGIPQYSDSVKGRFTISRDNAKNTVYLQMNNLKPE DTAVYYCAAKGRDDSYQPWNYWGQGTLVTVSS | | |
| **LG150D02** | **IL23R#19** | EVQLVESGGGLVQPGGSLTLSCVASGRTFSTDVMGWFRQAPGK EREFVAAHRTSGISTVYAASVKGRFTISRDNAKNTVYLGMKSLKP EDTAVYVCAAGSDASGGYDYWGQGTLVTVSS | | |
| **LG150H07** | **IL23R#20** | EVQLVESGGGLVQAGGSLRLSCAASGRTFSSYAMGWFRQAPGK DREFVAAISWIGESTYYADSVKGRFTISRDNAKNTVYLRMNSLKP EDTAVYYCAADLYYTAYVAAADEYDYWGQGTLVTVSS | | |
| **LG148C09** | **IL12Rb1#30** | EVQLVESGGGLVQTGGSLRLSCAASGRTPRLVAMGWFRQTPGK EREFVGEIILSKGFTYYADSVKGRFTISRVNAKNTITMYLQMNSLK SEDTAVYYCAGRQNWSGSPARTNEYEYWGQGTLVTVSS | | |
| **LG148F09** | **IL12Rb1#31** | EVQLVESGGGLVQTGGSLRLSCAASGRTPSIIAMGWFRQTPGKE REFVGEIILSKGFTYYADSVKGRFTISRANAKNTITMYLQMNSLKS EDTAVYYCAARQNWSGNPTRTNEYEYWGQGTLVTVSS | | |
| **LG135B08** | **IL12Rb2#1** | EVQLVESGGRLVQAGDSLRLSCAASGRTFISYRMGWFRQAPGKE REFVAALRWSSSNIDYTYYADSVKGRFSISGDYAKNTVYLQMNSL KAEDTAVYYCAASTRWGVMESDTEYTSWGQGTLVTVSS | | |
| **LG135A07** | **IL12Rb2#2** | EVQLVESGGRLVQAGDSLRLSCAASGRTFTSYRMGWFRQAPGK EREFVSALRWSSGNIDYTYYADSVKGRFSISGDYAKNTVYLQMNS LKAEDTAVYYCAASTRWGVMESDTEYTSWGQGTLVTVSS | | |
| **T023200036** | **IL12Rb2#1-CD4#8** | EVQLVESGGRLVQAGDSLRLSCAASGRTFISYRMGWFRQAPGKE REFVAALRWSSSNIDYTYYADSVKGRFSISGDYAKNTVYLQMNSL KAEDTAVYYCAASTRWGVMESDTEYTSWGQGTLVTVSSGGGGS GGGGSGGGGSGGGGSGGGGSGGGGSGGGGSEVQLVESGGGS VQPGGSLTLSCGTSGRTFNVMGWFRQAPGKEREFVAAVRWSST GIYYTQYADSVKSRFTISRDNAKNTVYLEMNSLKPEDTAVYYCAA DTYNSNPARWDGYDFRGQGTLVTVSS | | |
| **T023200037** | **IL12Rb2#2-CD4#8** | EVQLVESGGRLVQAGDSLRLSCAASGRTFTSYRMGWFRQAPGK EREFVSALRWSSGNIDYTYYADSVKGRFSISGDYAKNTVYLQMNS LKAEDTAVYYCAASTRWGVMESDTEYTSWGQGTLVTVSSGGGG SGGGGSGGGGSGGGGSGGGGSGGGGSGGGGSEVQLVESGGGS VQPGGSLTLSCGTSGRTFNVMGWFRQAPGKEREFVAAVRWSST GIYYTQYADSVKSRFTISRDNAKNTVYLEMNSLKPEDTAVYYCAA DTYNSNPARWDGYDFRGQGTLVTVSS | | |
| **T023200038** | **CD4#8-IL12Rb2#1** | EVQLVESGGGSVQPGGSLTLSCGTSGRTFNVMGWFRQAPGKER EFVAAVRWSSTGIYYTQYADSVKSRFTISRDNAKNTVYLEMNSLK PEDTAVYYCAADTYNSNPARWDGYDFRGQGTLVTVSSGGGGSG GGGSGGGGSGGGGSGGGGSGGGGSGGGGSEVQLVESGGRLVQ | | |
| | | AGDSLRLSCAASGRTFISYRMGWFRQAPGKEREFVAALRWSSSN IDYTYYADSVKGRFSISGDYAKNTVYLQMNSLKAEDTAVYYCAAS TRWGVMESDTEYTSWGQGTLVTVSS | | |
| **T023200039** | **CD4#8-IL12Rb2#2** | EVQLVESGGGSVQPGGSLTLSCGTSGRTFNVMGWFRQAPGKER EFVAAVRWSSTGIYYTQYADSVKSRFTISRDNAKNTVYLEMNSLK PEDTAVYYCAADTYNSNPARWDGYDFRGQGTLVTVSSGGGGSG GGGSGGGGSGGGGSGGGGSGGGGSGGGGSEVQLVESGGRLVQ AGDSLRLSCAASGRTFTSYRMGWFRQAPGKEREFVSALRWSSG NIDYTYYADSVKGRFSISGDYAKNTVYLQMNSLKAEDTAVYYCA ASTRWGVMESDTEYTSWGQGTLVTVSS | | |
| **T023200040** | **CD4#8-IL12Rb1#30** | EVQLVESGGGSVQPGGSLTLSCGTSGRTFNVMGWFRQAPGKER EFVAAVRWSSTGIYYTQYADSVKSRFTISRDNAKNTVYLEMNSLK PEDTAVYYCAADTYNSNPARWDGYDFRGQGTLVTVSSGGGGSG GGGSGGGGSGGGGSGGGGSGGGGSGGGGSEVQLVESGGGLV QTGGSLRLSCAASGRTPRLVAMGWFRQTPGKEREFVGEIILSKGF TYYADSVKGRFTISRVNAKNTITMYLQMNSLKSEDTAVYYCAGR QNWSGSPARTNEYEYWGQGTLVTVSS | | |
| **T023200041** | **CD4#8-IL12Rb1#31** | EVQLVESGGGSVQPGGSLTLSCGTSGRTFNVMGWFRQAPGKER EFVAAVRWSSTGIYYTQYADSVKSRFTISRDNAKNTVYLEMNSLK PEDTAVYYCAADTYNSNPARWDGYDFRGQGTLVTVSSGGGGSG GGGSGGGGSGGGGSGGGGSGGGGSGGGGSEVQLVESGGGLV QTGGSLRLSCAASGRTPSIIAMGWFRQTPGKEREFVGEIILSKGFT YYADSVKGRFTISRANAKNTITMYLQMNSLKSEDTAVYYCAARQ NWSGNPTRTNEYEYWGQGTLVTVSS | | |
| **T023200042** | **IL23R#19-CD4#8** | EVQLVESGGGLVQPGGSLTLSCVASGRTFSTDVMGWFRQAPGK EREFVAAHRTSGISTVYAASVKGRFTISRDNAKNTVYLGMKSLKP EDTAVYVCAAGSDASGGYDYWGQGTLVTVSSGGGGSGGGGSG GGGSGGGGSGGGGSGGGGSGGGGSEVQLVESGGGSVQPGGSL TLSCGTSGRTFNVMGWFRQAPGKEREFVAAVRWSSTGIYYTQY ADSVKSRFTISRDNAKNTVYLEMNSLKPEDTAVYYCAADTYNSN PARWDGYDFRGQGTLVTVSS | | |
| **T023200043** | **IL23R#20-CD4#8** | EVQLVESGGGLVQAGGSLRLSCAASGRTFSSYAMGWFRQAPGK DREFVAAISWIGESTYYADSVKGRFTISRDNAKNTVYLRMNSLKP EDTAVYYCAADLYYTAYVAAADEYDYWGQGTLVTVSSGGGGSG GGGSGGGGSGGGGSGGGGSGGGGSGGGGSEVQLVESGGGSV QPGGSLTLSCGTSGRTFNVMGWFRQAPGKEREFVAAVRWSSTG IYYTQYADSVKSRFTISRDNAKNTVYLEMNSLKPEDTAVYYCAAD TYNSNPARWDGYDFRGQGTLVTVSS | | |
| **T023200044** | **CD4#8-IL23R#20** | EVQLVESGGGSVQPGGSLTLSCGTSGRTFNVMGWFRQAPGKER EFVAAVRWSSTGIYYTQYADSVKSRFTISRDNAKNTVYLEMNSLK PEDTAVYYCAADTYNSNPARWDGYDFRGQGTLVTVSSGGGGSG GGGSGGGGSGGGGSGGGGSGGGGSGGGGSEVQLVESGGGLV QAGGSLRLSCAASGRTFSSYAMGWFRQAPGKDREFVAAISWIGE STYYADSVKGRFTISRDNAKNTVYLRMNSLKPEDTAVYYCAADLY YTAYVAAADEYDYWGQGTLVTVSS | | |
| **T023200045** | **CD4#8-IL23R#19** | EVQLVESGGGSVQPGGSLTLSCGTSGRTFNVMGWFRQAPGKER EFVAAVRWSSTGIYYTQYADSVKSRFTISRDNAKNTVYLEMNSLK PEDTAVYYCAADTYNSNPARWDGYDFRGQGTLVTVSSGGGGSG GGGSGGGGSGGGGSGGGGSGGGGSGGGGSEVQLVESGGGLV QPGGSLTLSCVASGRTFSTDVMGWFRQAPGKEREFVAAHRTSGI STVYAASVKGRFTISRDNAKNTVYLGMKSLKPEDTAVYVCAAGS DASGGYDYWGQGTLVTVSS | | |
| **T023200046** | **IL12Rb1#30-CD4#8** | EVQLVESGGGLVQTGGSLRLSCAASGRTPRLVAMGWFRQTPGK EREFVGEIILSKGFTYYADSVKGRFTISRVNAKNTITMYLQMNSLK SEDTAVYYCAGRQNWSGSPARTNEYEYWGQGTLVTVSSGGGGS GGGGSGGGGSGGGGSGGGGSGGGGSGGGGSEVQLVESGGGS VQPGGSLTLSCGTSGRTFNVMGWFRQAPGKEREFVAAVRWSST GIYYTQYADSVKSRFTISRDNAKNTVYLEMNSLKPEDTAVYYCAA DTYNSNPARWDGYDFRGQGTLVTVSS | | |
| **T023200047** | **IL12Rb1#31-CD4#8** | EVQLVESGGGLVQTGGSLRLSCAASGRTPSIIAMGWFRQTPGKE REFVGEIILSKGFTYYADSVKGRFTISRANAKNTITMYLQMNSLKS EDTAVYYCAARQNWSGNPTRTNEYEYWGQGTLVTVSSGGGGS GGGGSGGGGSGGGGSGGGGSGGGGSGGGGSEVQLVESGGGS VQPGGSLTLSCGTSGRTFNVMGWFRQAPGKEREFVAAVRWSST GIYYTQYADSVKSRFTISRDNAKNTVYLEMNSLKPEDTAVYYCAA DTYNSNPARWDGYDFRGQGTLVTVSS | | |

| | | **c- terminal** | | |
|---|---|---|---|---|
| | | **CD4#8** | **IL12Rb2#1** | **IL12Rb2#2** |
| **n-terminal** | **CD4#8** | | **BI#38** | **BI#39** |
| | **IL12Rb2#1** | **BI#36** | | |
| | **IL12Rb2#2** | **BI#37** | | |

| | | **c- terminal** | | |
|---|---|---|---|---|
| | | **CD4#8** | **IL12Rb1#30** | **IL12Rb1#31** |
| **n-terminal** | **CD4#8** | | **BI#40** | **BI#41** |
| | **IL12Rb1#30** | **BI#46** | | |
| | **IL12Rb1#31** | **BI#47** | | |

| | | **c- terminal** | | |
|---|---|---|---|---|
| | | **CD4#8** | **IL23R#19** | **IL23R#20** |
| **n-terminal** | **CD4#8** | | **BI#45** | **BI#44** |
| | **IL23R#19** | **BI#42** | | |
| | **IL23R#20** | **BI#43** | | |

**Table 13 CXCR4-CD123**

| **Nanobody ID** | | | | | | **Code used in text:** |
|---|---|---|---|---|---|---|
| **A011000003** | **4CXCR281F12(Q108L)-35GS-A0110055A01-CMYC-HIS6** | | | | | **CXCR4#2-CD123#5** |
| **A011000004** | **4CXCR281F12(Q108L)-35GS-A0110057A07-CMYC-HIS6** | | | | | **CXCR4#2-CD123#7** |
| **A011000007** | **4CXCR014D09(Q108L)-35GS-A0110055A01-CMYC-HIS6** | | | | | **CXCR4#1-CD123#5** |
| **A011000008** | **A0110055A01-35GS-4CXCR014D09(Q108L)-CMYC-HIS6** | | | | | **CD123#5-CXCR4#1** |
| **A011000010** | **A0110057A07-35GS-4CXCR281F12(Q108L)-CMYC-HIS6** | | | | | **CD123#7-CXCR4#2** |
| **A011000011** | **A0110057A07-35GS-4CXCR014D09(Q108L)-CMYC-HIS6** | | | | | **CD123#7-CXCR4#1** |
| **A011000015** | **A0110055A01-35GS-4CXCR281F12(Q108L)-CMYC-HIS6** | | | | | **CD123#5-CXCR4#2** |
| **A011000016** | **4CXCR014D09(Q108L)-35GS-A0110057A07-CMYC-HIS6** | | | | | **CXCR4#1-CD123#7** |
| | | | | | | |
| **A011000017** | **4CXCR281F12-35GS-A0110055A01-FLAG3-HIS6** | | | | | **CXCR4#2-CD123#5** |
| **A011000018** | **4CXCR281F12-35GS-A0110057A07-FLAG3-HIS6** | | | | | **CXCR4#2-CD123#7** |
| **A011000019** | **4CXCR014D09-35GS-A0110055A01-FLAG3-HIS6** | | | | | **CXCR4#1-CD123#5** |
| **A011000020** | **4CXCR014D09-35GS-A0110057A07-FLAG3-HIS6** | | | | | **CXCR4#1-CD123#7** |
| **A011000021** | **A0110057A07-35GS-4CXCR281F12-FLAG3-HIS6** | | | | | **CD123#7-CXCR4#2** |
| **A011000022** | **A0110055A01-35GS-4CXCR281F12-FLAG3-HIS6** | | | | | **CD123#5-CXCR4#1** |
| **A011000023** | **A0110057A07-35GS-4CXCR014D09-FLAG3-HIS6** | | | | | **CD123#7-CXCR4#1** |
| **A011000024** | **A0110055A01-35GS-4CXCR014D09-FLAG3-HIS6** | | | | | **CD123#5-CXCR4#2** |
| **A011000025** | **4CXCR281F12(L108Q)-35GS-4CD003F11(L108Q)-FLAG3- HIS6** | | | | | **CXCR#2-CD4#8** |
| **A011000026** | **4CD003F11(L108Q)-35GS-4CXCR281F12(L108Q)-FLAG3- HIS6** | | | | | **CD4#2-CXCR4#2** |
| **A011000027** | **4CXCR281F12-Flag3-His6** | | | | | **CXCR4#2** |
| **A011000028** | **4CXCR014D09-Flag3-His6** | | | | | **CXCR4#1** |

| | | **c- terminal** | | | | |
|---|---|---|---|---|---|---|
| | | **CXCR4#2** | **CXCR4#1** | **CD123#7** | **CD123#5** | |
| **n- terminal** | **CXCR4#2** | | | **BI#4/18** | **BI#3/17** | |
| | **CXCR4#1** | | | **BI#16/20** | **BI#7/19** | |
| | **CD123#7** | **BI#10/21** | **BI#11/23** | | | |
| | **CD123#5** | **BI#15/24** | **BI#8/22** | | | |

**Table B-4: Albumin binder sequences of the disclosure**

| | | |
|---|---|---|
| Alb11 | 114 | |
| Alb8 | 115 | EVQLVESGGGLVQPGNSLRLSCAASGFTFSSFGMSWVRQAPGKGLEWV SSISGSGSDTLYADSVKGRFTISRDNAKTTLYLQMNSLRPEDTAVYYC TIGGSLSRSSQGTLVTVSSAAAEQKLISEEDLNGAAHHHHHH |

**Table B-5: Linker sequences of the disclosure**

| Name of linker | SEQ ID NO: | Amino acid sequences |
|---|---|---|
| 5GS | 117 | GGGGS |
| 6GS | 118 | SGGSGGS |
| 9GS | 119 | GGGGSGGGS |
| 10GS | 120 | GGGGSGGGGS |
| 15GS | 121 | GGGGSGGGGSGGGGS |
| 18GS | 122 | GGGGSGGGGSGGGGGGGS |
| 20GS | 123 | GGGGSGGGGSGGGGSGGGGS |
| 25GS | 124 | GGGGSGGGGSGGGGSGGGGSGGGGS |
| 30GS | 125 | GGGGSGGGGSGGGGSGGGGSGGGGSGGGGS |
| 35GS | 126 | GGGGSGGGGSGGGGSGGGGSGGGGSGGGGSGGGGS |

## Claims

1. Polypeptide comprising a first and a second immunoglobulin single variable domain (ISV), wherein
- said first ISV binds to a first target with an average KD value of between 10 nM and 200 nM as measured by surface plasmon resonance;
- said second ISV binds to a second target with an average KD value of between 10 nM and 0.1 pM as measured by surface plasmon resonance; and
wherein said first ISV and said second ISV bind to said first target and said second target present on the surface of the same cell,
wherein said first target is different from said second target,
wherein said second ISV enhances binding of said first ISV,
wherein binding by said first ISV inhibits a function of said first target,
wherein said first target is chosen from the group consisting of Receptor Tyrosine Kinases (preferably class I), GPCRs, DDR1, Discoidin I (CD167a antigen), DDR2, ErbB-1, C-erbB-2, FGFR-1, FGFR-3, CD135 antigen, CD 117 antigen, Protein tyrosine kinase-1, c-Met, CD148 antigen, C-ret, ROR1, ROR2, Tie-1, Tie-2, CD202b antigen, Trk-A, Trk-B, Trk-C, VEGFR-1, VEGFR-2, VEGFR-3, Notch receptor 1-4, FAS receptor, DR5, DR4, CD47, CX3CR1, CXCR-3, CXCR-4, CXCR-7, Chemokine binding protein 2, and CCR1, CCR2, CCR3, CCR4, CCR5, CCR6, CCR7, CCR8, CCR9, CCR10 and CCR11; and
said second target is chosen from the group consisting of carcinoembryonic antigen ("CEA"), MART-1, gp100, MAGE-1, HER-2, and Lewis^{Y} antigens, CD123, CD44, CLL-1, CD96, CD47, CD32, CXCR4, Tim-3, CD25, TAG-72, Ep-CAM, PSMA, PSA, GD2, GD3, CD4, CD5, CD19, CD20, CD22, CD33, CD36, CD45, CD52, and CD147; and Cytokine receptors including Interleukin-2 receptor gamma chain (CD132 antigen); Interleukin-10 receptor alpha chain (IL-10R-A); Interleukin-10 receptor beta chain (IL-10R-B); Interleukin-12 receptor beta-1 chain (IL-12R-beta1); Interleukin-12 receptor beta-2 chain (IL-12 receptor beta-2); Interleukin-13 receptor alpha-1 chain (IL-13R-alpha-1) (CD213 al antigen); Interleukin-13 receptor alpha-2 chain (Interleukin-13 binding protein); Interleukin-17 receptor (IL-17 receptor); Interleukin-17B receptor (IL-17B receptor); Interleukin 21 receptor precursor (IL-21R); Interleukin-1 receptor, type I (IL-1R-1) (CD121a); Interleukin-1 receptor, type II (IL-1R-beta) (CDw121b); Interleukin-1 receptor antagonist protein (IL-1ra); Interleukin-2 receptor alpha chain (CD25 antigen); Interleukin-2 receptor beta chain (CD122 antigen); Interleukin-3 receptor alpha chain (IL-3R-alpha) (CD123 antigen).

2. The polypeptide according to claim 1, wherein said cell is a diseased cell, preferably a cancer cell.

3. The polypeptide according to claim 1 or 2, wherein said cell comprises a ratio of 0.01 to 0.9 of said first target and said second target, even more preferably between 0.2 to 0.8, 0.3 to 0.7, 0.4 to 0.6, such as a ratio of 0.02, 0.05, 0.08, 0.1, 0.2, 0.3, 0.4, 0.5, 0.6, 0.7, 0.8, 0.9, preferably a ratio of 0.5.

4. The polypeptide according to any of claims 1 to 3, wherein said polypeptide
- has an on rate constant (Kon) to said first target selected from the group consisting of: about 10² M⁻¹s⁻¹, about 10³ M⁻¹s⁻¹, about 10⁴ M⁻¹s⁻¹, at least about 10⁵ M⁻¹s⁻¹, and about 10⁶ M⁻¹s⁻¹, as measured by surface plasmon resonance, and/or
- has an off rate constant (Koff) to said first target selected from the group consisting of: about 10⁻³s⁻¹, about 10⁻⁴s⁻¹, about 10⁻⁵s⁻¹, and about 10⁻⁶s⁻¹, as measured by surface plasmon resonance, and/or
- has a dissociation constant (K_{D}) to said first target selected from the group consisting of: about 10⁻⁷ M, about 10⁻⁸ M, about 10⁻⁹ M, about 10⁻¹⁰ M, about 10⁻¹¹ M, and about 10⁻¹² M, as measured by surface plasmon resonance.

5. The polypeptide according to any of claims 1 to 4, wherein said first ISV
- binds to a first target with an average KD value of between 10 nM and 200 nM, such as an average KD value of 10, 15, 20, 30, 40, 50, 60, 70, 80, 90, 100, 110, 120, 130, 140, 150, 160, 170, 180, or 190 nM, as measured by SPR, and/or
- inhibits chemotaxis by about 10%, 20%, 30%, 40%, 50%, 60%, 80%, 90% and preferably 95% or more in a chemotaxis assay, and/or
- inhibits anaplasia, invasiveness, metastasis, proliferation, differentiation, migration and/or survival of said cell, and/or
- increases apoptosis, cell killing and/or growth arrest of said cell.

6. The polypeptide according to any of claims 1 to 5, wherein said second ISV
- binds to a second target with an average KD value of between 10 nM and 0.1 pM, such as at an average KD value of 10 nM or less, even more preferably at an average KD value of 9 nM or less, such as less than 8, 7, 6, 5, 4, 3, 2, 1, 0.5 nM or even less, such as less than 400, 300, 200, 100, 50, 40, 30, 20, 10, 9, 8, 7, 6, 5, 4, 3, 2, 1, 0.5 pM, or even less such as less than 0.4 pM, as measured by SPR, and/or
- inhibits binding of a natural ligand to said second target by less than 50%, such as 40%, 30%, or 20% or even less than 10%, such as less than 5%.

7. The polypeptide according to any one of claims 1 to 6, wherein said first target and said second target are chosen from the group consisting of:
- EGFR as first target and CEA as a second target;
- Receptor Tyrosine Kinase as a first target and a tumor-associated antigen (TAA) as a second target;
- G-Protein-Coupled Receptor (GPCR) as a first target and a hematopoietic differentiation antigen as a second target;
- Receptor Tyrosine Kinase as a first target and a hematopoietic differentiation antigen as a second target;
- G-Protein-Coupled Receptor (GPCR) as a first target and a tumor-associated antigen (TAA) as a second target;
- CXCR4 as a first target and CD123 as a second target;
- DR5 as first target and EpCam as a second target;
- DR4 as first target and EpCam as a second target;
- CD95 as first target and EpCam as a second target;
- CD47as first target and CD123 as a second target;
- CD47 as first target and EpCam as a second target;
- CD4 as first target and CXCR4 as a second target;
- IL12Rβ1 as first target and CD4 as a second target;
- IL12Rβ2 as first target and CD4 as a second target; and
- IL23R as first target and CD4 as a second target.

8. The polypeptide according to claim 7, wherein said first target and said second target are chosen from the group consisting of:
- EGFR as first target and CEA as a second target;
- CXCR4 as a first target and CD123 as a second target;
- CD4 as first target and CXCR4 as a second target;
- IL12Rβ2 as first target and CD4 as a second target; and
- IL23R as first target and CD4 as a second target.

9. The polypeptide according to claim 8,
- wherein said ISV binding EGFR is chosen from the group consisting of SEQ ID NO:s 39-45;
- wherein said ISV binding CEA is chosen from the group consisting of SEQ ID NO:s 32-38;
- wherein said ISV binding CXCR4 is chosen from the group consisting of SEQ ID NO:s 1-8 and 24;
- wherein said ISV binding CD123 is chosen from the group consisting of SEQ ID NO:s 9-15;
- wherein said ISV binding CD4 is chosen from the group consisting of SEQ ID NO:s 26 and 62;
- wherein said ISV binding IL12Rβ1 is chosen from the group consisting of SEQ ID NO:s 66-67;
- wherein said ISV binding IL12Rβ2 is chosen from the group consisting of SEQ ID NO:s 68-69;
- wherein said ISV binding IL23R is chosen from the group consisting of SEQ ID NO:s 63-65.

10. The polypeptide according to claim 8 chosen from the group consisting of SEQ ID NO:s 16-23, 26-31, 46-55 and 70-81.

11. The polypeptide according to any of claims 1 to 10, further comprising a drug, such as a toxin or toxin moiety.

12. The polypeptide according to any of claims 1 to 10, further comprising an imaging agent, including, but not limited to a molecule preferably selected from the group consisting of organic molecules, enzyme labels, radioactive labels, colored labels, fluorescent labels, chromogenic labels, luminescent labels, haptens, digoxigenin, biotin, metal complexes, metals, colloidal gold, fluorescent label, metallic label, biotin, chemiluminescent, bioluminescent, chromophore and mixtures thereof.

13. Pharmaceutical composition comprising a polypeptide according to any of claims 1 to 12.

14. Polypeptide according to any of claims 1 to 12 for use in a method for delivering a prophylactic or therapeutic polypeptide, a polypeptide-drug conjugate (PDC) or imaging agent to a specific location, tissue or cell type in the body, the method comprising the steps of administering to a subject a polypeptide according to any of claims 1 to 12.

15. Polypeptide according to any of claims 1 to 12 for use in treating a subject in need thereof.

## Patentansprüche

1. Polypeptid, umfassend eine erste und eine zweite variable Immunglobulin-Einzeldomäne (ISV), wobei
- die erste ISV an ein erstes Ziel mit einem durchschnittlichen KD-Wert von zwischen 10 nM und 200 nM, gemessen durch Oberflächenplasmonresonanz (*"surface plasmon resonance*"), bindet;
- die zweite ISV an ein zweites Ziel mit einem durchschnittlichen KD-Wert von zwischen 10 nM und 0,1 pM, gemessen durch Oberflächenplasmonresonanz, bindet;
und
wobei die erste ISV und die zweite ISV an das auf der Oberfläche derselben Zelle befindliche erste Ziel und zweite Ziel binden,
wobei das erste Ziel sich vom zweiten Ziel unterscheidet,
wobei die zweite ISV die Bindung der ersten ISV verstärkt;
wobei die Bindung durch die erste ISV eine Funktion des ersten Ziels inhibiert,
wobei das erste Ziel ausgewählt ist aus der Gruppe, bestehend aus: Rezeptor-Tyrosin-Kinasen (bevorzugt Klasse I), GPCRs, DDR1, Discoidin I (CD167a-Antigen), DDR2, ErbB-1, C-erbB-2, FGFR-1, FGFR-3, CD135-Antigen, CD 117-Antigen, Protein-Tyrosin-Kinase-1, c-Met, CD148-Antigen, C-ret, ROR1, ROR2, Tie-1, Tie-2, CD202b-Antigen, Trk-A, Trk-B, Trk-C, VEGFR-1, VEGFR-2, VEGFR-3, Notch-Rezeptor 1-4, FAS-Rezeptor, DR5, DR4, CD47, CX3CR1, CXCR-3, CXCR-4, CXCR-7, Chemokin-Bindeprotein 2 und CCR1, CCR2, CCR3, CCR4, CCR5, CCR6, CCR7, CCR8, CCR9, CCR10 und CCR11; und
das zweite Ziel ausgewählt ist aus der Gruppe, bestehend aus: carcinoembryonalem Antigen ("CEA"), MART-1, gp100, MAGE-1, HER-2 und LewisY-Antigenen, CD123, CD44, CLL-1, CD96, CD47, CD32, CXCR4, Tim-3, CD25, TAG-72, Ep-CAM, PSMA, PSA, GD2, GD3, CD4, CD5, CD19, CD20, CD22, CD33, CD36, CD45, CD52 und CD147; und Zytokin-Rezeptoren, einschließlich Interleukin-2-Rezeptor-gamma-Kette(CD132-Antigen); Interleukin-10-Rezeptor-alpha-Kette (IL-10R-A); Interleukin-10-Rezeptor-beta-Kette (IL-10R-B); Interleukin-12-Rezeptor-beta-1-Kette (IL-12R-beta1); Interleukin-12-Rezeptor-beta-2-Kette (IL-12-Rezeptor beta-2); Interleukin-13-Rezeptor-alpha-1-Kette (IL-13R-alpha-1) (CD213 al-Antigen); Interleukin-13-Rezeptor-alpha-2-Kette (Interleukin-13-Bindeprotein); Interleukin-17-Rezeptor (IL-17-Rezeptor); Interleukin-17B-Rezeptor (IL-17B-Rezeptor); Interleukin 21-Rezeptor-Vorläufer (IL-21R); Interleukin-1-Rezeptor, Typ I (IL-1R-1) (CD121a); Interleukin-1-Rezeptor, Typ II (IL-1R-beta) (CDw121b); Interleukin-1-Rezeptor-Antagonist-Protein (IL-1ra); Interleukin-2-Rezeptor-alpha-Kette (CD25-Antigen); Interleukin-2-Rezeptor-beta-Kette (CD122-Antigen); Interleukin-3-Rezeptor-alpha-Kette (IL-3R-alpha) (CD123-Antigen).

2. Polypeptid gemäß Anspruch 1, wobei die Zelle eine erkrankte Zelle ist, bevorzugt eine Krebszelle.

3. Polypeptid gemäß Anspruch 1 oder 2, wobei die Zelle ein Verhältnis von 0,01 bis 0,9 an erstem Ziel und zweitem Ziel umfasst, noch bevorzugter zwischen 0,2 bis 0,8, 0,3 bis 0,7, 0,4 bis 0,6, wie beispielsweise ein Verhältnis von 0,02, 0,05, 0,08, 0,1, 0,2, 0,3, 0,4, 0,5, 0,6, 0,7, 0,8, 0,9, bevorzugt ein Verhältnis von 0,5.

4. Polypeptid gemäß einem der Ansprüche 1 bis 3, wobei das Polypeptid
- eine Bindungsratenkonstante ("*on rate constant",* Kon) zum ersten Ziel aufweist, ausgewählt aus der Gruppe bestehend aus: etwa 10² M⁻¹s⁻¹, etwa 10³ M⁻¹s⁻¹, etwa 10⁴ M⁻¹s⁻¹, wenigstens etwa 10⁵ M⁻¹s⁻¹ und etwa 10⁶ M⁻¹s⁻¹, gemessen durch Oberflächenplasmonresonanz, und/oder
- eine Dissoziationsratenkonstante ("*off rate constant",* Koff) zum ersten Ziel aufweist, ausgewählt aus der Gruppe, bestehend aus: etwa 10⁻³s⁻¹, etwa 10⁻⁴s⁻¹, etwa 10⁻⁵s⁻¹ und etwa 10⁻⁶s⁻¹, gemessen durch Oberflächenplasmonresonanz, und/oder
- eine Dissoziationskonstante (KD) zum ersten Ziel aufweist, ausgewählt aus der Gruppe, bestehend aus: etwa 10⁻⁷ M, etwa 10⁻⁸ M, ewta 10⁻⁹ M, etwa 10⁻¹⁰ M, etwa 10⁻¹¹ M und etwa 10⁻¹² M, gemessen durch Oberflächenplasmonresonanz.

5. Polypeptid gemäß einem der Ansprüche 1 bis 4, wobei die erste ISV
- an ein erstes Ziel mit einem durchschnittlichen KD-Wert von zwischen 10 nM und 200 nM bindet, wie beispielsweise einem durchschnittlichen KD-Wert von 10, 15, 20, 30, 40, 50, 60, 70, 80, 90, 100, 110, 120, 130, 140, 150, 160, 170, 180 oder 190 nM, gemessen durch SPR, und/oder
- Chemotaxis zu etwa 10%, 20%, 30%, 40%, 50%, 60%, 80% 90% und bevorzugt 95% oder mehr in einem Chemotaxis-Assay inhibiert, und/oder
- Anaplasie, Invasivität, Metastasenbildung, Proliferation, Differenzierung, Migration und/oder Überleben der Zelle inhibiert, und/oder
- Apoptose, Zelltötung und/oder Wachstumsstillstand der Zelle erhöht.

6. Polypeptid gemäß einem der Ansprüche 1 bis 5, wobei die zweite ISV
- an ein zweites Ziel mit einem durchschnittlichen KD-Wert von zwischen 10 nM und 0,1 pM bindet, wie beispielsweise einem durchschnittlichen KD-Wert von 10 nM oder weniger, noch bevorzugter mit einem durchschnittlichen KD-Wert von 9 nM oder weniger, wie beispielsweise weniger als 8, 7, 6, 5, 4, 3, 2, 1, 0,5 nM oder noch weniger, wie beispielsweise 400, 300, 200, 100, 50, 40, 30, 20, 10, 9, 8, 7, 6, 5, 4, 3, 2, 1, 0,5 pM oder noch weniger, wie beispielsweise weniger als 0,4 pM, gemessen durch SPR, und/oder
- die Bindung eines natürlichen Liganden an das zweite Ziel um weniger als 50% inhibiert, wie beispielsweise 40%, 30% oder 20% oder sogar weniger als 10%, wie beispielsweise weniger als 5%.

7. Polypeptid gemäß einem der Ansprüche 1 bis 6, wobei das erste Ziel und das zweite Ziel ausgewählt sind aus der Gruppe, bestehend aus:
- EGFR als erstes Ziel und CEA als ein zweites Ziel;
- Rezeptor-Tyrosin-Kinase als ein erstes Ziel und ein Tumor-assoziiertes Antigen (TAA) als ein zweites Ziel;
- G-Protein-verknüpfter Rezeptor (GPCR) als ein erstes Ziel und ein hämatopoietisches Differenzierungsantigen als ein zweites Ziel;
- Rezeptor-Tyrosin-Kinase als ein erstes Ziel und ein hämatopoietisches Differenzierungsantigen als ein zweites Ziel;
- G-Protein-verknüpfter Rezeptor (GPCR) als ein erstes Ziel und ein Tumor-assoziiertes Antigen (TAA) als ein zweites Ziel;
- CXCR4 als ein erstes Ziel und CD123 als ein zweites Ziel;
- DR5 als erstes Ziel und EpCam als ein zweites Ziel;
- DR4 als erstes Ziel und EpCam als ein zweites Ziel;
- CD95 als erstes Ziel und EpCam als ein zweites Ziel;
- CD47 als erstes Ziel und CD123 als ein zweites Ziel;
- CD47 als erstes Ziel und EpCam als ein zweites Ziel;
- CD4 als erstes Ziel und CXCR4 als ein zweites Ziel;
- IL12Rβ1 als erstes Ziel und CD4 als ein zweites Ziel;
- IL12Rβ2 als erstes Ziel und CD4 als ein zweites Ziel; und
- IL23R als erstes Ziel und CD4 als ein zweites Ziel.

8. Polypeptid gemäß Anspruch 7, wobei das erste Ziel und das zweite Ziel ausgewählt sind aus der Gruppe, bestehend aus:
- EGFR als erstes Ziel und CEA als ein zweites Ziel;
- CXCR4 als ein erstes Ziel und CD123 als ein zweites Ziel;
- CD4 als erstes Ziel und CXCR4 als ein zweites Ziel;
- IL12Rβ2 als erstes Ziel und CD4 als ein zweites Ziel; und
- IL23R als erstes Ziel und CD4 als ein zweites Ziel.

9. Polypeptid gemäß Anspruch 8,
- wobei die EGFR-bindende ISV ausgewählt ist aus der Gruppe, bestehend aus SEQ ID NO:s 39-45;
- wobei die CEA-bindende ISV ausgewählt ist aus der Gruppe, bestehend aus SEQ ID NO:s 32-38;
- wobei die CXCR4-bindende ISV ausgewählt ist aus der Gruppe, bestehend aus SEQ ID NO:s 1-8 und 24;
- wobei die CD123-bindende ISV ausgewählt ist aus der Gruppe, bestehend aus SEQ ID NO:s 9-15;
- wobei die CD4-bindende ISV ausgewählt ist aus der Gruppe, bestehend aus SEQ ID NO:s 26 und 62;
- wobei die IL12Rβ1-bindende ISV ausgewählt ist aus der Gruppe, bestehend aus SEQ ID NO:s 66-67;
- wobei die IL12Rβ2-bindende ISV ausgewählt ist aus der Gruppe, bestehend aus SEQ ID NO:s 68-69;
- wobei die IL23R-bindende ISV ausgewählt ist aus der Gruppe, bestehend aus SEQ ID NO:s 63-65.

10. Polypeptid gemäß Anspruch 8, ausgewählt aus der Gruppe, bestehend aus SEQ ID NO:s 16-23, 26-31, 46-55 und 70-81.

11. Polypeptid gemäß einem der Ansprüche 1 bis 10, weiterhin umfassend einen Arzneistoff, wie beispielsweise ein Toxin oder einen Toxin-Rest.

12. Polypeptid gemäß einem der Ansprüche 1 bis 10, weiterhin umfassend ein Bildgebungsmittel, einschließlich, aber nicht darauf beschränkt, ein Molekül, bevorzugt ausgewählt aus der Gruppe, bestehend aus organischen Molekülen, Enzymmarkierungen, radioaktiven Markierungen, farbigen Markierungen, fluoreszenten Markierungen, chromogenen Markierungen, lumineszenten Markierungen, Haptenen, Digoxigenin, Biotin, Metall-Komplexen, Metallen, kolloidalem Gold, fluoreszenter Markierung, metallischer Markierung, Biotin, Chemilumineszentem, Biolumineszentem, Chromophoren und Mischungen davon.

13. Pharmazeutische Zusammensetzung, umfassend ein Polypeptid gemäß einem der Ansprüche 1 bis 12.

14. Polypeptid gemäß einem der Ansprüche 1 bis 12 zur Verwendung in einem Verfahren zum Zuführen eines prophylaktischen oder therapeutischen Polypeptids, eines Polypeptid-Arzneistoff-Konjugats (PDC) oder Bildgebungsmittels an eine spezifische Stelle, Gewebe oder Zelltyp im Körper, wobei das Verfahren die Schritte des Verabreichens eines Polypeptids gemäß einem der Ansprüche 1 bis 12 an ein Subjekt umfasst.

15. Polypeptid gemäß einem der Ansprüche 1 bis 12 zur Verwendung bei der Behandlung eines Subjekts, welches eine solche benötigt.

## Revendications

1. Polypeptide comprenant des premier et second domaines variables uniques (ISV) d'immunoglobuline, dans lequel
- ledit premier ISV se lie à une première cible avec une valeur KD moyenne comprise entre 10 nM et 200 nM telle que mesurée par résonance plasmonique de surface ;
- ledit second ISV se lie à une seconde cible avec une valeur KD moyenne comprise entre 10 nM et 0,1 pM telle que mesurée par résonance plasmonique de surface ; et
dans lequel ledit premier ISV et ledit second ISV se lient à ladite première cible et à ladite seconde cible présentes à la surface de la même cellule,
dans lequel ladite première cible est différente de ladite seconde cible,
dans lequel ledit second ISV améliore la liaison dudit premier ISV,
dans lequel la liaison par ledit premier ISV inhibe une fonction de ladite première cible,
dans lequel ladite première cible est choisie dans le groupe se composant de récepteur à activité tyrosine kinase (de préférence de classe I), GPCRs, DDR1, discoïdine I (antigène CD167a), DDR2, ErbB-1, C-erbB-2, FGFR-1, FGFR-3, antigène CD135, antigène CD 117, protéine tyrosine kinase-1, c-Met, antigène CD148, C-ret, ROR1, ROR2, Tie-1, Tie-2, antigène CD202b, Trk-A, Trk-B, Trk-C, VEGFR-1, VEGFR-2, VEGFR-3, récepteur Notch 1-4, récepteur FAS, DR5, DR4, CD47, CX3CR1, CXCR-3, CXCR-4, CXCR-7, protéine de liaison à la chimiokine 2 et CCR1, CCR2, CCR3, CCR4, CCR5, CCR6, CCR7, CCR8, CCR9, CCR10 et CCR11 ; et
ladite seconde cible est choisie dans le groupe se composant de : antigène carcino-embryonnaire ("CEA"), antigènes MART-1, gp100, MAGE-1, HER-2 et Lewis^{Y}, CD123, CD44, CLL-1, CD96, CD47, CD32, CXCR4, Tim-3, CD25, TAG-72, Ep-CAM, PSMA, PSA, GD2, GD3, CD4, CD5, CD19, CD20, CD22, CD33, CD36, CD45, CD52 et CD147 ; et des récepteurs de cytokine incluant la chaîne gamma du récepteur de l'interleukine-2 (antigène CD132) ; chaîne alpha du récepteur de l'interleukine-10 (IL-10R-A) ; chaîne bêta de récepteur de l'interleukine-10 (IL-10R-B) ; chaîne bêta-1 du récepteur de l'interleukine-12 (IL-12R-betal) ; chaîne bêta-2 du récepteur de l'interleukine-12 (récepteur beta-2 IL-12) ; chaîne alpha-1 du récepteur de l'interleukine-13 (IL-13R-alpha-1) (antigène CD213 al) ; chaîne alpha-2 du récepteur de l'interleukine-13 (protéine de liaison à l'interleukine-13) ; récepteur de l'interleukine-17 (récepteur IL-17) ; récepteur de l'interleukine-17B (récepteur IL-17B) ; précurseur du récepteur de l'interleukine-21 (IL-21R) ; récepteur de l'interleukine-1, type I (IL-1R-1) (CD121a) ; récepteur de l'interleukine-1, type II (IL-1R-beta) (CDw121b) ; protéine antagoniste du récepteur de l'interleukine-1 (IL-1ra) ; chaîne alpha du récepteur de l'interleukine-2 (antigène CD25) ; chaîne bêta du récepteur de l'interleukine-2 (antigène CD122) ; chaîne alpha du récepteur de l'interleukine-3 (IL-3R-alpha) (antigène CD123).

2. Polypeptide selon la revendication 1, dans lequel ladite cellule est une cellule malade, de préférence une cellule cancéreuse.

3. Polypeptide selon la revendication 1 ou 2, dans lequel ladite cellule comprend un rapport de 0,01 à 0,9 de ladite première cible et de ladite seconde cible, de manière encore plus préférée de 0,2 à 0,8, de 0,3 à 0,7, de 0,4 à 0,6, tel qu'un rapport de 0,02, 0,05, 0,08, 0,1, 0,2, 0,3, 0,4, 0,5, 0,6, 0,7, 0,8, 0,9, de préférence un rapport de 0,5.

4. Polypeptide selon l'une quelconque des revendications 1 à 3, dans lequel ledit polypeptide
- présente une constante de vitesse d'activation (Kon) par rapport à ladite première cible choisie dans le groupe se composant de : environ 10² M⁻¹s⁻¹, environ 10³ M⁻¹s⁻¹, environ 10⁴ M⁻¹s⁻¹, au moins environ 10⁵ M⁻¹s⁻¹, et environ 10⁶ M⁻¹s⁻¹, telle que mesurée par résonance plasmonique de surface, et/ou
- présente une constante de vitesse d'arrêt (Koff) par rapport à ladite première cible sélectionnée dans le groupe se composant de : environ 10⁻³s⁻¹, environ 10⁻⁴s⁻¹, environ 10⁻⁵s⁻¹, et environ 10⁻⁶s⁻¹, telle que mesurée par résonance plasmonique de surface, et/ou
- présente une constante de dissociation (K_{D}) par rapport à ladite première cible choisie dans le groupe se composant de : environ 10⁻⁷ M, environ 10⁻⁸ M, environ 10⁻⁹ M, environ 10⁻¹⁰ M, environ 10⁻¹¹ M et environ 10⁻¹² M, telle que mesurée par résonance plasmonique de surface.

5. Polypeptide selon l"une quelconque des revendications 1 à 4, dans lequel ledit premier ISV
- se lie à une première cible avec une valeur KD moyenne comprise entre 10 nM et 200 nM, telle qu"une valeur KD moyenne de 10, 15, 20, 30, 40, 50, 60, 70, 80, 90, 100, 110, 120, 130, 140, 150, 160, 170, 180 ou 190 nM, telle que mesurée par SPR, et/ou
- inhibe une chimiotaxie d'environ 10 %, 20 %, 30 %, 40 %, 50 %, 60 %, 80 %, 90 % et de préférence 95 % ou plus dans un test de chimiotaxie, et/ou
- inhibe une anaplasie, un caractère invasif, des métastases, une prolifération, une différenciation, une migration et/ou une survie de ladite cellule, et/ou
- augmente une apoptose, une mort cellulaire et/ou un arrêt de croissance de ladite cellule.

6. Polypeptide selon l'une quelconque des revendications 1 à 5, dans lequel ledit second ISV
- se lie à une seconde cible avec une valeur KD moyenne comprise entre 10 nM et 0,1 pM, telle qu'à une valeur KD moyenne de 10 nM ou moins, de manière encore plus préférée à une valeur KD moyenne de 9 nM ou moins, telle qu'inférieure à 8, 7, 6, 5, 4, 3, 2, 1, 0,5 nM ou même moins, telle qu'inférieure à 400, 300, 200, 100, 50, 40, 30, 20, 10, 9, 8, 7, 6, 5, 4, 3, 2, 1, 0,5 pM ou même moins, telle qu'inférieure à 0,4 pM, telle que mesurée par SPR, et/ou
- inhibe une liaison d'un ligand naturel à ladite seconde cible de moins de 50 %, telle que 40 %, 30 %, ou 20 % ou même moins de 10 %, telle que moins de 5 %.

7. Polypeptide selon l'une quelconque des revendications 1 à 6, dans lequel ladite première cible et ladite seconde cible sont choisies dans le groupe se composant de :
- EGFR en tant que première cible et CEA en tant que seconde cible ;
- récepteur à activité tyrosine kinase en tant que première cible et un antigène associé à une tumeur (TAA) en tant que seconde cible ;
- récepteur couplé aux protéines G (GPCR) en tant que première cible et un antigène de différenciation hématopoïétique en tant que seconde cible ;
- récepteur à activité tyrosine kinase en tant que première cible et un antigène de différenciation hématopoïétique en tant que seconde cible ;
- récepteur couplé aux protéines G (GPCR) en tant que première cible et un antigène associé à une tumeur (TAA) en tant que seconde cible ;
- CXCR4 en tant que première cible et CD123 en tant que seconde cible ;
- DR5 en tant que première cible et EpCam en tant que seconde cible ;
- DR4 en tant que première cible et EpCam en tant que seconde cible ;
- CD95 en tant que première cible et EpCam en tant que seconde cible ;
- CD47 en tant que première cible et CD123 en tant que seconde cible ;
- CD47 en tant que première cible et EpCam en tant que seconde cible ;
- CD4 en tant que première cible et CXCR4 en tant que seconde cible ;
- IL12Rβ1 en tant que première cible et CD4 en tant que seconde cible ;
- IL12Rβ2 en tant que première cible et CD4 en tant que seconde cible ; et
- IL23R comme première cible et CD4 comme seconde cible.

8. Polypeptide selon la revendication 7, dans lequel ladite première cible et ladite seconde cible sont choisies dans le groupe se composant de :
- EGFR en tant que première cible et CEA en tant que seconde cible ;
- CXCR4 en tant que première cible et CD123 en tant que seconde cible ;
- CD4 en tant que première cible et CXCR4 en tant que seconde cible ;
- IL12Rβ2 en tant que première cible et CD4 en tant que seconde cible ; et
- IL23R en tant que première cible et CD4 en tant que seconde cible.

9. Polypeptide selon la revendication 8,
- dans lequel ledit EGFR de liaison à un ISV est choisi dans le groupe se composant des SEQ ID N° : 39-45 ;
- dans lequel ledit CEA de liaison à un ISV est choisi dans le groupe se composant des SEQ ID N° : 32-38 ;
- dans lequel ledit CXCR4 de liaison à un ISV est choisi dans le groupe se composant des SEQ ID N° : 1-8 et 24 ;
- dans lequel ledit CD123 de liaison à un ISV est choisi dans le groupe se composant des SEQ ID N° : 9-15 ;
- dans lequel ledit CD4 de liaison à un ISV est choisi dans le groupe se composant des SEQ ID N° : 26 et 62 ;
- dans lequel ledit IL12Rβ1 de liaison à un ISV est choisi dans le groupe se composant des SEQ ID N° : 66-67 ;
- dans lequel ledit IL12Rβ2 de liaison à un ISV est choisi dans le groupe se composant des SEQ ID N° : 68-69 ;
- dans lequel ledit IL23R de liaison à l'ISV est choisi dans le groupe se composant des SEQ ID N° : 63-65.

10. Polypeptide selon la revendication 8, choisi dans le groupe se composant des SEQ ID N° : 16-23, 26-31, 46-55 et 70-81.

11. Polypeptide selon l'une quelconque des revendications 1 à 10, comprenant en outre un médicament, tel qu'une toxine ou un fragment de toxine.

12. Polypeptide selon l'une quelconque des revendications 1 à 10, comprenant en outre un agent d'imagerie, incluant, mais sans s'y limiter, une molécule de préférence choisie dans le groupe se composant de molécules organiques, de marqueurs enzymatiques, de marqueurs radioactifs, de marqueurs colorés, de marqueurs fluorescents, de marqueurs chromogéniques, de marqueurs luminescents, d'haptènes, de digoxigénine, de biotine, de complexes métalliques, de métaux, d'or colloïdal, d'un marqueur fluorescent, d'un marqueur métallique, de biotine, d'un chimiluminescent, d'un bioluminescent, d'un chromophore et de mélanges de ceux-ci.

13. Composition pharmaceutique comprenant un polypeptide selon l'une quelconque des revendications 1 à 12.

14. Polypeptide selon l'une quelconque des revendications 1 à 12, destiné à être utilisé dans un procédé d'administration d'un polypeptide prophylactique ou thérapeutique, d'un conjugué polypeptide-médicament (PDC) ou d'un agent d'imagerie à un emplacement spécifique, un type de tissu ou de cellule dans le corps, le procédé comprenant les étapes d'administration à un sujet d'un polypeptide selon l'une quelconque des revendications 1 à 12.

15. Polypeptide selon l'une quelconque des revendications 1 à 12, destiné à être utilisé dans le traitement d'un sujet en ayant besoin.
